# EUROPEAN PATENT APPLICATION

(11) **EP 4 759 303 A1**
(43) Date of publication of application: **17.06.2026**
(21) Application number: 24853706.0
(22) Date of filing: 09.08.2024
(51) Int. Cl.: A61K 9/127, C12N 15/117, A61K 45/00, A61P 35/00, A61K 31/7105

(54) **TARGETING LIPID COMPOSITION AND PREPARATION METHOD THEREFOR**

(30) Priority: 11.08.2023 CN 202311015976; 12.04.2024 CN 202410444806
(71) Applicant: Haichang Biotech Co., Ltd., Zhejiang 310018 (CN)
(72) Inventor: LIN, Jialiang, Hangzhou, Zhejiang 310018 (CN); LI, Ting, Hangzhou, Zhejiang 310018 (CN); YANG, Binbin, Hangzhou, Zhejiang 310018 (CN); LEE, Robert J., Hangzhou, Zhejiang 310018 (CN); YANG, Yongsheng, Hangzhou, Zhejiang 310018 (CN); ZHAO, Xiaobin, Hangzhou, Zhejiang 310018 (CN)
(74) Representative: Petraz, Gilberto Luigi
(86) International application number: PCT/CN2024/111109
(87) International publication number: WO 2025/036297

(57) **Abstract**

A targeting lipid composition comprising an active component, a lipid carrier, and a targeting component, wherein the component of the lipid carrier comprises a positively charged component and a phospholipid. By encapsulating the active component in the lipid carrier containing the positively charged component, its surface is positively charged, the lipid carrier and the negatively charged targeting component are then mixed, and the targeting component is adsorbed on the surface of the positively charged drug-loaded lipid carrier by means of electrostatic adsorption, so as to prepare a targeting lipid composition with high transfection efficiency and high targeting performance. A lipid nanoparticle delivery system having high bioactivity, high tumor targeting performance and good safety, which system comprises an active component, a cationic lipid, an ionizable lipid and a neutral phospholipid. By encapsulating the active component in a lipid nanoparticle containing a cationic lipid, an ionizable lipid, and a neutral phospholipid, when the molar ratio of the cationic lipid to the ionizable lipid is within a specific range, high biological activity, tumor targeting performance, and good safety are achieved.

## Description

### Cross-Reference to Related Applications

The present application claims priority to CN202311015976.1 filed on August 11, 2023, and CN202410444806.3 filed on April 12, 2024. The prior applications are considered part of the disclosure of the present application and are incorporated herein by reference in their entirety.

### Technical Field

The present invention is in the field of biomedical technology and relates to a targeting lipid composition and preparation method therefor.

### Background

Currently, researchers have attempted to modify the surface of lipid nanoparticle (LNP) carriers with targeting moieties by doping or chemical bonding methods to prepare targeting lipid compositions. The doping method mainly involves adding targeting moiety-modified polymer-conjugated lipids into a lipid carrier solution. Utilizing the amphiphilicity of the targeting moiety-modified polymer-conjugated lipids, the hydrophobic groups of the targeting moiety-modified polymer-conjugated lipids are inserted into the lipid layer of the lipid carrier, thereby the lipid carrier is modified with the targeting moiety. Since the surface of the assembled lipid carrier is already covered with a layer of polymer-conjugated lipids (e.g., PEG-DMG), the steric hindrance of the polymer (e.g., PEG) makes it difficult for targeting lipids to efficiently insert into the LNP lipid layer, resulting in low efficiency of modifying LNPs with targeting lipids via the doping method. The chemical bonding method mainly involves linking LNPs and targeting moieties through the reaction and bonding of groups A and B. Specifically, lipid materials containing active group A are added during the preparation of the lipid carrier. Then, the targeting moiety is modified with another active group B that can efficiently react with active group A under mild conditions. Subsequently, the active group B-modified targeting moiety is added to the lipid carrier, allowing active groups A and B to react and bond, thereby the lipid carrier is modified with the targeting moiety. The chemical bonding method has disadvantages such as involving many parameters, difficulty in controlling conditions, difficulty in ensuring the stability of active groups, and overly complex steps.

Furthermore, although there are research reports on preparing lipid nanoparticles using cationic lipids and ionizable lipid combinations, such as patent applications CN105163721A, US11229609, and US11648210B2, these studies did not discover that when the molar ratio of cationic lipid to ionizable lipid is within a certain range, the composition would simultaneously exhibit all three characteristics of high biological activity, tumor targeting, and good safety.

### Summary

The present disclosure provides a lipid composition comprising a lipid carrier, an active component, and a targeting component. Compared to a lipid composition without a targeting component, it has improved transfection efficiency of the active substance into host cells, can be used for transfecting or long-term transfecting host cells, can be used for *in vivo* tissue targeting and/or for targeting delivery of active components. The present disclosure also provides a method for modifying LNPs with targeting moieties on their surface, which is simple in steps and has high modification efficiency. The targeting lipid composition provided by the present invention has high transfection efficiency and good targeting ability. Active components known in the art to have therapeutic effects on diseases can be used as the active component to prepare the targeting lipid composition, which can then be formulated into a drug with one or more pharmaceutically acceptable excipients for the treatment of diseases. In the present disclosure, by means of encapsulating the active component in the lipid carrier containing the positively charged component, its surface is positively charged, the lipid carrier and the negatively charged targeting component are then mixed, and the targeting component is adsorbed on the surface of the positively charged drug-loaded lipid carrier by means of electrostatic adsorption, thereby preparing a lipid composition with high transfection rate and high targeting effect using this simple and highly efficient modification method.

The lipid composition provided by the present disclosure has high transfection efficiency and high targeting ability. It can effectively target its encapsulated active component to immune cells, tumor cells, liver cells, lung cells, and various other cells and tissues requiring regulation in a subject, thereby effectively improving the therapeutic effect or diagnostic accuracy of the active component.

In a first aspect, the present disclosure provides a lipid composition, comprising an active component, a lipid carrier, and a targeting component, wherein the lipid carrier comprises a positively charged component and a phospholipid, and the molar percentage of the positively charged component in the lipid carrier is 0.5%-80%. In some embodiments, the lipid carrier comprises any one of the following (a)-(f): (a) DOTAP at a molar percentage of 2.5%, SM-102 at 47.5%, DSPC at 10%, cholesterol at 38.5%, and DMG-PEG2000 at 1.5% in the lipid carrier; (b) DOTAP at a molar percentage of 5%, SM-102 at 45%, DSPC at 10%, cholesterol at 38.5%, and DMG-PEG2000 at 1.5% in the lipid carrier; (c) DOTAP at a molar percentage of 7.5%, SM-102 at 42.5%, DSPC at 10%, cholesterol at 38.5%, and DMG-PEG2000 at 1.5% in the lipid carrier; (d) DOTAP at a molar percentage of 10%, SM-102 at 40%, DSPC at 10%, cholesterol at 38.5%, and DMG-PEG2000 at 1.5% in the lipid carrier; (e) DOTAP at a molar percentage of 25%, SM-102 at 25%, DSPC at 10%, cholesterol at 38.5%, and DMG-PEG2000 at 1.5% in the lipid carrier; (f) DOTAP at a molar percentage of 45%, SM-102 at 5%, DSPC at 10%, cholesterol at 38.5%, and DMG-PEG2000 at 1.5% in the lipid carrier. In some embodiments, the lipid composition provided by the present disclosure comprises any one or more of the following lipid compositions: Archexin-LNPs-Trf, eGFP-mRNA-LNPs-Trf, LNPs-DIR-CD5, eGFP-mRNA-LNPs-CD5, CD19 CAR-mRNA-LNPs-Trf, CD19 CAR-mRNA-LNPs-CD5, Archexin-LNPs-albumin, eGFP-mRNA-LNPs-albumin, CD19 CAR-mRNA-LNPs-albumin. The lipid composition provided by the present disclosure, compared to a lipid composition without a targeting component, has improved transfection efficiency of the active substance into host cells, can be used for transfecting or long-term transfecting host cells, and can be used for *in vivo* tissue targeting and/or for targeting delivery of active components.

In a second aspect, the present disclosure provides a method for modifying LNPs with targeting moieties on their surface or a method for preparing the lipid composition of the first aspect of the present disclosure, comprising the following steps (a)-(e): (a) preparing a solution I comprising an active component using a buffer and the active component; (b) preparing a lipid solution using an organic solvent and a lipid carrier; (c) mixing the lipid solution and the solution I, diluting to obtain an initial lipid nanoparticle solution; optionally repeating steps (a)-(c); (d) removing the organic solvent from the initial lipid nanoparticle solution, diluting and adjusting pH to 6.0-8.0 to obtain an active component-loaded lipid nanoparticle suspension; (e) preparing a targeting component solution, then mixing the active component-loaded lipid nanoparticle suspension and the targeting component solution. This preparation method is simple in steps and has high efficiency in modifying LNPs with targeting moieties.

In a third aspect, the present disclosure provides a use of the lipid composition of the first aspect of the present disclosure in the manufacture of a medicament for treating or preventing a disease, or a disease diagnostic agent.

In a fourth aspect, the present disclosure provides a pharmaceutical composition comprising the lipid composition of the first aspect of the present disclosure and one or more pharmaceutically acceptable carriers or excipients.

In a fifth aspect, the present disclosure provides a method for treating or preventing a disease, comprising administering a therapeutically or prophylactically effective amount of the lipid composition of the first aspect of the present disclosure or the pharmaceutical composition of the third aspect of the present disclosure to a subject in need thereof.

In a sixth aspect, the present disclosure provides a method for transfecting a host cell, comprising administering the lipid composition of the first aspect of the present disclosure to the host cell.

In a seventh aspect, the present disclosure provides a method for targeting delivery of an active component, comprising administering the lipid composition of the first aspect of the present disclosure to a subject or a biological sample.

The present disclosure also provides lipid nanoparticles with an optimal ratio of cationic lipid to ionizable lipid. It was found that the molar ratio of cationic lipid to ionizable lipid is not simply "the higher the better" or "the lower the better", but rather there is a preferred range that cannot be derived solely from known literature or common knowledge.

In an eighth aspect, the present disclosure provides a lipid nanoparticle delivery system, comprising an active component, a lipid carrier, and optionally a targeting component, wherein the lipid carrier comprises a cationic lipid, an ionizable lipid, and a neutral phospholipid, and the molar ratio of the cationic lipid to the ionizable lipid is 0.5:49.5 to 49:1. In some preferred embodiments, the molar ratio of the cationic lipid to the ionizable lipid in the lipid nanoparticle delivery system provided by the present disclosure is 0.5:49.5 to 10:40. In some more preferred embodiments, the molar ratio of the cationic lipid to the ionizable lipid in the lipid nanoparticle delivery system provided by the present disclosure is 1.5:48.5 to 7.5:42.5. In some preferred embodiments, the lipid carrier is selected from lipid nanoparticles.

In a ninth aspect, the present disclosure provides a pharmaceutical composition comprising the lipid nanoparticle delivery system of the eighth aspect of the present disclosure and one or more pharmaceutically acceptable carriers or excipients.

In a tenth aspect, the present disclosure provides a method for treating or preventing a disease, comprising administering a therapeutically or prophylactically effective amount of the lipid nanoparticle delivery system of the eighth aspect of the present disclosure or the pharmaceutical composition of the ninth aspect of the present disclosure to a subject in need thereof.

In an eleventh aspect, the present disclosure provides a use of the lipid nanoparticle delivery system of the eighth aspect of the present disclosure in the manufacture of a medicament for treating or preventing a disease, or a disease diagnostic agent.

In a twelfth aspect, the present disclosure provides a method for transfecting a host cell, comprising administering the lipid nanoparticle delivery system of the eighth aspect of the present disclosure to the host cell.

In a thirteenth aspect, the present disclosure provides a method for targeting delivery of an active component, comprising administering the lipid nanoparticle delivery system of the eighth aspect of the present disclosure to a subject or a biological sample.

In a fourteenth aspect, the present disclosure provides a method for preparing the lipid nanoparticle delivery system of the eighth aspect of the present disclosure, comprising the following steps (a)-(d) of process I, or steps (e)-(i) of process II: Process I: (a) preparing a solution I comprising an active component using a buffer and the active component, (b) preparing a lipid solution using an organic solvent and a lipid carrier, (c) mixing the lipid solution and the solution I, diluting to obtain an initial lipid nanoparticle (LNP) solution, optionally repeating steps (a)-(c), (d) removing the organic solvent from the initial LNP solution, diluting and adjusting pH to 6.0-8.0, to obtain an active component-loaded LNP suspension;

Process II: (e) preparing a lipid solution using an organic solvent and a lipid carrier, (f) preparing an initial blank LNP solution using a buffer and the lipid solution, optionally repeating steps (e)-(f), (g) removing the organic solvent from the initial LNP solution, diluting and adjusting pH to 6.0-8.0, to obtain a blank LNP suspension, (h) preparing a solution I comprising an active component using a buffer and the active component, (i) mixing the solution I and the blank LNP suspension, then adjusting pH to 6.0-8.0, to obtain an active component-loaded LNP suspension.

### Brief Description of the Figures

Figure 1 shows the particle size, PDI, and zeta potential results of nucleic acid-loaded lipid nanoparticles (stock solution) prepared in Examples 1-6 measured in Tirs buffer at pH 5.0.
Figure 2 shows the particle size, PDI, and zeta potential results of nucleic acid-loaded lipid nanoparticles (stock solution) prepared in Examples 1-6 measured in Tirs buffer at pH 7.0.
Figure 3 shows the zeta potential results of nucleic acid-loaded lipid nanoparticles prepared in Examples 1-6 measured in Tirs buffer at pH 7.0 after 20-fold dilution with DEPC water or PBS.
Figure 4 shows the particle size, PDI, and zeta potential results of targeting lipid compositions prepared in Examples 1-6 measured in Tirs buffer at pH 7.0 after 20-fold dilution with DEPC water.
Figure 5 shows the particle size and PDI results of targeting lipid compositions prepared in Examples 1-6 measured in Tirs buffer at pH 7.0 after 20-fold dilution with PBS.
Figure 6 shows the change in GFP green fluorescence intensity over increasing transfection time after transfection of mouse RAW264.7 with eGFP-mRNA-LNPs and eGFP-mRNA-LNPs-Trf.
Figure 7 shows the transfection effect of eGFP-mRNA-LNPs and eGFP-mRNA-LNPs-Trf on macrophages in PBMC cells *in vitro.*
Figure 8 shows the transfection effect of eGFP-mRNA-LNPs and eGFP-mRNA-LNPs-Trf on T cells in PBMC cells *in vitro.*
Figure 9 shows the transfection effect of eGFP-mRNA-LNPs and eGFP-mRNA-LNPs-Trf on macrophages *in vivo.*
Figure 10 shows the transfection effect of eGFP-mRNA-LNPs and eGFP-mRNA-LNPs-Trf on splenic T cells *in vivo.*
Figure 11 shows the transfection effect of eGFP-mRNA-LNPs and eGFP-mRNA-LNPs-Trf on splenic NK cells *in vivo.*
Figure 12 shows the *in vivo* tissue targeting effect of LNPs-DIR and LNPs-DIR-CD5 (DOTAP/SM102=2.5/47.5).
Figure 13 shows the *in vivo* tissue targeting effect of LNPs-DIR and LNPs-DIR-CD5 (DOTAP/SM102=5/45).
Figure 14 shows the transfection effect on splenic T cells *in vivo* at 12 h after intravenous injection of eGFP-mRNA-LNPs and eGFP-mRNA-LNPs-CD5.
Figure 15 shows the transfection effect on splenic T cells *in vivo* at 48 h after intravenous injection of eGFP-mRNA-LNPs and eGFP-mRNA-LNPs-CD5.
Figure 16 shows the transfection effect on splenic NK cells *in vivo* at 12 h after intravenous injection of eGFP-mRNA-LNPs and eGFP-mRNA-LNPs-CD5.
Figure 17 shows the transfection effect on splenic NK cells *in vivo* at 24 h after intravenous injection of eGFP-mRNA-LNPs and eGFP-mRNA-LNPs-CD5.
Figure 18 shows the transfection effect on splenic NK cells *in vivo* at 48 h after intravenous injection of eGFP-mRNA-LNPs and eGFP-mRNA-LNPs-CD5.
Figure 19 shows the transfection effect on splenic T cells *in vivo* at 24 h after intravenous injection of CD19 CAR-mRNA-LNPs and CD19 CAR-mRNA-LNPs-CD5.
Figure 20 shows the transfection effect on splenic T cells *in vivo* at 72 h after intravenous injection of CD19 CAR-mRNA-LNPs and CD19 CAR-mRNA-LNPs-CD5.
Figure 21 shows the transfection effect on splenic T cells *in vivo* at 120 h after intravenous injection of CD19 CAR-mRNA-LNPs and CD19 CAR-mRNA-LNPs-CD5.
Figure 22 shows the transfection effect on splenic T cells *in vivo* at 168 h after intravenous injection of CD19 CAR-mRNA-LNPs and CD19 CAR-mRNA-LNPs-CD5.
Figure 23 shows the transfection effect on splenic NK cells *in vivo* at 24 h after intravenous injection of CD19 CAR-mRNA-LNPs and CD19 CAR-mRNA-LNPs-CD5.
Figure 24 shows the transfection effect on splenic NK cells *in vivo* at 72 h after intravenous injection of CD19 CAR-mRNA-LNPs and CD19 CAR-mRNA-LNPs-CD5.
Figure 25 shows the transfection effect on splenic NK cells *in vivo* at 120 h after intravenous injection of CD19 CAR-mRNA-LNPs and CD19 CAR-mRNA-LNPs-CD5.
Figure 26 shows the transfection effect on splenic NK cells *in vivo* at 168 h after intravenous injection of CD19 CAR-mRNA-LNPs and CD19 CAR-mRNA-LNPs-CD5.
Figure 27 shows histograms of the relative fluorescence intensity and cell viability after mRNA uptake by cells treated with different formulations.
Figure 28 shows histograms of the tumor fluorescence intensity in tumor-bearing mice treated with Fluc-eGFP mRNA encapsulated by different formulations.
Figure 29 shows graphs of the anti-tumor efficacy of Archexin encapsulated by different formulations in tumor-bearing mice, showing changes in tumor size with treatment days.
Figure 30 shows graphs of the anti-tumor efficacy of doxorubicin encapsulated by different formulations in tumor-bearing mice, showing changes in tumor size with treatment days.
Figure 31 shows a graph of the anti-tumor efficacy in tumor-bearing mice of Formulation 303 experimental group loaded with TP53 mRNA, Moderna formulation group loaded with TP53 mRNA, and PBS negative control group, showing changes in tumor size with treatment days.

### Detailed Description of the Invention

Unless otherwise specified, the terms used herein have the usual meanings understood by those skilled in the relevant art. Those skilled in the art can vary these terms based on the desired properties and effects sought through this application. Each numerical parameter should be interpreted in view of the number of significant digits and ordinary rounding methods or as understood by those skilled in the art. In general, the nomenclature and the experimental procedures of organic chemistry, medicinal chemistry, and biology described herein are well-known and commonly used in the field. Unless otherwise defined, all technical and scientific terms used herein generally have the same meaning as commonly understood by one of ordinary skill in the art to which this application belongs. In case of multiple definitions for a term used herein, the definition in this section applies unless otherwise stated.

Unless otherwise indicated, all numbers expressing quantities, concentrations, proportions, weights, particle sizes, percentages, technical effects, etc., used in the specification and claims are to be understood as being modified in all instances by the term "about" or "approximately". Accordingly, unless indicated to the contrary, the numerical parameters set forth in the following specification and appended claims are approximations. "About" or "approximately" can be understood as a range of plus or minus 10%, 20%, 30%, 40%, or 50% of the indicated value.

As used herein, the expression "A and/or B" includes three cases: (1) A; (2) B; and (3) A and B. The expression "A, B and/or C" includes seven cases: (1) A; (2) B; (3) C; (4) A and B; (5) A and C; (6) B and C; and (7) A, B, and C. The meanings of similar expressions can be deduced accordingly.

As used herein, the terms "comprise", "include", and "contain" mean that in addition to the listed elements, other elements are not excluded.

### Definitions

As used herein, the term "antibody" should be understood in its broadest interpretation, including but not limited to monoclonal antibodies (mAbs), polyclonal antibodies, fusion antibodies, multispecific (e.g., bispecific) antibodies, diabodies, nanobodies, triabodies, multivalent antibodies, chimeric antibodies, humanized antibodies, fully human antibodies, single-chain antibodies, antibody fragments retaining antigen-binding specificity, such as antigen-binding fragments. Antibodies may contain additional modifications, such as in non-CDR regions, constant regions, glycosylation sites, post-translational modifications, etc., provided that the resulting antibody retains binding specificity for the target antigen.

As used herein, "tissue" includes but is not limited to animal tissues, such as epithelial tissue, connective tissue (including blood, lymph, proper connective tissue, cartilage, and bone), muscle tissue, and nervous tissue.

### Lipid Composition, Lipid Nanoparticle Delivery System

The present disclosure provides a lipid composition comprising an active component, a lipid carrier, and a targeting component. In some embodiments, the lipid carrier of the present disclosure comprises a positively charged component (e.g., cationic lipid) and a phospholipid. In some embodiments, the molar percentage of the positively charged component in the lipid carrier of the present disclosure is 0.5%-80%.

In some embodiments, the targeting component of the present disclosure is one or more selected from the group consisting of transferrin (Trf), lactoferrin, albumin, fibronectin, vascular endothelial growth factor, epidermal growth factor, insulin-like growth factor, antibodies (including but not limited to monoclonal antibodies, polyclonal antibodies, single-chain antibodies, nanobodies, tumor-associated antigen (TAA) antibodies, etc.), polypeptides, polysaccharides, and glycolipids. In some preferred embodiments, the targeting component of the present disclosure is one or more selected from the group consisting of transferrin, serum albumin, and anti-CD5 antibodies. In some more preferred embodiments, the targeting component of the present disclosure is transferrin or anti-CD5 antibody.

In some embodiments, the active component is selected from components having at least one effect of preventing, treating, or diagnosing diseases, including but not limited to nucleic acid drugs, mRNA, proteins, small nucleic acid drugs, chemical drugs, polypeptides, fluorescent labels, radioactive diagnostic agents, etc. In some embodiments, the active component of the present disclosure is one or more selected from the group consisting of nucleic acid drugs, small molecule drugs, polypeptides, proteins, antibodies, chemical drugs, chemotherapeutic drugs, fluorescent labels, and radioactive diagnostic agents. In some embodiments, the nucleic acid drugs of the present disclosure include but are not limited to small nucleic acid drugs, mRNA drugs (e.g., TP53 mRNA). In some embodiments, the small nucleic acid drug is at least one selected from the group consisting of antisense oligonucleotides (ASO), small interfering RNA (siRNA), microRNA (miRNA), small activating RNA (saRNA), messenger RNA (mRNA), and RNA aptamers. In some preferred embodiments, the active component of the present disclosure is at least one selected from the group consisting of fluorescently labeled mRNAs (e.g., GFP-mRNA), fluorescent labels (e.g., DIR), small nucleic acids (e.g., Archexin), chimeric antigen receptor (CAR) mRNAs (e.g., CD19 CAR-mRNA), and small molecule drugs (e.g., doxorubicin). In some embodiments, the active component is one or more selected from drugs for preventing, treating, and/or diagnosing tumors, immune diseases, inflammatory diseases, infectious diseases, cardiovascular and cerebrovascular diseases, and/or endocrine diseases.

In some embodiments, the active component is selected from antibodies with anti-tumor effects. Antibodies with anti-tumor effects include but are not limited to one or more of antibodies targeting PD-1; antibodies targeting PD-L1; and antibodies targeting CTLA4. In some embodiments, the active component includes but is not limited to one or more antibodies against tumor-associated antigens (TAAs). TAAs include but are not limited to GPC, CEA, immature laminin receptor, TAG-72, HPV E6, HPV E7, EGFR, Ep-CAM, EphA3, Her2, Her3, ROR2, PSMA, STEAP1, FGFR2, TROP2, B7-H3, B7-H4, B7-H6, FOLR1, BAGE family, CAGE family, GAGE family, MAGE family, SAGE family, XAGE family, SSX-2, fibronectin, MART-2, PDL-1, PD-1, CTLA4, VEGFR, CLAUDIN, etc.

In some embodiments, chemotherapeutic drugs include but are not limited to doxorubicin, paclitaxel, gemcitabine, and radiotherapy.

In some embodiments, small molecule or nucleic acid drugs include but are not limited to pattern recognition receptor agonists, such as Toll-like receptor agonists, anthracyclines, camptothecins, immunogenic cell death (ICD) inducers, tyrosine kinase inhibitors, taxanes, Bruton's tyrosine kinase (BTK) inhibitors, Akt-1 inhibitors, PI3K inhibitors, HDAC inhibitors, ERK inhibitors, MAPK inhibitors, PD-1/PD-L1 inhibitors, CTLA-4 inhibitors, TIGIT inhibitors, TIM3 inhibitors, EGFR inhibitors, VEGF inhibitors, PARP inhibitors, Her2 inhibitors, LAG-3 inhibitors, TNFR2 inhibitors, etc.

In some embodiments, anthracyclines include but are not limited to doxorubicin, epirubicin, pirarubicin, daunorubicin, aclarubicin, idarubicin, amrubicin, etc.

In some embodiments, camptothecins include but are not limited to topotecan, irinotecan, belotecan, exatecan (DX-8951), lurtotecan, sinotecan, rubitecan (9-NC), 9-aminocamptothecin (9-AC), gimatecan, karenitecin, DB-67, etc.

In some embodiments, tyrosine kinase inhibitors include but are not limited to sorafenib, sunitinib, gefitinib, erlotinib, lapatinib, afatinib, dacomitinib, vandetanib, neratinib, pelitinib (EKB-569), canertinib (CI-1033), osimertinib, rociletinib (CO-1686, Clovis Oncology), olmutinib (HM61713, Hanmi Pharmaceutical), naquotinib (ASP8273, Astellas), tesevatinib (XL647/KD019, Kadmon Corporation), nazartinib (EGF816, Novartis), and PF-06747775 (Pfizer), etc.

In some embodiments, immunogenic cell death inducers include but are not limited to DNA damaging agents, bleomycin, etc.

In some embodiments, taxanes include but are not limited to paclitaxel, docetaxel, etc.

In some embodiments, pattern recognition receptor agonists include but are not limited to Toll-like receptors, RIG-I-like receptors, Nod-like receptors, AIM2-like receptors, and C-type lectin receptors, as well as cGas and other intracellular DNA sensor agonists.

In some embodiments, the lipid carrier has a pKa value measured by the TNS method greater than 6.8. In some preferred embodiments, the lipid carrier has a pKa value measured by the TNS method of 6.8-11. In some more preferred embodiments, the lipid carrier has a pKa value measured by the TNS method of 6.8-10. In some embodiments, the lipid carrier has a pKa value measured by the TNS method greater than 7.0. In some preferred embodiments, the lipid carrier has a pKa value measured by the TNS method of 7.0-10. In some more preferred embodiments, the lipid carrier has a pKa value measured by the TNS method of 7.0-9, for example, 7.0-8. In some embodiments, the lipid carrier has a pKa value measured by the TNS method of 7.17, 7.33, 7.47, 7.61, 7.92, 8.59, 9.67. In some embodiments, the lipid carrier is selected from lipid nanoparticles.

In some embodiments, the positively charged component (e.g., cationic lipid) is one or more selected from the group consisting of quaternary ammonium and derivatives thereof, tertiary amine and derivatives thereof, guanidine-containing compounds, polyethyleneimine and derivatives thereof, alkanolamine and derivatives thereof, meglumine and derivatives thereof, lysine and derivatives thereof, histidine and derivatives thereof, arginine and derivatives thereof, protamine and derivatives thereof. In some embodiments, the positively charged component includes but is not limited to DOTAP (CAS:132172-61-3 , (2,3-Dioleoyloxy-propyl)-trimethylammonium-chloride), DOTMA (CAS No.104872-42-6), DDBA, DMRIE, DOTIM, SAINT, DC-Chol (DC cholesterol, 3β-[N-(N',N'-dimethylaminoethane)-carbamoyl]cholesterol hydrochloride), BGTC, CTAP, DOSPA, DORIE, DODAB, DOIC, DMEPC, DOGS, DIMRI, DC-6-14, CLIP1, DORIE, DOSPA, CLIP6, CLIP9, and those disclosed in U.S. Patent No. 5,049,386, International Publications WO91/16024, WO97/019675, WO2005/121348, WO2009/086558, and WO2011/13636. In some more preferred embodiments, the positively charged component is DOTAP.

In some embodiments, the phospholipid of the present disclosure is one or more selected from the group consisting of ionizable lipids, neutral phospholipids, and PEGylated lipids. Ionizable lipids include but are not limited to tertiary amines and derivatives thereof, pyrrolidines and derivatives thereof, piperazines and derivatives thereof, piperidines and derivatives thereof, for example, DODMA (1,2-dioleyloxy-3-dimethylaminopropane, CAS:104162-47-2), ALC-0159, A066 (Z016) (1-(2,3-bis(((9Z,12Z)-octadeca-9,12-dien-1-yl)oxy)propyl)pyrrolidine), L-319 (CAS:1351586-50-9), DODAP, C12-200, 5A2-SC8, 306Oi10, Moderna Lipid 5, Acuitas A9, ALC-0315, SM-102 (also known as HUO, Moderna Lipid H; 1-octylnonyl 8-[(2-hydroxyethyl)[6-oxo-6-(undecyloxy)hexyl]amino]-octanoate), DLin-MC3-DMA ((6Z,9Z,28Z,31Z)-heptatriacont-6,9,28,31-tetraene-19-yl 4-(dimethylamino)butanoate), DLin-K-DMA, DLin-KC2-DMA, DLin-KC3-DMA, DLin-KC4-DMA, DLinDMA, other DLinDMA types, other DLin-K-DMA types, and Lipoid 5A2-SC8. Neutral phospholipids include but are not limited to phosphatidylcholines and/or phosphatidylethanolamines, for example, distearoylphosphatidylcholine (DSPC), egg phosphatidylcholine (EPC), soybean lecithin, hydrogenated soybean lecithin (HSPC), dioleoylphosphatidylethanolamine (DOPE), dilauroylphosphatidylcholine (DLPC), dimyristoylphosphatidylcholine (DMPC), dipalmitoylphosphatidylcholine (DPPC), 1,2-dioleoylphosphatidylcholine (DOPC), diarachidoylphosphatidylcholine (DAPC), dimyristoylphosphatidylethanolamine (DMPE), dilauroylphosphatidylethanolamine (DLPE), distearoylphosphatidylethanolamine (DSPE), dipalmitoylphosphatidylethanolamine (DPPE), 1-palmitoyl-2-oleoylphosphatidylethanolamine (POPE), 1-hexadecan-2-(cis-9-octadecenoyl)-sn-glycero-3-phosphocholine (POPC). PEGylated phospholipids include but are not limited to DSG-PEG, DMG-PEG (e.g., DMG-PEG-2000).

In some embodiments, the phospholipids can be conventional phospholipid components for preparing liposomes or lipid nanoparticles, including but not limited to phosphatidylcholines and/or phosphatidylethanolamines, for example, distearoylphosphatidylcholine (DSPC), egg phosphatidylcholine (EPC), soybean lecithin, hydrogenated soybean lecithin (HSPC), dioleoylphosphatidylethanolamine (DOPE), dilauroylphosphatidylcholine (DLPC), dimyristoylphosphatidylcholine (DMPC), dipalmitoylphosphatidylcholine (DPPC), 1,2-dioleoylphosphatidylcholine (DOPC), diarachidoylphosphatidylcholine (DAPC), dimyristoylphosphatidylethanolamine (DMPE), dilauroylphosphatidylethanolamine (DLPE), distearoylphosphatidylethanolamine (DSPE), dipalmitoylphosphatidylethanolamine (DPPE), 1-palmitoyl-2-oleoylphosphatidylethanolamine (POPE), ionizable lipids, PEGylated lipids, etc.

In some embodiments, the phospholipids of the present disclosure are one or more selected from the group consisting of SM-102, distearoylphosphatidylcholine, egg phosphatidylcholine, soybean lecithin, hydrogenated soybean lecithin, dioleoylphosphatidylethanolamine, dilauroylphosphatidylcholine, dimyristoylphosphatidylcholine, dipalmitoylphosphatidylcholine, 1,2-dioleoylphosphatidylcholine, diarachidoylphosphatidylcholine, dimyristoylphosphatidylethanolamine, dilauroylphosphatidylethanolamine, distearoylphosphatidylethanolamine, dipalmitoylphosphatidylethanolamine, and DMG-PEG-2000.

In some embodiments, in the lipid composition of the present disclosure, the molar percentage of the phospholipids in the lipid carrier is 8.5%-85%, for example, 59%, 56.5%, 54%, 51.5%, 36.5%, 16.5%.

In some embodiments, the lipid carrier in the lipid composition of the present disclosure comprises a positively charged component, a phospholipid, and a non-phospholipid neutral lipid. In some preferred embodiments, the non-phospholipid neutral lipid comprises cholesterol.

In some embodiments, in the lipid composition of the present disclosure, the molar percentage of the non-phospholipid neutral lipid in the lipid carrier is 20%-75%, for example, 38.5%.

In some embodiments, the lipid carrier in the lipid composition of the present disclosure comprises a positively charged component, an ionizable lipid, a neutral phospholipid, a PEGylated lipid, and a non-phospholipid neutral lipid. In some preferred embodiments, the molar ratio of the positively charged component to the ionizable lipid in the present disclosure is 1:0.1 to 1:30. In some more preferred embodiments, the molar percentage of the positively charged component in the lipid carrier of the present disclosure is 0.5%-80%, the molar percentage of the ionizable lipid in the lipid carrier of the present disclosure is 2.5%-70%, the molar percentage of the neutral phospholipid in the lipid carrier of the present disclosure is 5%-15%, the molar percentage of the PEGylated lipid in the lipid carrier of the present disclosure is 0.5%-5%, and the molar percentage of the non-phospholipid neutral lipid in the lipid carrier of the present disclosure is 20%-75%.

In some embodiments, in the lipid composition of the present disclosure, the positively charged component comprises DOTAP, the ionizable lipid comprises SM-102, the neutral phospholipid comprises DSPC, the non-phospholipid neutral lipid comprises cholesterol, and the PEGylated lipid comprises DMG-PEG2000.

In some embodiments, the lipid composition provided by the present disclosure comprises a lipid carrier comprising any one of the following (a)-(f):
(a) DOTAP at a molar percentage of 2.5%, SM-102 at 47.5%, DSPC at 10%, cholesterol at 38.5%, and DMG-PEG2000 at 1.5% in the lipid carrier;
(b) DOTAP at a molar percentage of 5%, SM-102 at 45%, DSPC at 10%, cholesterol at 38.5%, and DMG-PEG2000 at 1.5% in the lipid carrier;
(c) DOTAP at a molar percentage of 7.5%, SM-102 at 42.5%, DSPC at 10%, cholesterol at 38.5%, and DMG-PEG2000 at 1.5% in the lipid carrier;
(d) DOTAP at a molar percentage of 10%, SM-102 at 40%, DSPC at 10%, cholesterol at 38.5%, and DMG-PEG2000 at 1.5% in the lipid carrier;
(e) DOTAP at a molar percentage of 25%, SM-102 at 25%, DSPC at 10%, cholesterol at 38.5%, and DMG-PEG2000 at 1.5% in the lipid carrier;
(f) DOTAP at a molar percentage of 45%, SM-102 at 5%, DSPC at 10%, cholesterol at 38.5%, and DMG-PEG2000 at 1.5% in the lipid carrier.

In some embodiments, the lipid composition provided by the present disclosure comprises any one or more of the following lipid compositions: Archexin-LNPs-Trf, eGFP-mRNA-LNPs-Trf, LNPs-DIR-CD5, eGFP-mRNA-LNPs-CD5, CD19 CAR-mRNA-LNPs-Trf, CD19 CAR-mRNA-LNPs-CD5, Archexin-LNPs-albumin, eGFP-mRNA-LNPs-albumin, CD19 CAR-mRNA-LNPs-albumin, wherein, Archexin, eGFP-mRNA, DIR, CD19 CAR-mRNA are active components, LNPs are lipid carriers, and Trf, CD5, albumin are targeting components.

In some embodiments, in the lipid composition provided by the present disclosure, the mass ratio of the lipid carrier to the active component is 1:0.01-1:0.5 (e.g., 1:0.01, 1:0.05) and/or the mass ratio of the lipid carrier to the targeting component is 1:0.01-1:0.5 (e.g., 1:0.1, 1:0.25).

In some embodiments, by weight percentage, in the lipid composition of the present disclosure, the active component may account for 0.1%-50%; the lipid carrier may account for 10%-90%; the targeting component may account for 5%-90%.

In some embodiments, the lipid carrier in the lipid composition of the present disclosure comprises the positively charged component DOTAP, cholesterol, the neutral lipid DSPC, the ionizable lipid SM-102 (1-octylnonyl 8-[(2-hydroxyethyl)[6-oxo-6-(undecyloxy)hexyl]amino]-octanoate, abbreviated as HUO), and the PEGylated lipid.

In some embodiments, by molar percentage, the lipid carrier in the lipid composition of the present disclosure comprises DOTAP accounting for 0.5%-80%; SM-102 accounting for 5%-80%; DSPC accounting for 1%-90%; cholesterol accounting for 1%-50%; and PEGylated lipid accounting for 0%-10%.

The present disclosure provides a lipid nanoparticle delivery system comprising an active component, a lipid carrier, and optionally a targeting component, wherein the lipid carrier comprises a cationic lipid, an ionizable lipid, and a neutral phospholipid. In some embodiments, the lipid carrier is selected from lipid nanoparticles.

In some embodiments, the molar ratio of the cationic lipid to the ionizable lipid is 0.5:49.5 to 49:1, for example, 0.5:49.5, 1.5:48.5, 2.5:47.5, 5:45, 7.5:42.5, 10:40, 25:25, 35:15, 45:5, 49:1. In some preferred embodiments, the molar ratio of the cationic lipid to the ionizable lipid is 0.5:49.5 to 10:40, for example, 0.5:49.5, 1.5:48.5, 2.5:47.5, 5:45, 7.5:42.5, 10:40. In some more preferred embodiments, the molar ratio of the cationic lipid to the ionizable lipid is 1.5:48.5 to 7.5:42.5, for example, 1.5:48.5, 2.5:47.5, 5:45, 7.5:42.5.

In some embodiments, the lipid carrier has a pKa value measured by the TNS method greater than 6.8, for example, greater than 7.0. In some preferred embodiments, the lipid carrier has a pKa value measured by the TNS method of 6.8-11, for example, 7.0-11. In some more preferred embodiments, the lipid carrier has a pKa value measured by the TNS method of 6.8-10, for example, 7.0-10. In some most preferred embodiments, the lipid carrier has a pKa value measured by the TNS method of 6.8-8, for example, 7.0-8.

In some embodiments, the lipid carrier has a pKa value measured by the TNS method of 6.52, 6.73, 6.77, 6.89, 6.92, 6.94, 7.03, 7.14, 7.18, 7.17, 7.27, 7.33, 7.34, 7.36, 7.39, 7.47, 7.59, 7.60, 7.61, 7.71, 7.79, 7.85, 7.88, 7.92, 7.95, 8.02, 8.03, 8.49, 8.59, 9.21, 9.45, 9.67, 9.89, 10.00, 10.08, 10.20, 10.22, 10.37. In some preferred embodiments, the lipid carrier has a pKa value measured by the TNS method of 6.89, 6.94, 7.03, 7.47, 7.59, 7.60, 7.61.

In some embodiments, the active component is selected from components having at least one effect of preventing, treating, or diagnosing diseases, including but not limited to one or more of nucleic acid drugs, small molecule drugs, polypeptides, proteins, antibodies, chemical drugs, chemotherapeutic drugs, fluorescent labels, and radioactive diagnostic agents. In some preferred embodiments, the active component is at least one selected from the group consisting of GFP-mRNA, Archexin, doxorubicin, CD19 CAR-mRNA, DIR, and TP53 mRNA.

In some embodiments, the cationic lipid is one or more selected from the group consisting of quaternary ammonium and derivatives thereof, guanidine-containing compounds, polyethyleneimine and derivatives thereof, alkanolamine and derivatives thereof, meglumine and derivatives thereof, lysine and derivatives thereof, histidine and derivatives thereof, arginine and derivatives thereof, protamine and derivatives thereof. In some preferred embodiments, the cationic lipid is at least one selected from the group consisting of DOTAP, DOTMA, and DDBA.

In some embodiments, the ionizable lipid is one or more selected from the group consisting of tertiary amine and derivatives thereof, pyrrolidine and derivatives thereof, piperazine and derivatives thereof, piperidine and derivatives thereof. In some preferred embodiments, the ionizable lipid is at least one selected from the group consisting of SM-102, DODMA, DLin-MC3-DMA, ALC-0315, A066, DODAP, Acuitas A9, Moderna Lipid 5, C12-200, and Lipoid 5A2-SC8.

In some embodiments, the neutral phospholipid is one or more selected from the group consisting of phosphatidylcholines and/or phosphatidylethanolamines. In some preferred embodiments, the neutral phospholipid is at least one selected from the group consisting of DSPC, DOPC, EPC, HSPC, DOPE, POPE, DPPE, and DSPE.

In some embodiments, the molar percentage of the neutral phospholipid in the lipid carrier is 5%-15%. In some preferred embodiments, the molar percentage of the neutral phospholipid in the lipid carrier is 10%.

In some embodiments, the lipid nanoparticle delivery system provided herein comprises an active component, a lipid carrier, and optionally a targeting component, further comprising cholesterol and/or a PEGylated lipid. In some preferred embodiments, the molar percentage of cholesterol in the lipid carrier of the lipid nanoparticle delivery system is 20%-75%. In some more preferred embodiments, the molar percentage of cholesterol in the lipid carrier of the lipid nanoparticle delivery system is 38.5%. In some preferred embodiments, the molar percentage of the PEGylated lipid in the lipid carrier of the lipid nanoparticle delivery system is 0.5%-5%. In some more preferred embodiments, the molar percentage of the PEGylated lipid in the lipid carrier of the lipid nanoparticle delivery system is 1.5%.

In some embodiments, the lipid carrier in the lipid nanoparticle delivery system of the present disclosure comprises any one of the following formulations:

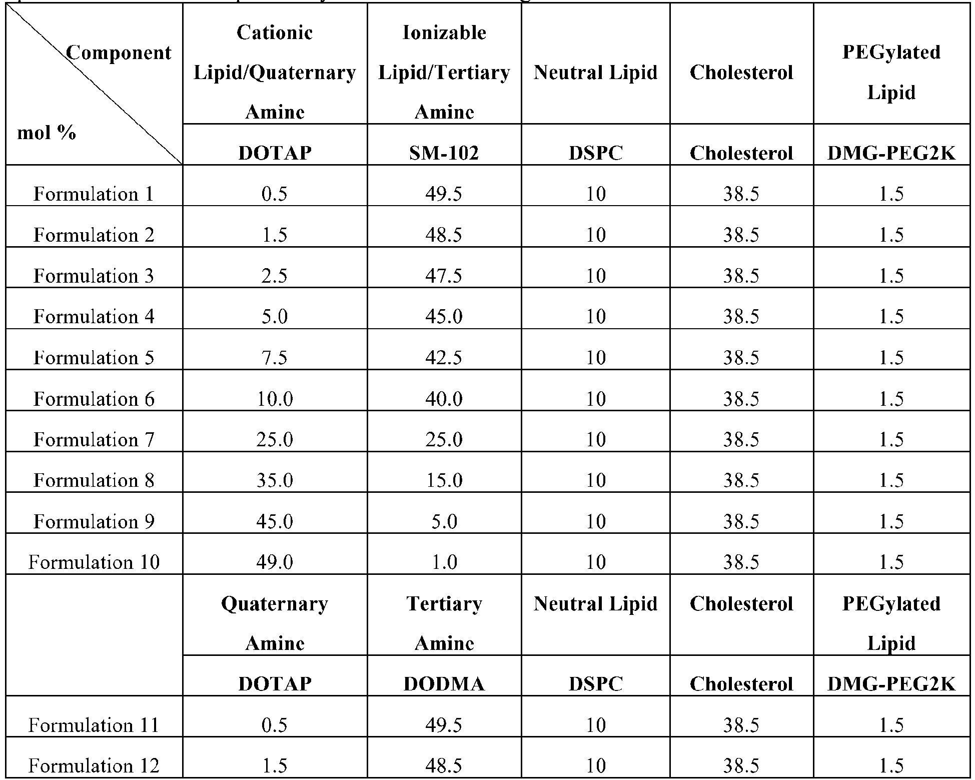

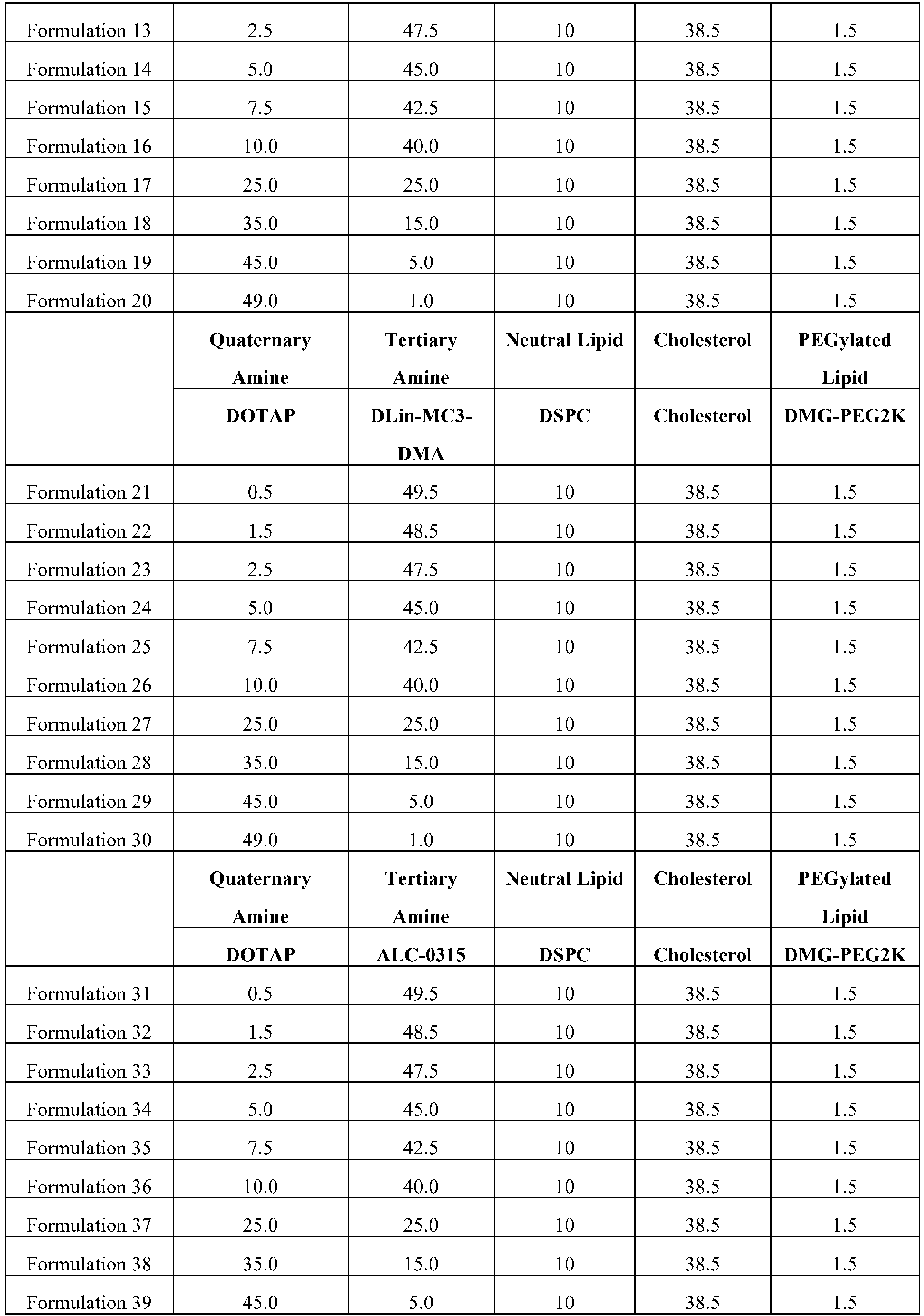

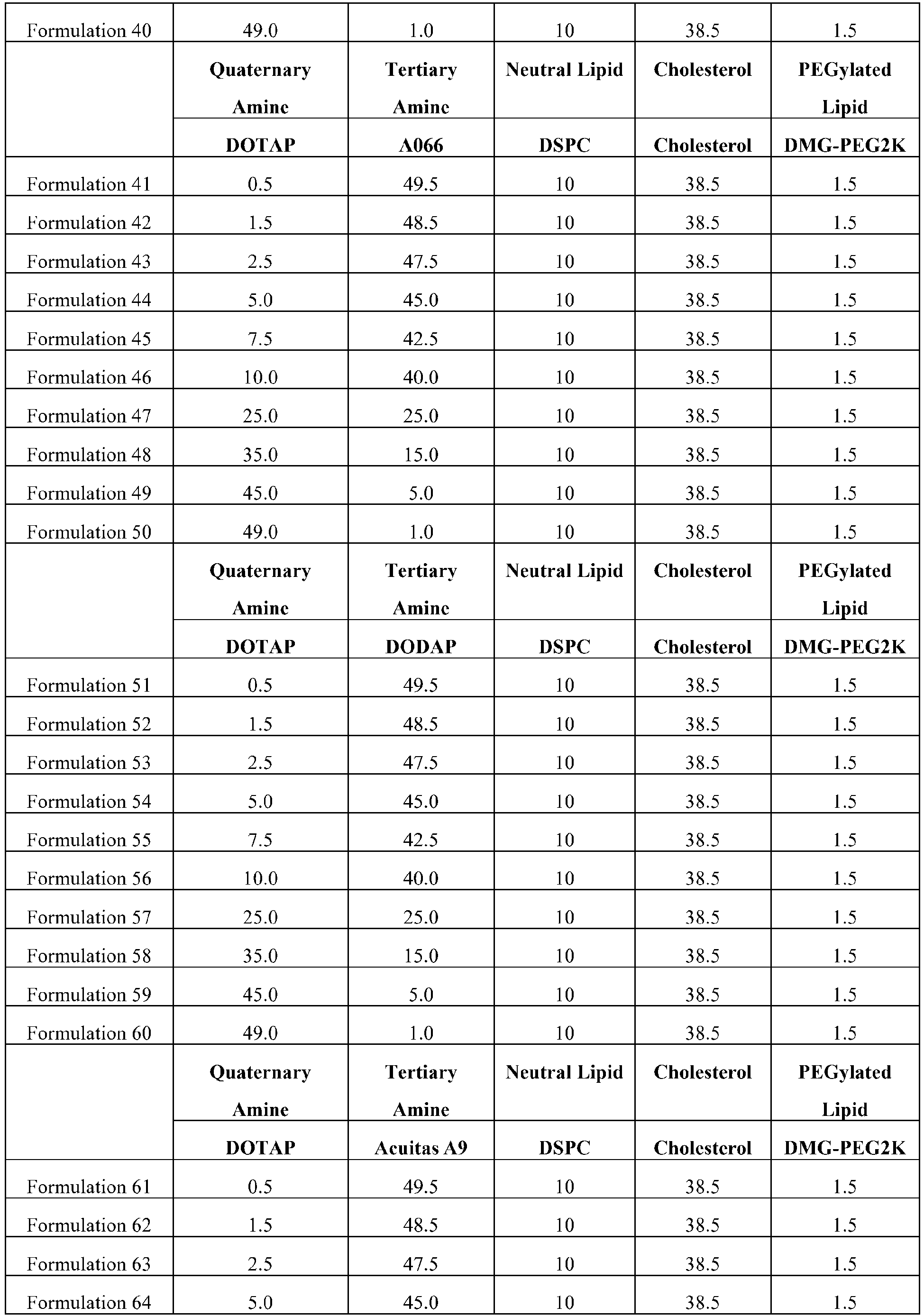

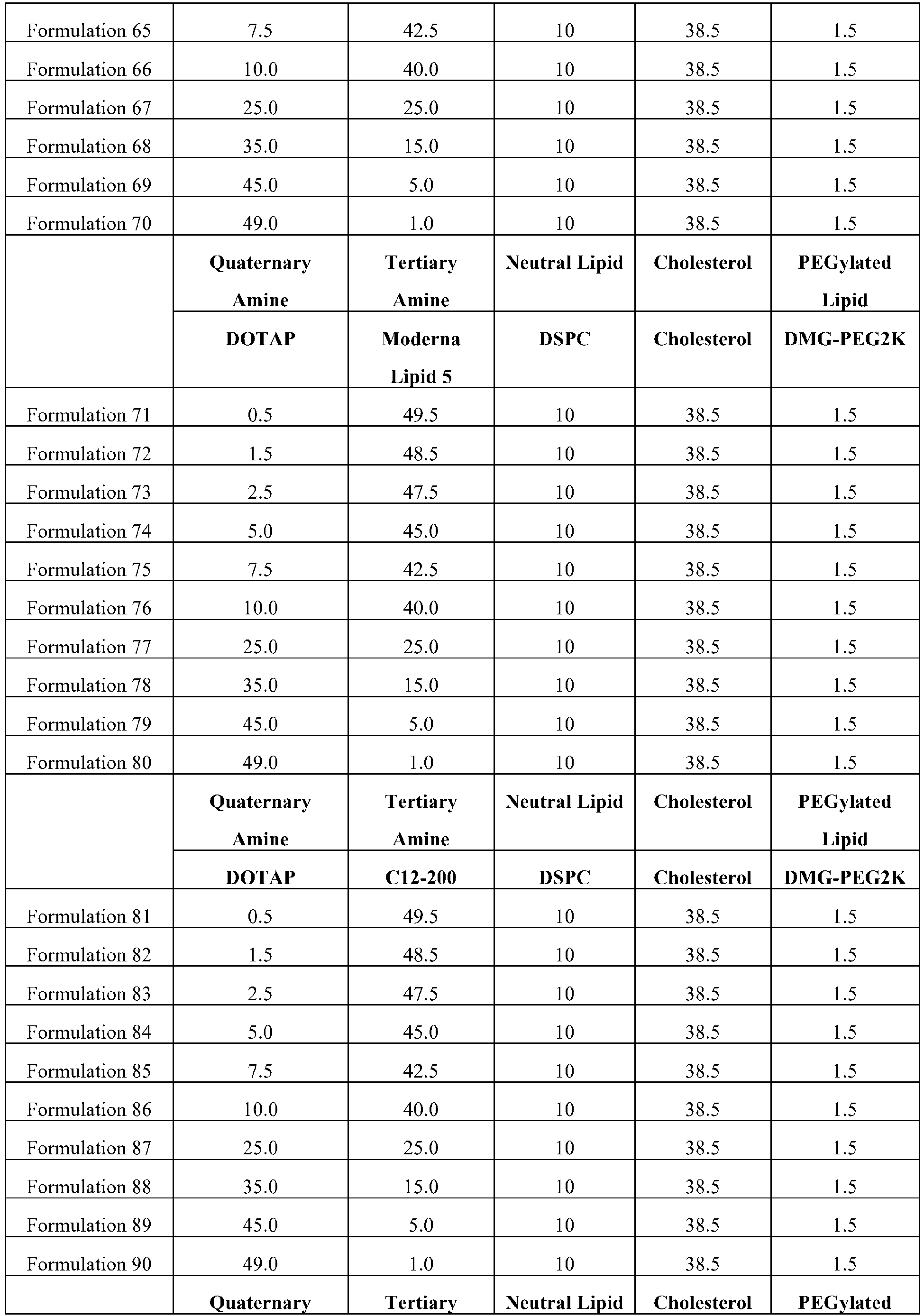

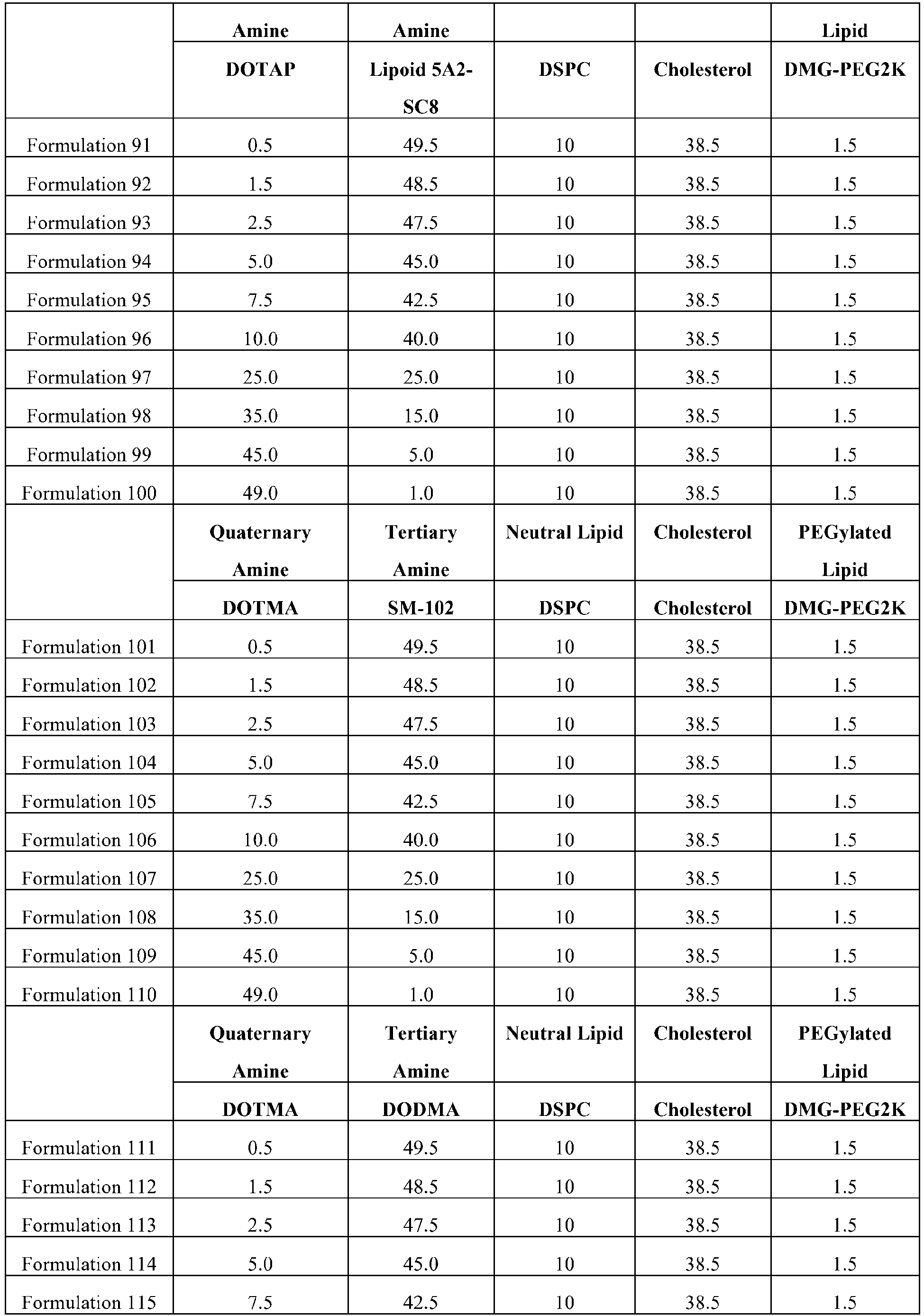

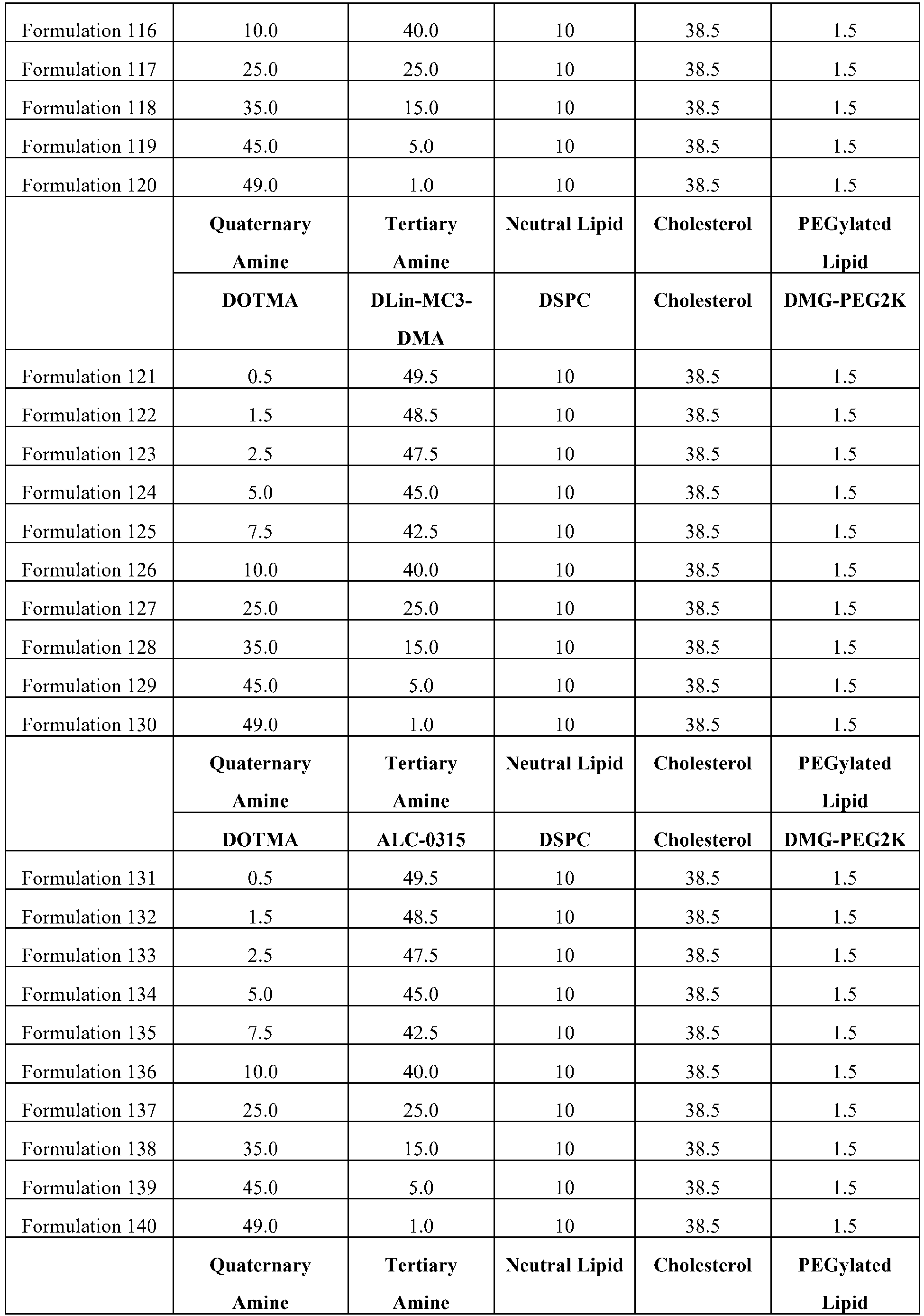

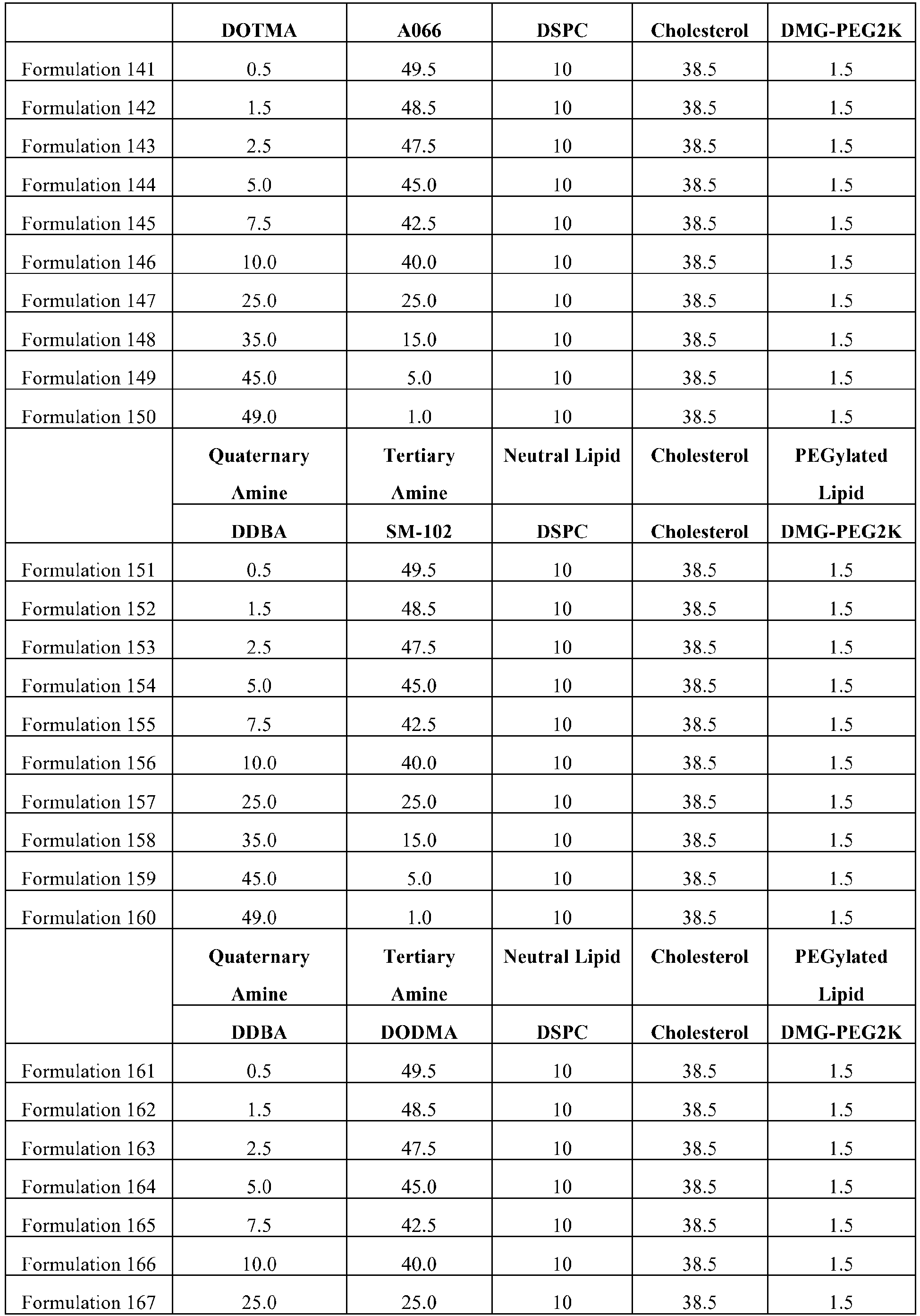

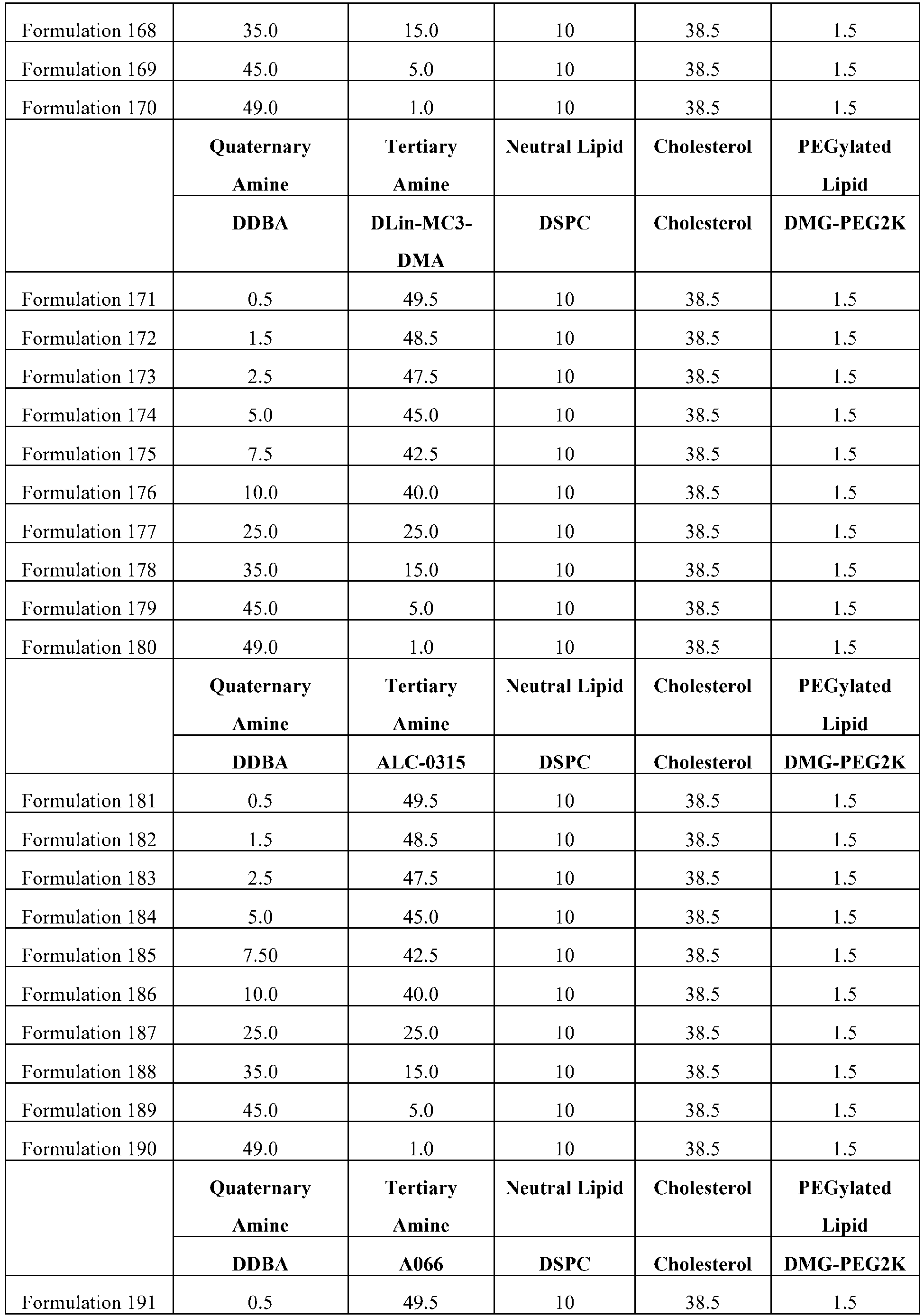

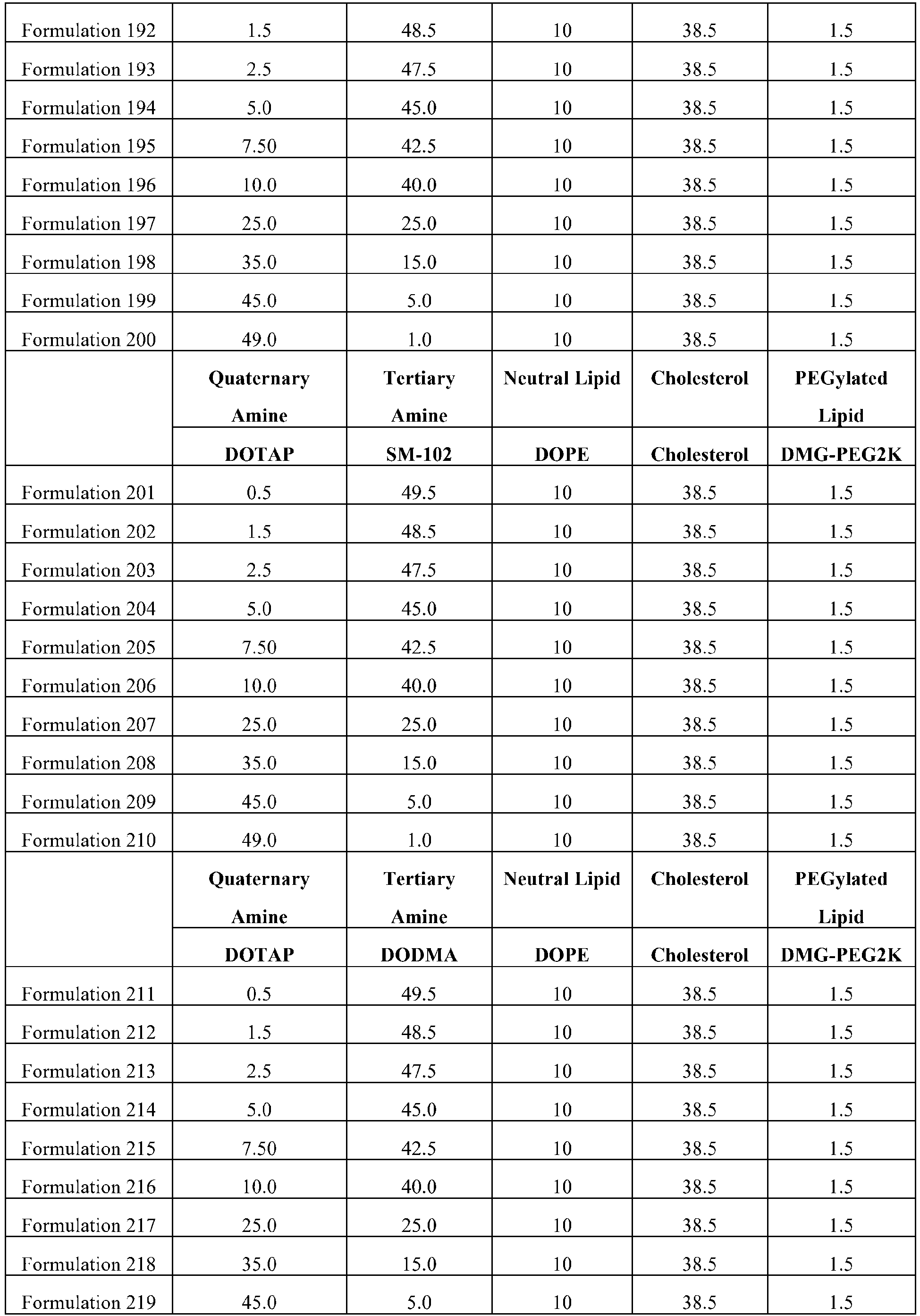

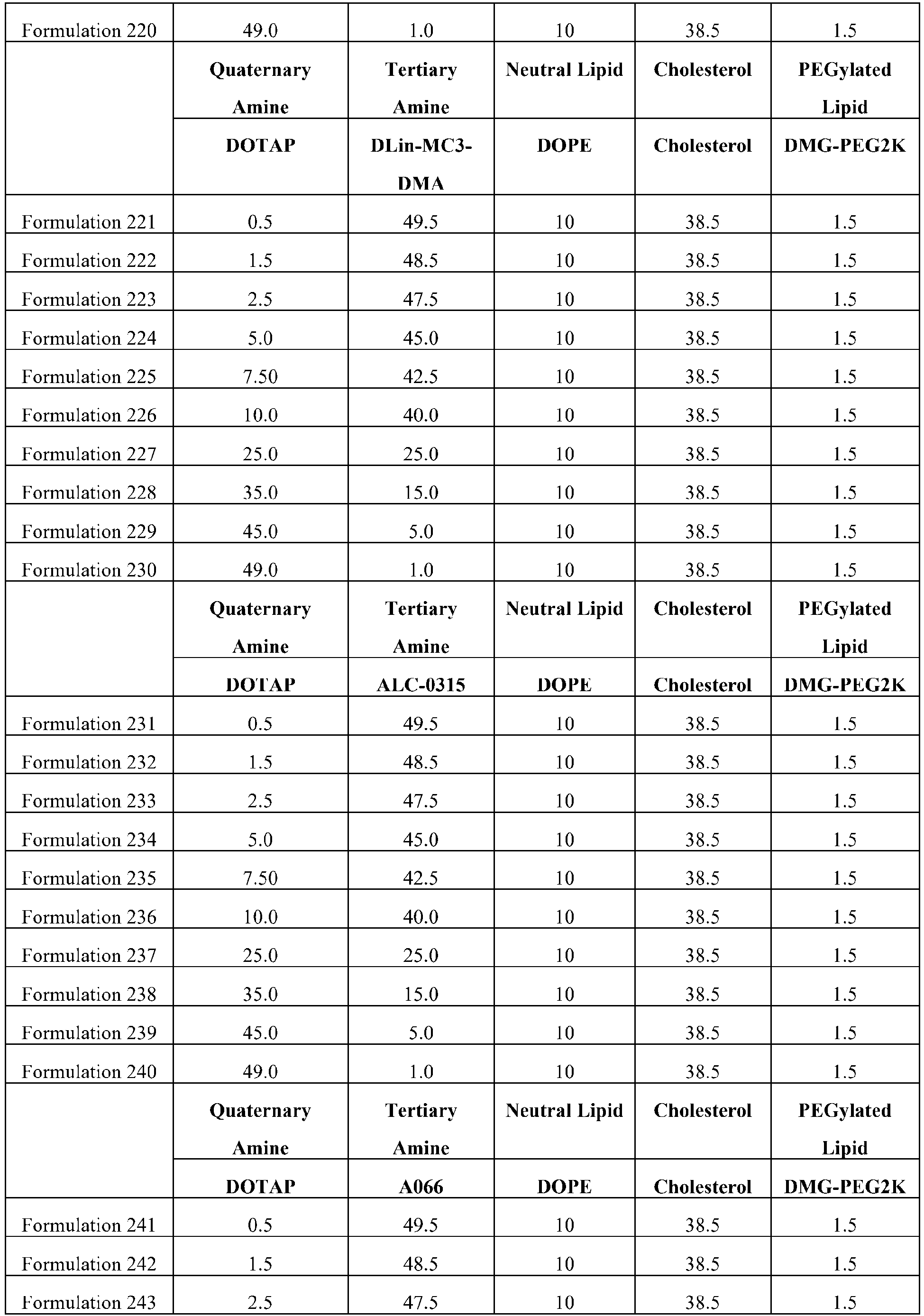

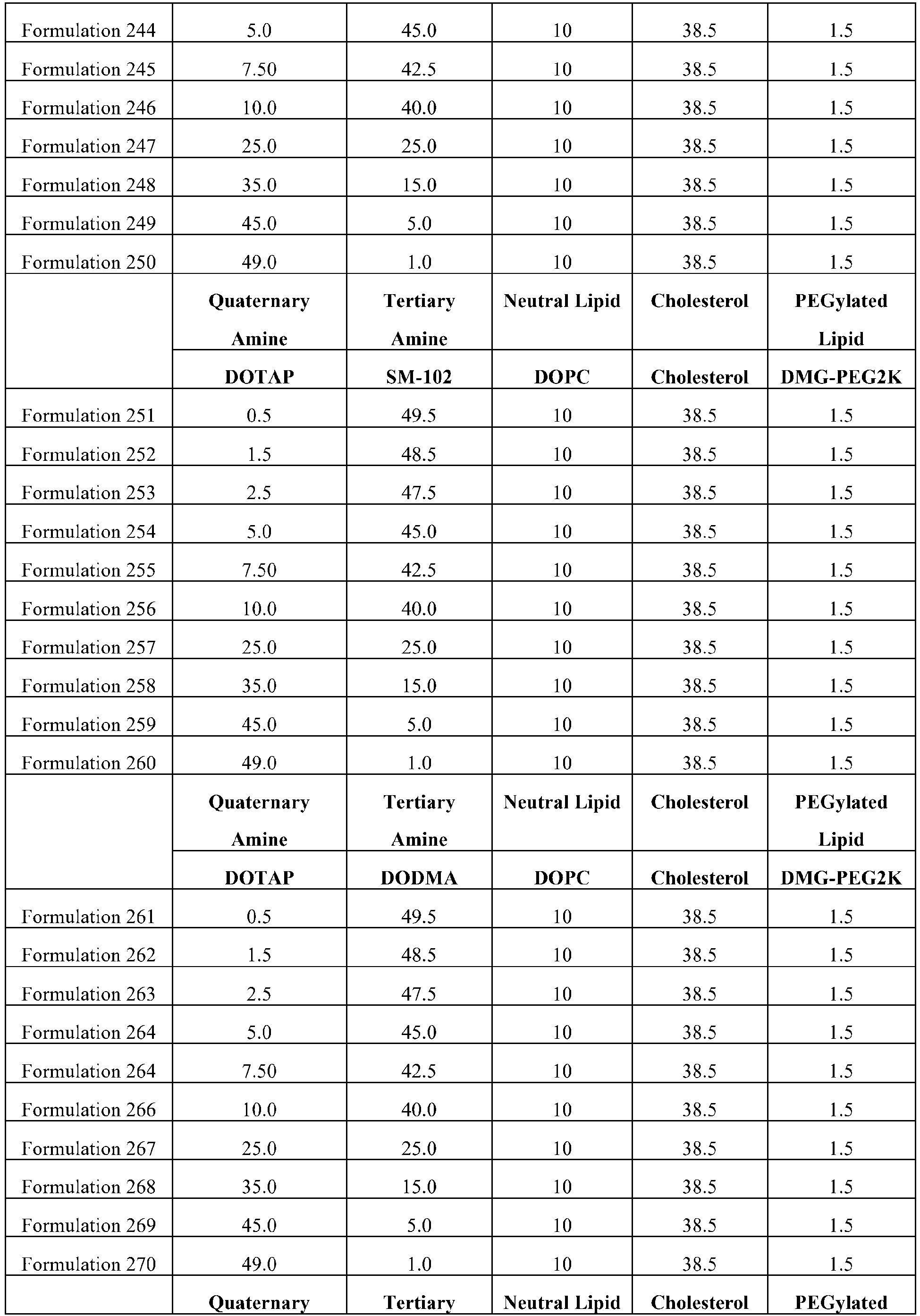

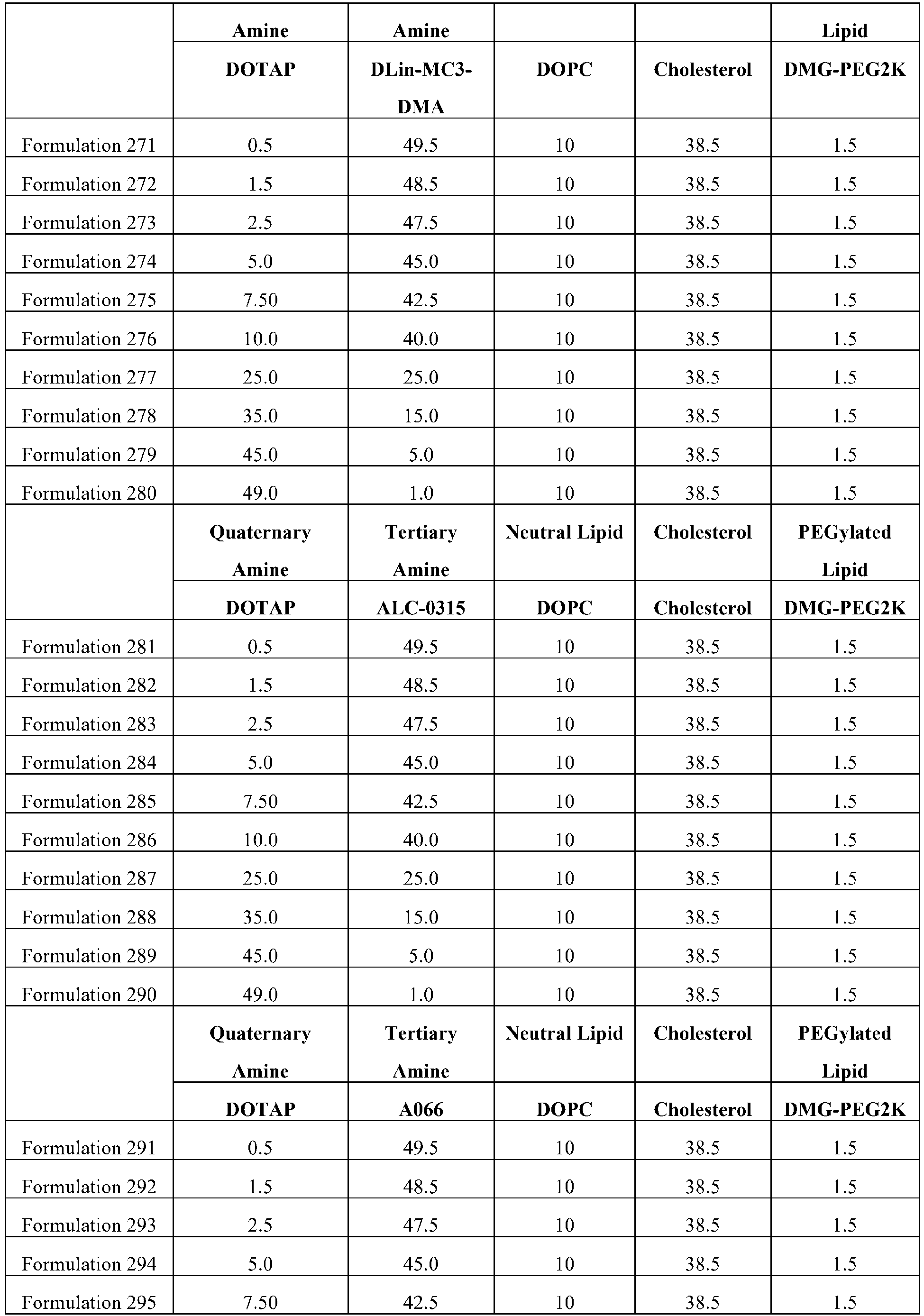

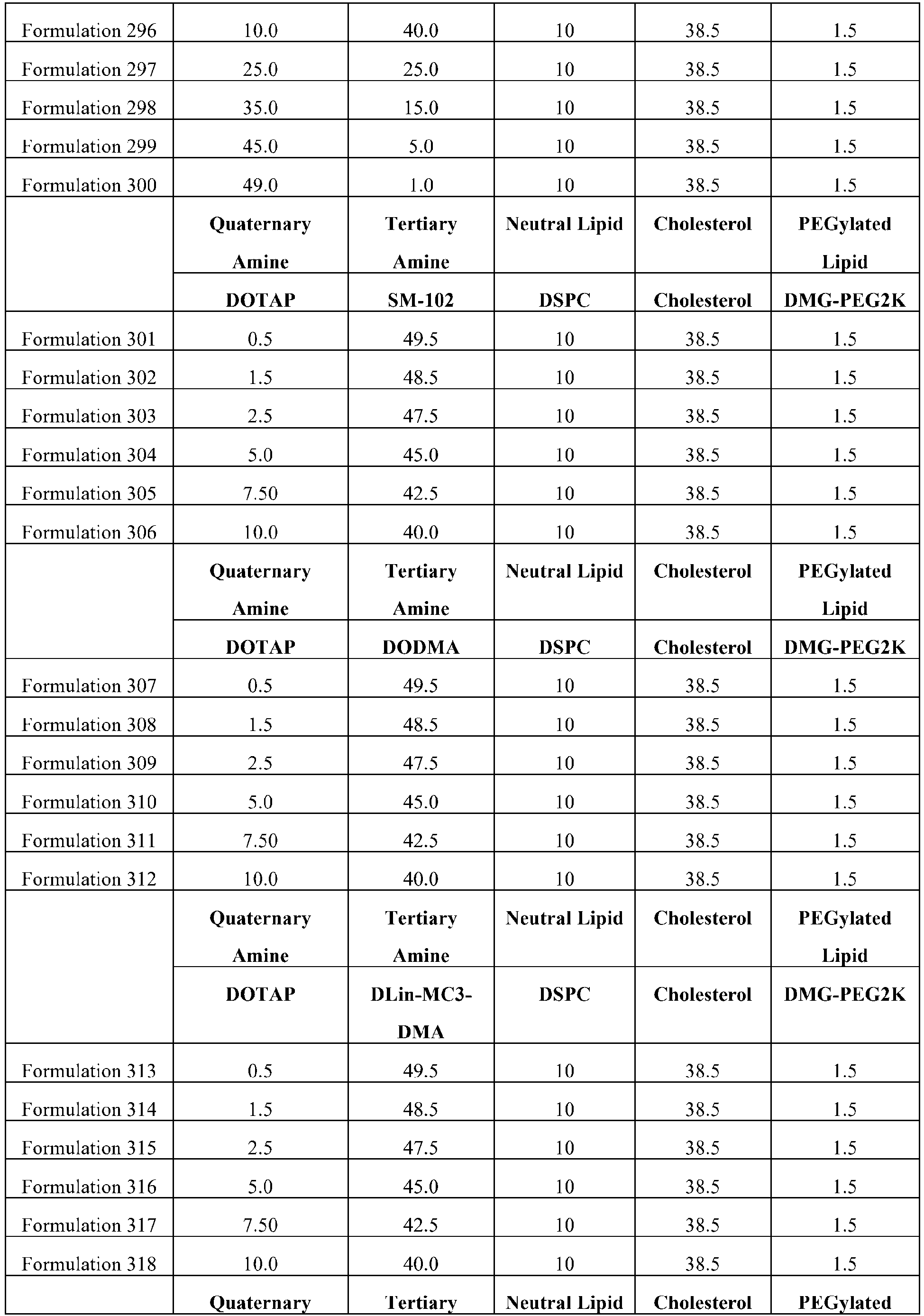

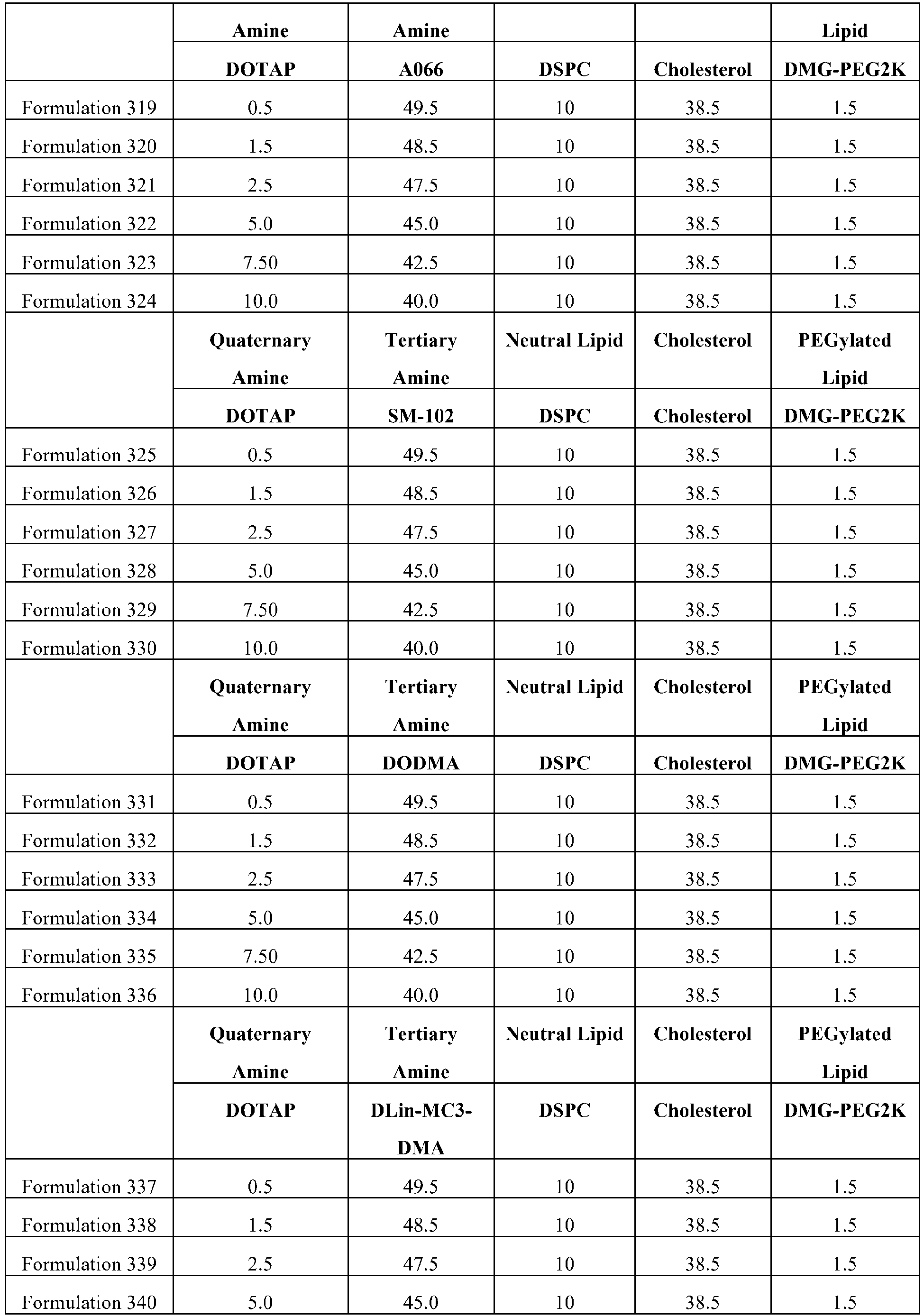

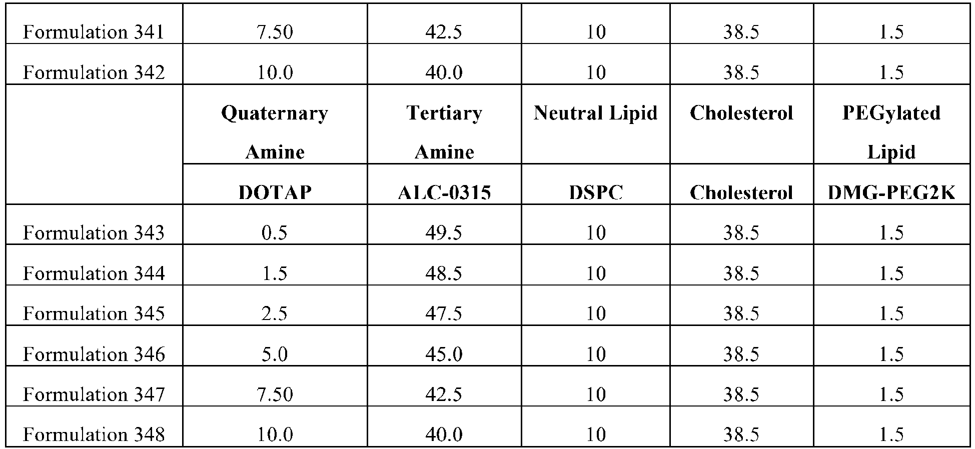

In some embodiments, the active component in the lipid nanoparticle delivery system of the present disclosure is at least one selected from the group consisting of GFP-mRNA, TP53 mRNA, Archexin, and doxorubicin. In some preferred embodiments, the mass ratio of the active component to the lipid carrier in the lipid nanoparticle delivery system is 1:5-1:30. In some more preferred embodiments, the mass ratio of the active component to the lipid carrier in the lipid nanoparticle delivery system is 1:10 or 1:20.

### Preparation Method

The present disclosure provides a method for modifying LNPs with targeting moieties on their surface, comprising the following steps (a)-(e): (a) preparing a solution I comprising an active component using a buffer and the active component; (b) preparing a lipid solution using an organic solvent and a lipid carrier; (c) mixing the lipid solution and the solution I, diluting to obtain an initial lipid nanoparticle solution; optionally repeating steps (a)-(c); (d) removing the organic solvent from the initial lipid nanoparticle solution, diluting and adjusting pH to 6.0-8.0 to obtain an active component-loaded lipid nanoparticle suspension; (e) preparing a targeting component solution, then mixing the active component-loaded lipid nanoparticle suspension and the targeting component solution. In a preferred embodiment, the active component-loaded lipid nanoparticle suspension and the targeting component solution are mixed before use. In one embodiment, the organic solvent is removed from the initial lipid nanoparticle solution by ultrafiltration and/or dialysis in step (d). In some embodiments, the solution I is prepared using an active component and any commonly used buffer with a pH of 3.0-6.0, such as sodium acetate buffer, citrate buffer, Tris buffer, or PBS buffer. In some embodiments, the organic solvent is ethanol. In some embodiments, the targeting component solution is prepared by dissolving the targeting component in water, sodium acetate buffer, citrate buffer, Tris buffer, or PBS buffer. In some embodiments, the solution I comprising the active component and the lipid solution are pre-warmed to 20°C-30°C, e.g., 25°C, before mixing. In some embodiments, the organic solvent is removed from the initial lipid nanoparticle solution by dialysis, ultrafiltration, tangential flow filtration, etc. In some embodiments, the organic solvent is removed from the initial lipid nanoparticle solution by ultrafiltration.

The present disclosure provides a method for preparing the lipid composition of the present disclosure, comprising the following steps (a)-(e): (a) preparing a solution I comprising an active component using a buffer and the active component; (b) preparing a lipid solution using an organic solvent and a lipid carrier; (c) mixing the lipid solution and the solution I, diluting to obtain an initial lipid nanoparticle solution; optionally repeating steps (a)-(c); (d) removing the organic solvent from the initial lipid nanoparticle solution, diluting and adjusting pH to 6.0-8.0 to obtain an active component-loaded lipid nanoparticle suspension; (e) preparing a targeting component solution, then mixing the active component-loaded lipid nanoparticle suspension and the targeting component solution. In one embodiment, the organic solvent is removed from the initial lipid nanoparticle solution by ultrafiltration and/or dialysis in step (d). In some embodiments, the solution I is prepared using an active component and any commonly used buffer with a pH of 3.0-6.0, such as sodium acetate buffer, citrate buffer, Tris buffer, or PBS buffer. In some embodiments, the organic solvent is ethanol. In some embodiments, the targeting component solution is prepared by dissolving the targeting component in water, sodium acetate buffer, citrate buffer, Tris buffer, or PBS buffer. In some embodiments, the solution I comprising the active component and the lipid solution are pre-warmed to 20°C-30°C, e.g., 25°C, before mixing. In some embodiments, the organic solvent is removed from the initial lipid nanoparticle solution by dialysis, ultrafiltration, tangential flow filtration, etc. In some embodiments, the organic solvent is removed from the initial lipid nanoparticle solution by ultrafiltration.

The present disclosure provides a method for preparing the lipid nanoparticle delivery system of the present disclosure, comprising the following steps (a)-(d) of process I, or steps (e)-(i) of process II: Process I: (a) preparing a solution I comprising an active component using a buffer and the active component, (b) preparing a lipid solution using an organic solvent and a lipid carrier, (c) mixing the lipid solution and the solution I, diluting to obtain an initial lipid nanoparticle (LNP) solution, optionally repeating steps (a)-(c), (d) removing the organic solvent from the initial LNP solution, diluting and adjusting pH to 6.0-8.0, to obtain an active component-loaded LNP suspension; Process II: (e) preparing a lipid solution using an organic solvent and a lipid carrier, (f) preparing an initial blank LNP solution using a buffer and the lipid solution, optionally repeating steps (e)-(f), (g) removing the organic solvent from the initial LNP solution, diluting and adjusting pH to 6.0-8.0, to obtain a blank LNP suspension, (h) preparing a solution I comprising an active component using a buffer and the active component, (i) mixing the solution I and the blank LNP suspension, then adjusting pH to 6.0-8.0, to obtain an active component-loaded LNP suspension.

In some embodiments, when the active component is a nucleic acid or small nucleic acid, steps (a)-(d) of process I are used to prepare the lipid nanoparticle delivery system of the present disclosure. In some embodiments, when the active component is a small molecule drug, steps (e)-(i) of process II are used to prepare the lipid nanoparticle delivery system of the present disclosure.

In some preferred embodiments, the organic solvent is removed from the initial LNP solution by ultrafiltration and/or dialysis.

In some embodiments, the solution I is prepared using an active component and any commonly used buffer with a pH of 3.0-6.0, such as sodium acetate buffer, citrate buffer, Tris buffer, or PBS buffer. In some embodiments, the organic solvent is ethanol. In some embodiments, the solution I comprising the active component and the lipid solution are pre-warmed to 20°C-30°C, e.g., 25°C, before mixing. In some embodiments, the organic solvent is removed from the initial lipid nanoparticle solution by dialysis, ultrafiltration, tangential flow filtration, etc. In some embodiments, the organic solvent is removed from the initial lipid nanoparticle solution by ultrafiltration.

### Pharmaceutical Composition

The present disclosure provides a pharmaceutical composition comprising the lipid composition of the present disclosure and one or more pharmaceutically acceptable carriers or excipients. The present disclosure also provides a pharmaceutical composition comprising the lipid nanoparticle delivery system of the present disclosure and one or more pharmaceutically acceptable carriers or excipients.

In some embodiments, the pharmaceutical composition of the present disclosure further comprises a second therapeutic agent.

In some embodiments, the second therapeutic agent is one or more selected from the group consisting of polypeptides, proteins, antibodies, chemotherapeutic drugs, chemical drugs, nucleic acid drugs, and small molecule drugs. In some embodiments, the second therapeutic agent is one or more selected from the group consisting of polyinosinic:polycytidylic acid (Poly(I:C)), STING agonists, imiquimod, resiquimod, gardiquimod, sorafenib, sunitinib, erlotinib, doxorubicin, paclitaxel, gemcitabine, and radiotherapy.

In some embodiments, antibodies include but are not limited to antibodies with anti-tumor effects. Antibodies with anti-tumor effects include but are not limited to antibodies targeting PD-1, such as nivolumab, pembrolizumab, cemiplimab, toripalimab, sintilimab, camrelizumab, etc.; antibodies targeting PD-L1, such as atezolizumab, avelumab, and durvalumab, etc.; antibodies targeting CTLA4, such as ipilimumab, tremelimumab, etc. In some embodiments, antibodies include but are not limited to antibodies against tumor-associated antigens (TAAs). TAAs include but are not limited to GPC, CEA, immature laminin receptor, TAG-72, HPV E6, HPV E7, EGFR, Ep-CAM, EphA3, Her2, Her3, ROR2, PSMA, STEAP1, FGFR2, TROP2, B7-H3, B7-H4, B7-H6, FOLR1, BAGE family, CAGE family, GAGE family, MAGE family, SAGE family, XAGE family, SSX-2, fibronectin, MART-2, PDL-1, PD-1, CTLA4, VEGFR, CLAUDIN, etc.

In some embodiments, chemotherapeutic drugs include but are not limited to doxorubicin, paclitaxel, gemcitabine, and radiotherapy.

In some embodiments, small molecule or nucleic acid drugs include but are not limited to pattern recognition receptor agonists, such as Toll-like receptor agonists, anthracyclines, camptothecins, anti-tumor antibiotics, immunogenic cell death (ICD) inducers, tyrosine kinase inhibitors, taxanes, Bruton's tyrosine kinase (BTK) inhibitors, PI3K inhibitors, HDAC inhibitors, ERK inhibitors, MAPK inhibitors, PD-1/PD-L1 inhibitors, CTLA-4 inhibitors, TIGIT inhibitors, TIM3 inhibitors, Akt-1 inhibitors, EGFR inhibitors, VEGF inhibitors, PARP inhibitors, Her2 inhibitors, LAG-3 inhibitors, TNFR2 inhibitors, etc.

In some embodiments, anthracyclines include but are not limited to doxorubicin, epirubicin, pirarubicin, daunorubicin, aclarubicin, idarubicin, amrubicin, etc.

In some embodiments, camptothecins include but are not limited to topotecan, irinotecan, belotecan, exatecan (DX-8951), lurtotecan, sinotecan, rubitecan (9-NC), 9-aminocamptothecin (9-AC), gimatecan, karenitecin, DB-67, etc.

In some embodiments, tyrosine kinase inhibitors include but are not limited to sorafenib, sunitinib, gefitinib, erlotinib, lapatinib, afatinib, dacomitinib, vandetanib, neratinib, pelitinib (EKB-569), canertinib (CI-1033), osimertinib, rociletinib (CO-1686, Clovis Oncology), olmutinib (HM61713, Hanmi Pharmaceutical), naquotinib (ASP8273, Astellas), tesevatinib (XL647/KD019, Kadmon Corporation), nazartinib (EGF816, Novartis), and PF-06747775 (Pfizer), etc.

In some embodiments, immunogenic cell death inducers include but are not limited to DNA damaging agents, bleomycin, etc.

In some embodiments, taxanes include but are not limited to paclitaxel, docetaxel, etc.

In some embodiments, pattern recognition receptor agonists include but are not limited to Toll-like receptors, RIG-I-like receptors, Nod-like receptors, AIM2-like receptors, and C-type lectin receptors, as well as cGas and other intracellular DNA sensor agonists.

As used herein, the term "pharmaceutical composition" refers to a mixture of the lipid composition of the present disclosure, the lipid composition and the second therapeutic agent with other chemical components such as carriers, stabilizers, diluents, dispersants, suspending agents, thickeners, and/or excipients. The pharmaceutical composition facilitates the administration of the lipid composition, the lipid composition and the second therapeutic agent to an organism. There are various ways known in the art for administering pharmaceutical compositions, including but not limited to, intratumoral injection, intravenous injection, subcutaneous injection, intramuscular injection, intra-arterial injection, intraperitoneal injection, intra-central-nervous-system injection, intracavitary instillation, such as intrathecal injection, intravesical instillation, interventional therapy, oral, transdermal, pulmonary, ocular, and topical administration.

As used herein, the pharmaceutical composition can be formulated into a dosage form suitable for administration to a subject via the desired route of administration. The dosage forms include but are not limited to tablets, capsules, caplets, pills, lozenges, powders, syrups, elixirs, suspensions, solutions, emulsions, transdermal patches, suppositories, inhalants, creams, ointments, lotions, pastes, sprays, lyophilized formulations, injections, and gels, etc.

The term "pharmaceutically acceptable carrier" includes pharmaceutically acceptable materials, compositions, or carriers, such as liquid or solid fillers, diluents, excipients, solvents, or encapsulating materials, which are involved in carrying or transporting the lipid composition of the present disclosure, the lipid composition and the second therapeutic agent within or to a subject, enabling it to perform its intended function. Each salt or carrier must be "acceptable" in the sense of being compatible with the other ingredients of the formulation and not harmful to the subject. Some examples of materials that can be used as pharmaceutically acceptable carriers include: sugars, such as lactose, glucose, and sucrose; starches, such as corn starch and potato starch; cellulose and its derivatives, such as sodium carboxymethylcellulose, ethyl cellulose, and cellulose acetate; powdered tragacanth; malt; gelatin; talc; excipients, such as cocoa butter and suppository waxes; oils, such as peanut oil, cottonseed oil, safflower oil, sesame oil, olive oil, corn oil, and soybean oil; glycols, such as propylene glycol; polyols, such as glycerol, sorbitol, mannitol, and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethanol; phosphate buffer solutions; diluents; granulating agents; lubricants; binders; disintegrants; wetting agents; emulsifiers; coloring agents; release agents; coating agents; sweeteners; flavoring agents; perfuming agents; preservatives; antioxidants; plasticizers; gelling agents; thickeners; hardeners; setting agents; suspending agents; surfactants; humectants; carriers; stabilizers; and other non-toxic compatible substances used in pharmaceutical formulations, or any combination thereof.

### Medical Uses and Methods

The present disclosure provides a use of the lipid composition of the present disclosure in the manufacture of a medicament for treating or preventing a disease or a disease diagnostic agent. The present disclosure also provides a use of the lipid nanoparticle delivery system of the present disclosure in the manufacture of a medicament for treating or preventing a disease or a disease diagnostic agent.

In some embodiments, the disease is one or more selected from the group consisting of tumors, immune diseases, inflammatory diseases, infectious diseases, cardiovascular and cerebrovascular diseases, and endocrine diseases.

Tumors include but are not limited to solid tumors and hematological tumors. Solid tumors include but are not limited to oral cancer, esophageal cancer, gastric cancer, pancreatic cancer, thyroid cancer, nasopharyngeal cancer, colon cancer, rectal cancer, glioma, melanoma, lung cancer, liver cancer, breast cancer, spleen cancer, head and neck cancer, non-melanoma skin cancer, renal cell carcinoma, cervical cancer, uterine cancer, ovarian cancer, fallopian tube cancer, prostate cancer, bladder cancer, bone cancer, osteosarcoma, chondroma, liposarcoma, neuroblastoma, synovial sarcoma, astrocytoma, glioblastoma multiforme, brain tumor, anaplastic astrocytoma, peritoneal cavity cancer, soft tissue sarcoma, sarcoma, rhabdomyosarcoma, advanced myxoid disease. Hematological tumors include but are not limited to myelodysplastic syndrome, leukemia, acute myeloid leukemia, chronic myeloid leukemia, acute lymphocytic leukemia, chronic lymphocytic leukemia, Hodgkin's lymphoma, non-Hodgkin's lymphoma, Hodgkin's disease, myeloma, multiple myeloma.

Inflammatory diseases or immune diseases include but are not limited to autoimmune diseases, delayed-type hypersensitivity, allergic diseases, lupus, graft-versus-host disease, vasculitis caused by hepatitis C, type I diabetes, type II diabetes, multiple sclerosis, rheumatoid arthritis, alopecia areata, atherosclerosis, psoriasis, organ transplant rejection, Sjögren's syndrome, Behçet's disease, spontaneous abortion, atopic diseases, asthma, and inflammatory bowel disease.

Infectious diseases include but are not limited to fungal infectious diseases, bacterial infectious diseases, mycoplasma or chlamydia infectious diseases, viral infectious diseases, such as viral hepatitis.

Cardiovascular and cerebrovascular diseases include but are not limited to atherosclerosis, hypertension, hyperlipidemia, heart failure, myocardial infarction, coronary heart disease, cerebral infarction, and cerebral thrombosis.

Endocrine diseases include but are not limited to growth hormone deficiency, hyperthyroidism, hypothyroidism, diabetes, female menopause syndrome, polycystic ovary syndrome, and Cushing's syndrome.

The present disclosure provides a method for preventing or treating a disease, comprising administering a therapeutically or prophylactically effective amount of the lipid composition or pharmaceutical composition of the present disclosure to a subject in need thereof. In some embodiments, the lipid composition of the present disclosure is administered together with an additional medicament. In some preferred embodiments, the lipid composition of the present disclosure can be administered sequentially or simultaneously with an additional medicament. In some embodiments, the route of administration of the lipid composition of the present disclosure and the additional medicament can be the same or different. In some embodiments, the lipid composition of the present disclosure is administered before the additional medicament. In some embodiments, the lipid composition of the present disclosure is administered after the additional medicament. In some embodiments, the lipid composition of the present disclosure is administered simultaneously with the additional medicament. In some preferred embodiments, prior to administering a therapeutically or prophylactically effective amount of the lipid composition of the present disclosure to a subject in need thereof, the active component-loaded lipid nanoparticle suspension and the targeting component solution are mixed as in the preparation process of the lipid composition of the present disclosure.

The present disclosure also provides a method for preventing or treating a disease, comprising administering a therapeutically or prophylactically effective amount of the lipid nanoparticle delivery system or pharmaceutical composition of the present disclosure to a subject in need thereof. In some embodiments, the lipid nanoparticle delivery system of the present disclosure is administered together with an additional medicament. In some preferred embodiments, the lipid nanoparticle delivery system of the present disclosure can be administered sequentially or simultaneously with an additional medicament. In some embodiments, the route of administration of the lipid nanoparticle delivery system of the present disclosure and the additional medicament can be the same or different. In some embodiments, the lipid nanoparticle delivery system of the present disclosure is administered before the additional medicament. In some embodiments, the lipid nanoparticle delivery system of the present disclosure is administered after the additional medicament. In some embodiments, the lipid nanoparticle delivery system of the present disclosure is administered simultaneously with the additional medicament.

The additional medicament includes but is not limited to one or more of polypeptides, proteins, antibodies, chemotherapeutic drugs, chemical drugs, small molecule drugs, and nucleic acid drugs.

In some embodiments, the lipid composition, lipid nanoparticle delivery system, or pharmaceutical composition of the present disclosure is administered via one or more of the following routes: intratumoral injection, intravenous injection, subcutaneous injection, intramuscular injection, intra-arterial injection, intraperitoneal injection, intra-central-nervous-system injection, intracavitary instillation, intravesical instillation, interventional therapy, oral, transdermal, pulmonary, ocular, and topical administration.

In some embodiments, the disease is one or more selected from the group consisting of tumors, immune diseases, inflammatory diseases, and infectious diseases.

Tumors include but are not limited to solid tumors and hematological tumors. Solid tumors include but are not limited to oral cancer, esophageal cancer, gastric cancer, pancreatic cancer, thyroid cancer, nasopharyngeal cancer, colon cancer, rectal cancer, glioma, melanoma, lung cancer, liver cancer, breast cancer, spleen cancer, head and neck cancer, non-melanoma skin cancer, renal cell carcinoma, cervical cancer, uterine cancer, ovarian cancer, fallopian tube cancer, prostate cancer, bladder cancer, bone cancer, osteosarcoma, chondroma, liposarcoma, neuroblastoma, synovial sarcoma, astrocytoma, glioblastoma multiforme, brain tumor, anaplastic astrocytoma, peritoneal cavity cancer, soft tissue sarcoma, sarcoma, rhabdomyosarcoma, advanced myxoid disease. Hematological tumors include but are not limited to myelodysplastic syndrome, leukemia, acute myeloid leukemia, chronic myeloid leukemia, acute lymphocytic leukemia, chronic lymphocytic leukemia, Hodgkin's lymphoma, non-Hodgkin's lymphoma, Hodgkin's disease, myeloma, multiple myeloma.

Inflammatory diseases or immune diseases include but are not limited to autoimmune diseases, delayed-type hypersensitivity, allergic diseases, lupus, graft-versus-host disease, vasculitis caused by hepatitis C, type I diabetes, type II diabetes, multiple sclerosis, rheumatoid arthritis, alopecia areata, atherosclerosis, psoriasis, organ transplant rejection, Sjögren's syndrome, Behçet's disease, spontaneous abortion, atopic diseases, asthma, and inflammatory bowel disease.

Infectious diseases include but are not limited to fungal infectious diseases, bacterial infectious diseases, mycoplasma or chlamydia infectious diseases, viral infectious diseases, such as viral hepatitis.

As used herein, the term "subject" includes animals, such as vertebrates, preferably mammals, such as dogs, cats, pigs, cattle, sheep, horses, rodents (e.g., mice, rats, or guinea pigs) or primates (e.g., gorillas, chimpanzees, and humans).

As used herein, the term "treat" or "treating" refers to alleviating or ameliorating a disease or disorder (i.e., slowing or halting the development of the disease or at least one clinical symptom thereof); or alleviating or ameliorating at least one physical parameter or biomarker associated with the disease or disorder.

As used herein, the term "therapeutically effective dose" refers to an amount that, compared to a corresponding subject not receiving that amount, results in benefit from or treatment of the disease, but this amount is sufficiently low within the scope of reasonable medical judgment to avoid serious side effects. The therapeutically effective dose described herein will vary depending on factors such as the chosen lipid composition, lipid nanoparticle delivery system, or pharmaceutical composition; route of administration; severity of the disease being treated; age, size, weight, and physical condition of the patient being treated; medical history of the patient being treated; duration of treatment; nature of concurrent therapy; desired therapeutic effect, etc., but can still be determined by those skilled in the art in a conventional manner.

As used herein, the term "prevent" includes providing protection against the occurrence or recurrence of a disease for individuals who may be predisposed to the disease but have not yet been diagnosed with the disease.

As used herein, the term "prophylactically effective dose" refers to an amount that can prevent the disease compared to a corresponding subject not receiving that amount, and this amount is sufficiently low within the scope of reasonable medical judgment to avoid serious side effects. The prophylactically effective dose described herein will vary depending on factors such as the chosen lipid composition, lipid nanoparticle delivery system, or pharmaceutical composition; route of administration; severity of the disease to be prevented; age, size, weight, and physical condition of the subject; desired prophylactic effect, etc., but can still be determined by those skilled in the art in a conventional manner.

The present disclosure provides a method for transfecting a host cell, comprising administering the lipid composition of the present disclosure to the host cell. The present disclosure also provides a method for transfecting a host cell, comprising administering the lipid nanoparticle delivery system of the present disclosure to the host cell.

The host cells can be animal cells (e.g., mammalian cells), plant cells, yeast cells, and/or bacterial cells. Host cells can be *in vivo* cells and/or *in vitro* cells. In some embodiments, the host cells are immune cells or tissue cells. In some embodiments, the host cells are human cells, such as human immune cells (e.g., human macrophages, human T cells, human NK cells, etc.), human tissue cells (e.g., human liver cells, human heart cells, human spleen cells, human lung cells, human kidney cells, etc.). In some embodiments, host cells include, for example, CHO cells, such as CHOS cells and CHO-K1 cells, HEK293 cells, such as HEK293A, HEK293T, and HEK293FS. In some embodiments, the host cells are one or more selected from the group consisting of *in vivo* and *in vitro* cells, including but not limited to mouse macrophages (e.g., mouse RAW264.7), macrophages in PBMC, T cells in PBMC, *in vivo* splenic macrophages, *in vivo* splenic T cells, and *in vivo* splenic natural killer cells.

The present disclosure provides a method for targeting delivery of an active component, comprising administering the lipid composition or lipid nanoparticle delivery system of the present disclosure to a subject or a biological sample.

As used herein, "biological sample" includes but is not limited to body fluid samples from a subject, such as blood samples, saliva samples; tissue samples; lesion samples, tumor samples, organ samples, heart samples, liver samples, spleen samples, lung samples, kidney samples, bone samples, muscle samples, soft tissue samples.

The various embodiments described above for the lipid composition or lipid nanoparticle delivery system of the present disclosure are also applicable to the pharmaceutical compositions, uses, and methods of the present disclosure (as long as they are not intrinsically contradictory to each other), and thus the various embodiments formed by combination are considered part of the present disclosure.

### Examples

The following describes exemplary embodiments of the present application in conjunction with the figures, including various details of the embodiments of the present application to aid understanding. It should be understood that they are considered merely exemplary and are not intended to limit the scope of protection of the present application. The scope of protection of the present application is defined only by the claims. Therefore, those of ordinary skill in the art will recognize that various changes and modifications can be made to the embodiments described herein without departing from the scope of the present application. Similarly, descriptions of known functions and structures are omitted from the following description for clarity and conciseness.

The experimental methods in the following examples, unless otherwise specified, are conventional methods performed according to the techniques or conditions described in the literature in the field or according to product instructions. The materials, reagents, etc., used in the following examples, unless otherwise specified, are commercially available.

### Examples 1-6. Preparation of Targeting Lipid Compositions

The targeting lipid compositions prepared in Examples 1-6 comprised the small nucleic acid Archexin (RX-0201, provided by Rexahn Pharmaceuticals) as the active component, a 20-oligonucleotide small molecule nucleic acid complementary to AKT1 mRNA, which can directly act on AKT to inhibit its production; transferrin as the targeting component; and a lipid carrier based on a quaternary amine-tertiary amine lipid nanoparticle system (QTsome) using DOTAP (quaternary amine) and SM-102 (tertiary amine). The specific molar percentages of lipid components are shown in Table 1.

**Table 1. Lipid Compositions of the Lipid Carrier in Examples 1-6**

| | **Positively Charged Lipid** | **Ionizable Lipid** | **Neutral Phospholipid** | **Non-Phospholipid Neutral Lipid** | **PEGylated Lipid** |
|---|---|---|---|---|---|
| | **DOTAP** | **SM-102** | **DSPC** | **Cholesterol** | **DMG-PEG2000** |
| **Example 1** | 2.5 (mol%) | 47.5 | 10 | 38.5 | 1.5 |
| **Example 2** | 5.0 | 45.0 | 10 | 38.5 | 1.5 |
| **Example 3** | 7.5 | 42.5 | 10 | 38.5 | 1.5 |
| **Example 4** | 10.0 | 40.0 | 10 | 38.5 | 1.5 |
| **Example 5** | 25.0 | 25.0 | 10 | 38.5 | 1.5 |
| **Example 6** | 45.0 | 5.0 | 10 | 38.5 | 1.5 |

The preparation method included the following steps:
(1) An Archexin sodium acetate solution was prepared using the small nucleic acid Archexin and sodium acetate buffer (pH=5.0). This solution had a pH=5.0, Archexin concentration of 0.3334 mg/mL, and sodium acetate concentration of 25 mM;
(2) A lipid ethanol solution with a total lipid concentration of 20 mg/mL was prepared using the lipids listed in Table 1 and anhydrous ethanol;
(3) 0.75 mL of the Archexin sodium acetate solution prepared in step (1) (nucleic acid concentration 0.3334 mg/mL, total amount 0.25 mg) and 0.25 mL of the lipid ethanol solution prepared in step (2) (total lipid concentration 20 mg/mL, total amount 5 mg) were pre-warmed in a 25°C water bath. Then, under magnetic stirring in a 25°C water bath, the lipid ethanol solution was injected into the Archexin sodium acetate solution, incubated for 1 min, and then diluted to 4 times its volume with sodium acetate buffer (pH=5.0, concentration 25 mM) to obtain the initial Archexin-loaded LNP solution. Steps (1)-(3) could be repeated to combine and obtain more initial Archexin-loaded LNP solution;
(4) Using an ultrafiltration tube with a molecular weight cutoff of 100 KD, ethanol was removed from the initial LNP solution by centrifugation ultrafiltration at 4°C with a centrifugal acceleration of 2000g. The nucleic acid concentration was adjusted to an appropriate level using 25 mM Tris buffer, and the pH was adjusted to 7.0 using 0.1 M sodium hydroxide solution to obtain the nucleic acid-loaded lipid nanoparticle (stock solution);
(5) A 5 mg/mL transferrin solution was prepared using Transferrin and deionized water, or a human serum albumin solution was prepared using human serum albumin. 0.5 mL of the Archexin-loaded LNP suspension prepared in step (4) (total lipid amount 1.25 mg) was taken, and 0.025 mL of the 5 mg/mL transferrin or human serum albumin solution was added. After stirring in a 25°C water bath for 30 min, the targeting lipid composition (denoted as Archexin-LNPs-Trf) was obtained. The Archexin-loaded LNP suspension and the transferrin solution or human serum albumin solution were mixed before use of the targeting lipid composition Archexin-LNPs-Trf.

### Example 7. Characterization of Lipid Nanoparticles

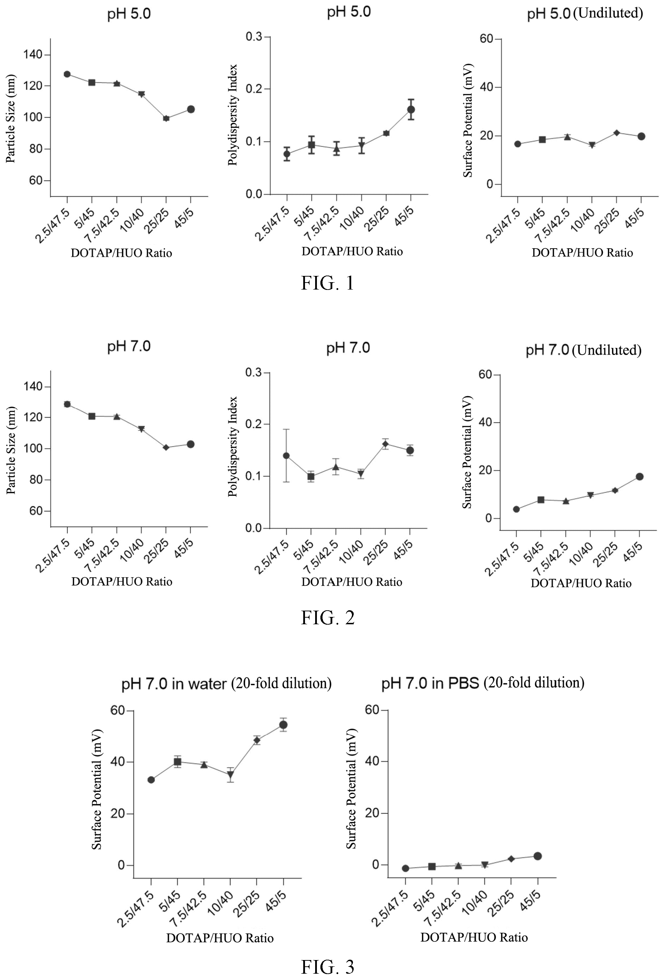

The particle size, polydispersity index (PDI), and zeta potential of the nucleic acid-loaded lipid nanoparticles (stock solution) prepared in Examples 1-6 were measured in Tirs buffer at pH 5.0, and the results are shown in Figure 1. The results in Figure 1 show that in Tirs buffer at pH 5.0, as the DOTAP/SM-102 ratio increased, the size of the lipid nanoparticles gradually decreased, the PDI slightly increased, and the zeta potential for different DOTAP/SM-102 ratios was around 20 mV.

The particle size, PDI, and zeta potential of the nucleic acid-loaded lipid nanoparticles (stock solution) were measured in Tirs buffer at pH 7.0, and the results are shown in Figure 2. The results in Figure 2 show that in Tirs buffer at pH 7.0, as the DOTAP/SM-102 ratio increased, the size of the lipid nanoparticles gradually decreased, the PDI did not change significantly, and the zeta potential gradually increased, with a maximum value around 20 mV.

The zeta potential of the nucleic acid-loaded lipid nanoparticles (stock solution) prepared in Examples 1-6 was measured in Tirs buffer at pH 7.0 after 20-fold dilution with DEPC water or PBS, and the results are shown in Figure 3. The results in Figure 3 show that after 20-fold dilution with DEPC water, the zeta potential of the nanoparticles increased significantly. When the DOTAP/SM-102 ratio was 25/25, it could reach 48.5 mV, and when the DOTAP/SM-102 ratio was 45/5, it could reach 54.6 mV. After 20-fold dilution with PBS, the zeta potential of the lipid nanoparticles decreased significantly.

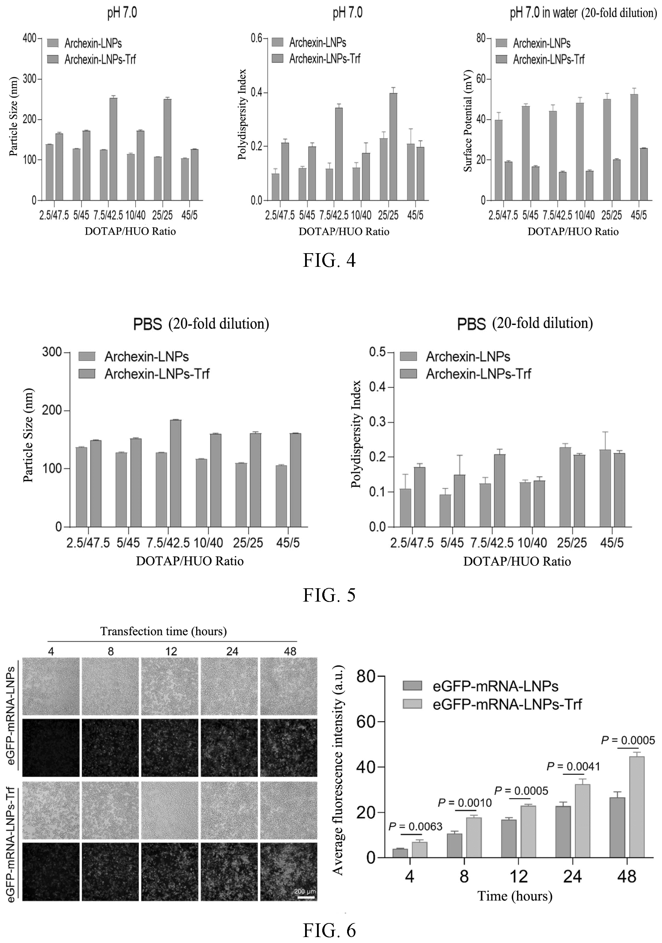

The particle size, PDI, and zeta potential of the targeted lipid compositions (labeled as Archexin-LNPs-Trf) prepared in Examples 1-6 were measured in Tirs buffer at pH 7.0 after 20-fold dilution with DEPC water or PBS, and the results are shown in Figures 4-5. The control used was Archexin-LNPs, i.e., the nucleic acid-loaded lipid nanoparticles prepared in step (4) of Examples 1-6. The results in Figure 4 show that after 20-fold dilution with DEPC water, the size of the nucleic acid-loaded lipid nanoparticles increased after adsorbing transferrin. When the DOTAP/SM-102 ratios were 2.5/47.5, 5/45, 10/40, and 45/5, the size of the lipid nanoparticles increased by about 20 nm after adsorbing transferrin, and the PDI did not change significantly. When the DOTAP/SM-102 ratios were 7.5/42.5 and 25/25, the size of the lipid nanoparticles increased by about 150 nm, and the PDI increased significantly. After adsorbing transferrin, the zeta potential decreased significantly for all DOTAP/SM-102 ratios. The results in Figure 5 show that after diluting the samples with PBS, as the DOTAP/SM-102 ratio increased, the size of the lipid nanoparticles increased after adsorbing transferrin, and the PDI did not change significantly. When the DOTAP/SM-102 ratio was 5/45, the size of the lipid nanoparticles increased by about 30 nm after adsorbing human serum albumin, and the PDI did not change significantly. This proves that the DOTAP/SM-102 ratio regulation strategy of the lipid nanoparticles provided by the present invention can adsorb multiple targeting proteins.

### Example 8. In Vivo and In Vitro Transfection and Tissue Targeting Experiments

### (1) Preparation of eGFP-mRNA-LNPs-Trf

Referring to the preparation method of Example 2, using eGFP mRNA (Nanjing Shenji Biotechnology Co., Ltd.) as the active component, lipid nanoparticles encapsulating eGFP mRNA were prepared (denoted as eGFP-mRNA-LNPs). Then, using transferrin (Trf) as the targeting component, eGFP-mRNA-LNPs and transferrin were mixed and incubated at a mass ratio of lipid nanoparticles:transferrin = 1:0.1 to obtain a targeting lipid composition (denoted as eGFP-mRNA-LNPs-Trf).

### (2) Preparation of LNPs-DIR-CD5

Referring to the preparation method of Example 2, using Archexin as the active component, DIR (a fluorescent dye, Beijing Lanbolide Trading Co., Ltd.) was mixed into the lipid mixture at a mass ratio of lipid nanoparticles:DIR = 1:0.01, followed by the addition of Archexin, to prepare lipid nanoparticles encapsulating Archexin (denoted as LNPs-DIR). Then, using a CD5 single-chain antibody (Biointron Biotechnology Co., Ltd.) as the targeting component, LNPs-DIR and the CD5 single-chain antibody were mixed and incubated at a mass ratio of lipid nanoparticles:CD5 single-chain antibody = 1:0.1 to obtain a targeting lipid composition (denoted as LNPs-DIR-CD5).

### (3) Preparation of eGFP-mRNA-LNPs-CD5

Referring to the preparation method of Example 2, using eGFP mRNA as the active component, lipid nanoparticles encapsulating eGFP mRNA were prepared (denoted as eGFP-mRNA-LNPs). Then, using a CD5 single-chain antibody as the targeting component, eGFP-mRNA-LNPs and the CD5 single-chain antibody were mixed and incubated at a mass ratio of lipid nanoparticles:CD5 single-chain antibody = 1:0.1 to obtain a targeting lipid composition (denoted as eGFP-mRNA-LNPs-CD5).

### (4) Preparation of CD19 CAR-mRNA-LNPs-CD5

Referring to the preparation method of Example 2, using CD19 CAR mRNA as the active component, lipid nanoparticles encapsulating CD19 CAR mRNA were prepared (denoted as CD19 CAR-mRNA-LNPs). Then, using a CD5 single-chain antibody as the targeting component, CD19 CAR-mRNA-LNPs and the CD5 single-chain antibody were mixed and incubated at a mass ratio of lipid nanoparticles:CD5 single-chain antibody = 1:0.1 to obtain a targeting lipid composition (denoted as CD19 CAR-mRNA-LNPs-CD5).

### (5) eGFP-mRNA-LNPs-Trf Transfection Experiment in Macrophages (Mouse RAW264.7 Cells)

Approximately 100,000 mouse RAW264.7 cells were seeded per well, and mRNA equivalent to a concentration of 5 µg/mL was added per well. Pre-warmed eGFP-mRNA-LNPs and eGFP-mRNA-LNPs-Trf were added dropwise to the wells, shaken to mix, and the cells were cultured further. Imaging was performed using a Leica fluorescence microscope at 4, 8, 12, 24, and 48 hours, and fluorescence intensity was quantified and statistically analyzed using ImageJ software. The results are shown in Figure 6.

The results in Figure 6 show that as transfection time increased, GFP green fluorescence intensity gradually increased, and the transfection efficiency of both eGFP-mRNA-LNPs and eGFP-mRNA-LNPs-Trf significantly improved. After 4-48 hours of transfection, the transfection efficiency of transferrin-modified lipid nanoparticles (eGFP-mRNA-LNPs-Trf) was significantly better than that of unmodified lipid nanoparticles (eGFP-mRNA-LNPs), demonstrating that transferrin adsorption can enhance the transfection efficiency of LNPs.

### (6) eGFP-mRNA-LNPs-Trf In Vitro Transfection Experiment in Macrophages within PBMCs

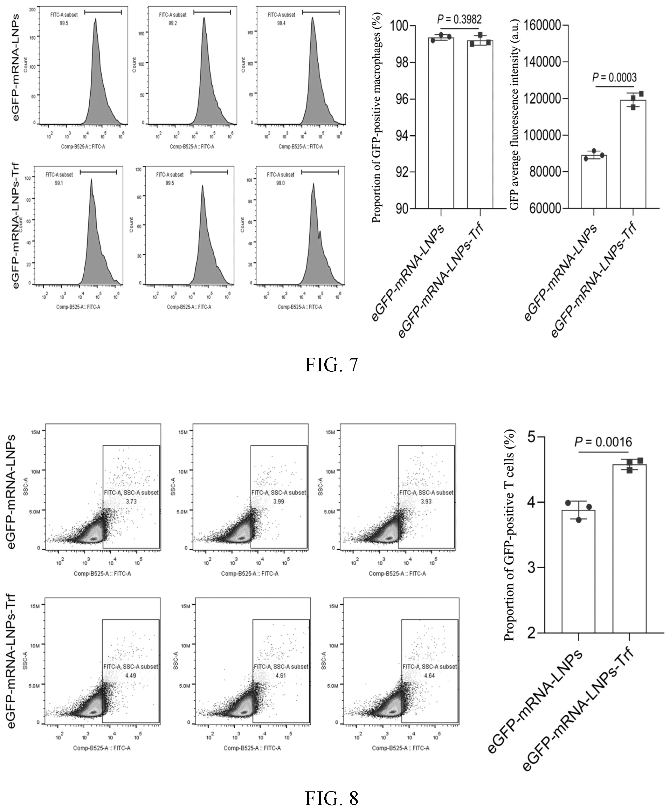

mRNA equivalent to a concentration of 1 µg/mL was added per well (containing approximately 100,000 PBMCs (Shanghai Saili Biotechnology Co., Ltd.) per well). Pre-warmed eGFP-mRNA-LNPs and eGFP-mRNA-LNPs-Trf were added dropwise to the wells, shaken to mix, and the PBMC cells were cultured for a further 24 h. PBMC cells were collected, centrifuged at 1500 r/min for 5 min, and filtered through a 70 µm cell strainer. Macrophages were then labeled by staining with PE Mouse Anti-Human CD11b (ICRF44) and BV786 Mouse Anti-Human CD14 (M5E2) for 45 min, followed by centrifugation and washing three times. Finally, changes in the proportion of GFP-positive macrophages were detected using an Agilent flow cytometer and statistically analyzed. The results are shown in Figure 7.

The results in Figure 7 show that both eGFP-mRNA-LNPs and eGFP-mRNA-LNPs-Trf exhibited close to 100% *in vitro* transfection efficiency with PBMC cells. However, the GFP expression level in the eGFP-mRNA-LNPs-Trf transfection group was significantly higher than that in the eGFP-mRNA-LNPs transfection group, demonstrating that transferrin adsorption enhances the transfection efficiency of LNPs for macrophages within PBMC cells.

### (7) eGFP-mRNA-LNPs-Trf In Vitro Transfection Experiment in T Cells within PBMCs

mRNA equivalent to a concentration of 1 µg/mL was added per well (containing approximately 100,000 PBMCs per well). Pre-warmed eGFP-mRNA-LNPs and eGFP-mRNA-LNPs-Trf were added dropwise to the wells, shaken to mix, and the PBMC cells were cultured for a further 24 h. PBMC cells were collected, centrifuged at 1500 r/min for 5 min, and filtered through a 70 µm cell strainer. T cells were then labeled by staining with APC Mouse Anti-Human CD3 (UCHT1) for 45 min, followed by centrifugation and washing three times. Finally, changes in the proportion of GFP-positive T cells were detected using an Agilent flow cytometer and statistically analyzed. The results are shown in Figure 8.

The results in Figure 8 show that after incubating PBMC cells with eGFP-mRNA-LNPs and eGFP-mRNA-LNPs-Trf for 24 h, eGFP-mRNA-LNPs could achieve transfection of T cells within PBMCs *in vitro,* with about 3.88% of T cells in PBMCs being GFP-positive. eGFP-mRNA-LNPs-Trf could further enhance the transfection efficiency of T cells *in vitro,* with about 4.58% of T cells in PBMCs being GFP-positive, demonstrating that transferrin adsorption can enhance the transfection efficiency of LNPs for T cells within PBMC cells.

### (8) eGFP-mRNA-LNPs-Trf In Vivo Splenic Macrophage Transfection Experiment

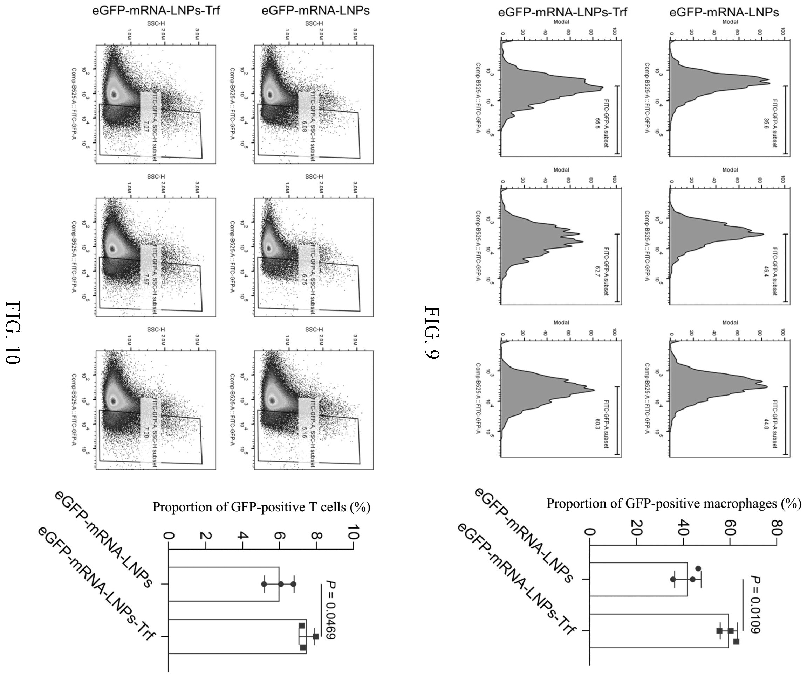

BALB/c mice were taken and injected intravenously via the tail vein with eGFP-mRNA-LNPs or eGFP-mRNA-LNPs-Trf at a dose of 0.1 mg/kg to verify the *in vivo* transfection effect of the targeting lipid composition of the present invention. Mice were sacrificed 24 hours post-injection, spleens were removed and homogenized, followed by lysis with red blood cell lysis buffer for 30 min, centrifugation at 1500 r/min for 5 min, and filtration through a 70 µm cell strainer. Macrophages were then labeled by staining with BV786 Rat Anti-CD11b (M1/70) and BV421 Rat Anti-Mouse F4/80 (T45-2342) for 45 min, followed by centrifugation and washing three times. Finally, changes in the proportion of GFP-positive macrophages were detected using an Agilent flow cytometer and statistically analyzed. The results are shown in Figure 9.

The results in Figure 9 show that 24 hours after intravenous injection of eGFP-mRNA-LNPs and eGFP-mRNA-LNPs-Trf, eGFP-mRNA-LNPs could achieve macrophage transfection *in vivo,* with about 42% of macrophages in the mouse spleen being GFP-positive. The eGFP-mRNA-LNPs-Trf group had about 59.5% of T cells being GFP-positive, significantly higher than the eGFP-mRNA-LNPs group, demonstrating that the targeting lipid composition provided by the present invention can be used for *in vivo* macrophage transfection.

### (9) eGFP-mRNA-LNPs-Trf In Vivo Splenic T Cell Transfection Experiment

BALB/c mice were taken and injected intravenously via the tail vein with eGFP-mRNA-LNPs or eGFP-mRNA-LNPs-Trf at a dose of 0.1 mg/kg to verify the *in vivo* transfection effect of the targeting lipid composition of the present invention. Mice were sacrificed 24 hours post-injection, spleens were removed and homogenized, followed by lysis with red blood cell lysis buffer for 30 min, centrifugation at 1500 r/min for 5 min, and filtration through a 70 µm cell strainer. T cells were then labeled by staining with BV421 Hamster Anti-Mouse CD3e (145-2C11) for 45 min, followed by centrifugation and washing three times. Finally, changes in the proportion of GFP-positive T cells were detected using an Agilent flow cytometer and statistically analyzed. The results are shown in Figure 10.

The results in Figure 10 show that 48 hours after intravenous injection of eGFP-mRNA-LNPs and eGFP-mRNA-LNPs-Trf, eGFP-mRNA-LNPs could achieve T cell transfection *in vivo,* with about 5.99% of T cells in the mouse spleen being GFP-positive. eGFP-mRNA-LNPs-Trf could further enhance the transfection efficiency of T cells *in vivo,* with about 7.48% of T cells in the mouse spleen being GFP-positive, demonstrating that the targeting lipid composition provided by the present invention can be used for *in vivo* T cell transfection.

### (10) eGFP-mRNA-LNPs-Trf In Vivo Splenic Natural Killer Cell (NK Cell) Transfection Experiment

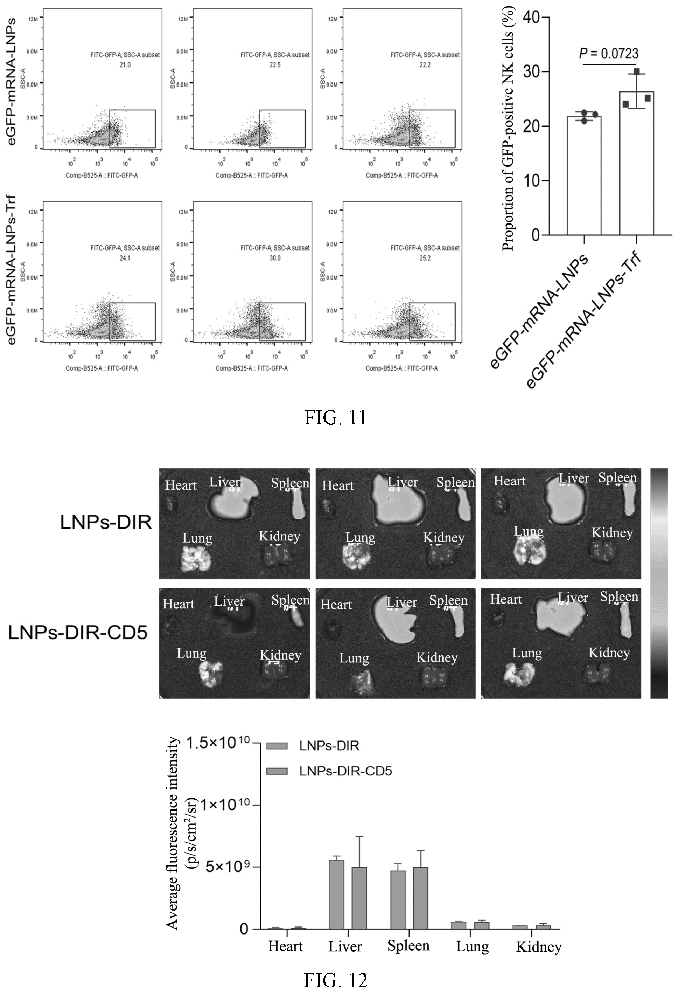

BALB/c mice were taken and injected intravenously via the tail vein with eGFP-mRNA-LNPs or eGFP-mRNA-LNPs-Trf at a dose of 0.1 mg/kg to verify the *in vivo* transfection effect of the targeting lipid composition of the present invention. Mice were sacrificed 24 hours post-injection, spleens were removed and homogenized, followed by lysis with red blood cell lysis buffer for 30 min, centrifugation at 1500 r/min for 5 min, and filtration through a 70 µm cell strainer. NK cells were then labeled by staining with BV421 Hamster Anti-Mouse CD3e (145-2C11) and APC Rat Anti-Mouse CD49b (DX5) for 45 min, followed by centrifugation and washing three times. Finally, changes in the proportion of GFP-positive NK cells were detected using an Agilent flow cytometer and statistically analyzed. The results are shown in Figure 11.

The results in Figure 11 show that 24 hours after intravenous injection of eGFP-mRNA-LNPs and eGFP-mRNA-LNPs-Trf, eGFP-mRNA-LNPs could achieve NK cell transfection *in vivo,* with about 21.9% of NK cells in the mouse spleen being GFP-positive. The eGFP-mRNA-LNPs-Trf group had about 26.4% of NK cells being GFP-positive, higher than the eGFP-mRNA-LNPs group, demonstrating that the targeting lipid composition provided by the present invention can be used for *in vivo* NK cell transfection.

### (11) LNPs-DIR-CD5 (DOTAP/SM102=2.5/47.5) In Vivo Tissue Targeting Experiment

Referring to (2), LNPs-DIR and LNPs-DIR-CD5 with a molar ratio of DOTAP to SM102 of 2.5:47.5 were prepared. BALB/c mice were taken and injected intravenously via the tail vein with LNPs-DIR or LNPs-DIR-CD5 at a dose of 0.25 mg/kg to verify the *in vivo* transfection effect of the targeting lipid composition of the present invention. Mice were sacrificed 24 hours post-injection, and heart, liver, spleen, lung, and kidney tissues were collected. The distribution of LNPs-DIR in various organs was observed using IVIS Lumina III and statistically analyzed. The results are shown in Figure 12.

The results in Figure 12 show that 24 hours after intravenous injection of LNPs-DIR and LNPs-DIR-CD5, there was no significant difference in the tissue distribution of LNPs-DIR and LNPs-DIR-CD5 in various organs. This demonstrates that when DOTAP/SM102=2.5/47.5, CD5 single-chain antibody adsorption has no significant effect on the spleen-targeting behavior of LNPs.

### (12) LNPs-DIR-CD5 (DOTAP/SM102=5/45) In Vivo Tissue Targeting Experiment

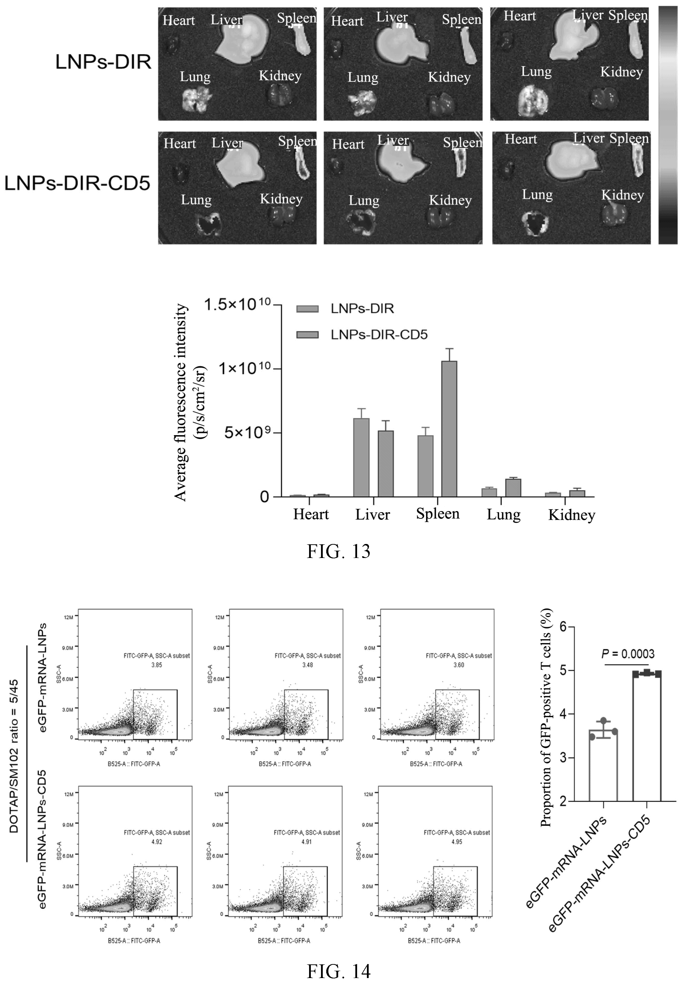

Referring to (2), LNPs-DIR and LNPs-DIR-CD5 with a molar ratio of DOTAP to SM102 of 5:45 were prepared. BALB/c mice were taken and injected intravenously via the tail vein with LNPs-DIR or LNPs-DIR-CD5 at a dose of 0.25 mg/kg to verify the *in vivo* transfection effect of the targeting lipid composition of the present invention. Mice were sacrificed 24 hours post-injection, and heart, liver, spleen, lung, and kidney tissues were collected. The distribution of LNPs-DIR in various organs was observed using IVIS Lumina III and statistically analyzed. The results are shown in Figure 13.

The results in Figure 13 show that 24 hours after intravenous injection of LNPs-DIR and LNPs-DIR-CD5, the fluorescence intensity of LNPs-DIR-CD5 in the spleen was approximately twice that of the LNPs-DIR group. This demonstrates that when DOTAP/SM102=5/45, CD5 single-chain antibody adsorption can significantly enhance the spleen-targeting ability of LNPs, potentially improving the transfection efficiency of LNPs for splenic immune cells.

### (13) eGFP-mRNA-LNPs-CD5 In Vivo Splenic T Cell Transfection Experiment

BALB/c mice were taken and injected intravenously via the tail vein with eGFP-mRNA-LNPs or eGFP-mRNA-LNPs-CD5 at a dose of 0.1 mg/kg to verify the *in vivo* transfection effect of the targeting lipid composition of the present invention. Mice were sacrificed 12 hours post-injection, spleens were removed and homogenized, followed by lysis with red blood cell lysis buffer for 30 min, centrifugation at 1500 r/min for 5 min, and filtration through a 70 µm cell strainer. T cells were then labeled by staining with BV421 Hamster Anti-Mouse CD3e (145-2C11) for 45 min, followed by centrifugation and washing three times. Finally, changes in the proportion of GFP-positive T cells were detected using an Agilent flow cytometer and statistically analyzed. The results are shown in Figure 14.

The results in Figure 14 show that 12 hours after intravenous injection of eGFP-mRNA-LNPs or eGFP-mRNA-LNPs-CD5, eGFP-mRNA-LNPs could achieve T cell transfection *in vivo,* with about 3.64% of T cells in the mouse spleen being GFP-positive. eGFP-mRNA-LNPs-CD5 could further enhance the transfection efficiency of T cells *in vivo,* with about 4.93% of T cells in the mouse spleen being GFP-positive, demonstrating that the targeting lipid composition provided by the present invention can be used for *in vivo* T cell transfection.

### (14) eGFP-mRNA-LNPs-CD5 In Vivo Splenic T Cell Transfection Experiment

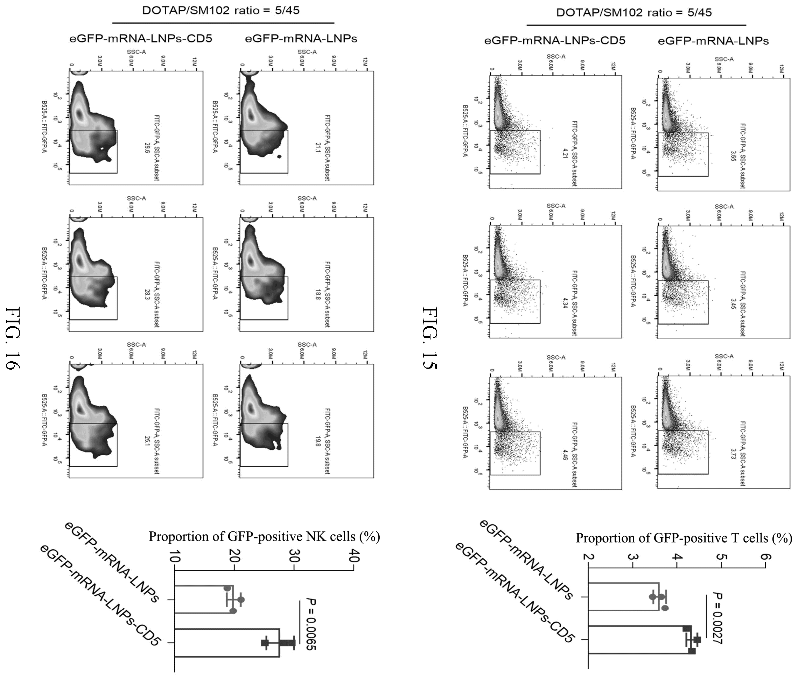

BALB/c mice were taken and injected intravenously via the tail vein with eGFP-mRNA-LNPs or eGFP-mRNA-LNPs-CD5 at a dose of 0.1 mg/kg to verify the *in vivo* transfection effect of the targeting lipid composition of the present invention. Mice were sacrificed 48 hours post-injection, spleens were removed and homogenized, followed by lysis with red blood cell lysis buffer for 30 min, centrifugation at 1500 r/min for 5 min, and filtration through a 70 µm cell strainer. T cells were then labeled by staining with BV421 Hamster Anti-Mouse CD3e (145-2C11) for 45 min, followed by centrifugation and washing three times. Finally, changes in the proportion of GFP-positive T cells were detected using an Agilent flow cytometer and statistically analyzed. The results are shown in Figure 15.

The results in Figure 15 show that 48 hours after intravenous injection of eGFP-mRNA-LNPs or eGFP-mRNA-LNPs-CD5, eGFP-mRNA-LNPs could achieve T cell transfection *in vivo,* with about 3.61% of T cells in the mouse spleen being GFP-positive. eGFP-mRNA-LNPs-CD5 could further enhance the transfection efficiency of T cells *in vivo,* with about 4.34% of T cells in the mouse spleen being GFP-positive, demonstrating that the targeting lipid composition provided by the present invention can be used for *in vivo* T cell transfection.

### (15) eGFP-mRNA-LNPs-CD5 In Vivo Splenic NK Cell Transfection Experiment

BALB/c mice were taken and injected intravenously via the tail vein with eGFP-mRNA-LNPs or eGFP-mRNA-LNPs-CD5 at a dose of 0.1 mg/kg to verify the *in vivo* transfection effect of the targeting lipid composition of the present invention. Mice were sacrificed 12 hours post-injection, spleens were removed and homogenized, followed by lysis with red blood cell lysis buffer for 30 min, centrifugation at 1500 r/min for 5 min, and filtration through a 70 µm cell strainer. NK cells were then labeled by staining with BV421 Hamster Anti-Mouse CD3e (145-2C11) and APC Rat Anti-Mouse CD49b (DX5) for 45 min, followed by centrifugation and washing three times. Finally, changes in the proportion of GFP-positive NK cells were detected using an Agilent flow cytometer and statistically analyzed. The results are shown in Figure 16.

The results in Figure 16 show that 12 hours after intravenous injection of eGFP-mRNA-LNPs or eGFP-mRNA-LNPs-CD5, eGFP-mRNA-LNPs could achieve NK cell transfection *in vivo,* with about 19.9% of NK cells in the mouse spleen being GFP-positive. eGFP-mRNA-LNPs-CD5 could further enhance the transfection efficiency of NK cells *in vivo,* with about 27.7% of T cells in the mouse spleen being GFP-positive, demonstrating that the targeting lipid composition provided by the present invention can be used for *in vivo* NK cell transfection.

### (16) eGFP-mRNA-LNPs-CD5 In Vivo Splenic NK Cell Transfection Experiment

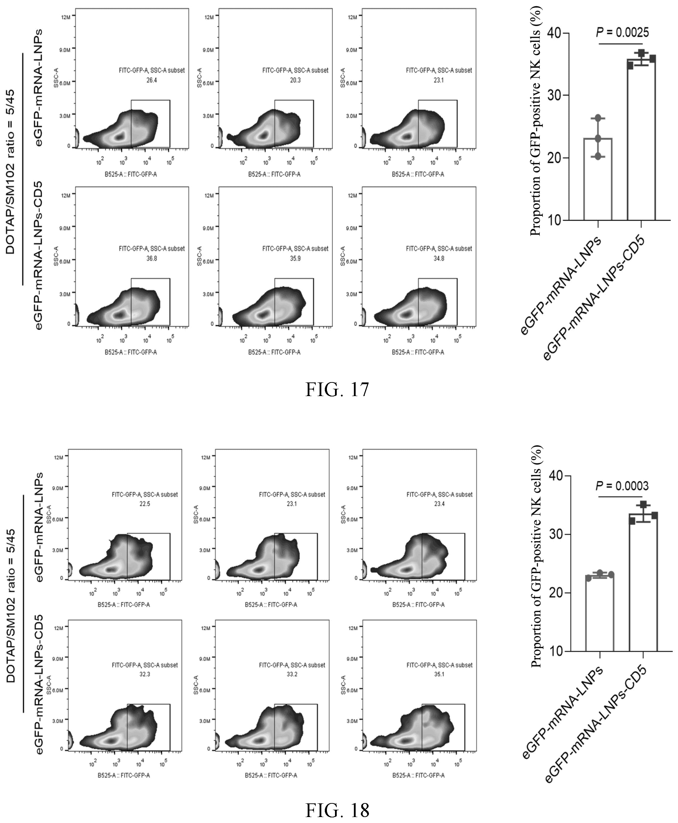

BALB/c mice were taken and injected intravenously via the tail vein with eGFP-mRNA-LNPs or eGFP-mRNA-LNPs-CD5 at a dose of 0.1 mg/kg to verify the *in vivo* transfection effect of the targeting lipid composition of the present invention. Mice were sacrificed 24 hours post-injection, spleens were removed and homogenized, followed by lysis with red blood cell lysis buffer for 30 min, centrifugation at 1500 r/min for 5 min, and filtration through a 70 µm cell strainer. NK cells were then labeled by staining with BV421 Hamster Anti-Mouse CD3e (145-2C11) and APC Rat Anti-Mouse CD49b (DX5) for 45 min, followed by centrifugation and washing three times. Finally, changes in the proportion of GFP-positive NK cells were detected using an Agilent flow cytometer and statistically analyzed. The results are shown in Figure 17.

The results in Figure 17 show that 24 hours after intravenous injection of eGFP-mRNA-LNPs or eGFP-mRNA-LNPs-CD5, eGFP-mRNA-LNPs could achieve NK cell transfection *in vivo,* with about 23.3% of NK cells in the mouse spleen being GFP-positive. eGFP-mRNA-LNPs-CD5 could further enhance the transfection efficiency of NK cells *in vivo,* with about 35.8% of T cells in the mouse spleen being GFP-positive, demonstrating that the targeting lipid composition provided by the present invention can be used for *in vivo* NK cell transfection.

### (17) eGFP-mRNA-LNPs-CD5 In Vivo Splenic NK Cell Transfection Experiment

BALB/c mice were taken and injected intravenously via the tail vein with eGFP-mRNA-LNPs or eGFP-mRNA-LNPs-CD5 at a dose of 0.1 mg/kg to verify the *in vivo* transfection effect of the targeted lipid composition of the present invention. Mice were sacrificed 48 hours post-injection, spleens were removed and homogenized, followed by lysis with red blood cell lysis buffer for 30 min, centrifugation at 1500 r/min for 5 min, and filtration through a 70 µm cell strainer. NK cells were then labeled by staining with BV421 Hamster Anti-Mouse CD3e (145-2C11) and APC Rat Anti-Mouse CD49b (DX5) for 45 min, followed by centrifugation and washing three times. Finally, changes in the proportion of GFP-positive NK cells were detected using an Agilent flow cytometer and statistically analyzed. The results are shown in Figure 18.

The results in Figure 18 show that 48 hours after intravenous injection of eGFP-mRNA-LNPs or eGFP-mRNA-LNPs-CD5, eGFP-mRNA-LNPs could achieve NK cell transfection *in vivo,* with about 23% of NK cells in the mouse spleen being GFP-positive. eGFP-mRNA-LNPs-CD5 could further enhance the transfection efficiency of NK cells *in vivo,* with about 33.5% of T cells in the mouse spleen being GFP-positive, demonstrating that the targeting lipid composition provided by the present invention can be used for *in vivo* NK cell transfection.

### (18) CD19 CAR-mRNA-LNPs-CD5 In Vivo Splenic T Cell Transfection Experiment

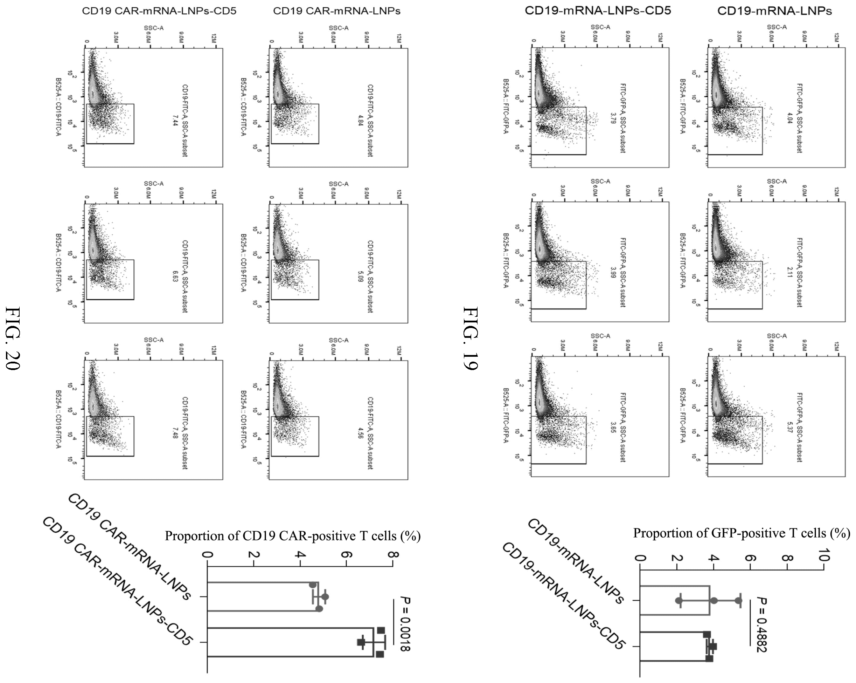

BALB/c mice were taken and injected intravenously via the tail vein with eGFP-mRNA-LNPs or eGFP-mRNA-LNPs-CD5 at a dose of 0.1 mg/kg to verify the *in vivo* transfection effect of the targeting lipid composition of the present invention. Mice were sacrificed at 24, 72, 120, 168 hours post-injection. Spleens were removed and homogenized, followed by lysis with red blood cell lysis buffer for 30 min, centrifugation at 1500 r/min for 5 min, and filtration through a 70 µm cell strainer. T cells were then labeled by staining with BV421 Hamster Anti-Mouse CD3e (145-2C11) for 45 min, followed by centrifugation and washing three times. Finally, changes in the proportion of GFP-positive T cells were detected using an Agilent flow cytometer and statistically analyzed. The results are shown in Figures 19-22.

The results in Figure 19 show that 24 hours after intravenous injection of CD19 CAR-mRNA-LNPs or CD19 CAR-mRNA-LNPs-CD5, CD19 CAR-mRNA-LNPs could achieve T cell transfection *in vivo,* with about 3.84% of T cells in the mouse spleen being CD19 CAR-positive. In the CAR-mRNA-LNPs-CD5 group, about 3.81% of T cells in the mouse spleen were CD19 CAR-positive, demonstrating that the targeting lipid composition provided by the present invention can be used for *in vivo* T cell transfection.

The results in Figure 20 show that 72 hours after intravenous injection of CD19 CAR-mRNA-LNPs or CD19 CAR-mRNA-LNPs-CD5, CD19 CAR-mRNA-LNPs could achieve T cell transfection *in vivo,* with about 4.83% of T cells in the mouse spleen being CD19 CAR-positive. In the CAR-mRNA-LNPs-CD5 group, about 7.18% of T cells in the mouse spleen were CD19 CAR-positive, demonstrating that the targeting lipid composition provided by the present invention can be used for *in vivo* T cell transfection.

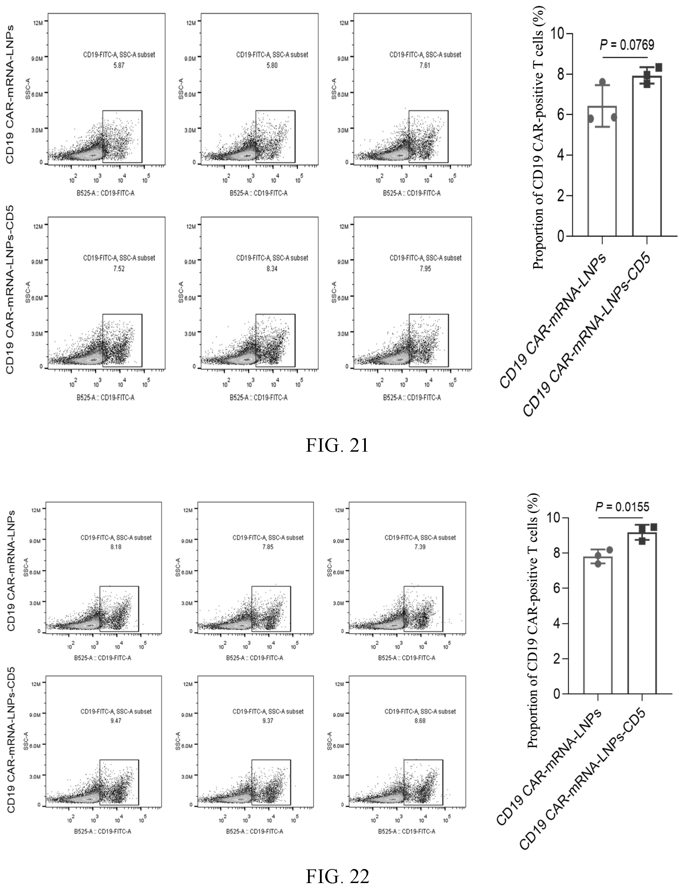

The results in Figure 21 show that 120 hours after intravenous injection of CD19 CAR-mRNA-LNPs or CD19 CAR-mRNA-LNPs-CD5, CD19 CAR-mRNA-LNPs could achieve T cell transfection *in vivo,* with about 6.43% of T cells in the mouse spleen being CD19 CAR-positive. In the CAR-mRNA-LNPs-CD5 group, about 7.94% of T cells in the mouse spleen were CD19 CAR-positive, demonstrating that the targeting lipid composition provided by the present invention can be used for *in vivo* T cell transfection.

The results in Figure 22 show that 168 hours after intravenous injection of CD19 CAR-mRNA-LNPs or CD19 CAR-mRNA-LNPs-CD5, CD19 CAR-mRNA-LNPs could achieve T cell transfection *in vivo,* with about 7.81% of T cells in the mouse spleen being CD19 CAR-positive. In the CAR-mRNA-LNPs-CD5 group, about 9.17% of T cells in the mouse spleen were CD19 CAR-positive, demonstrating that the targeting lipid composition provided by the present invention can be used for long-term *in vivo* T cell transfection.

### (19) CD19 CAR-mRNA-LNPs-CD5 In Vivo Splenic NK Cell Transfection Experiment

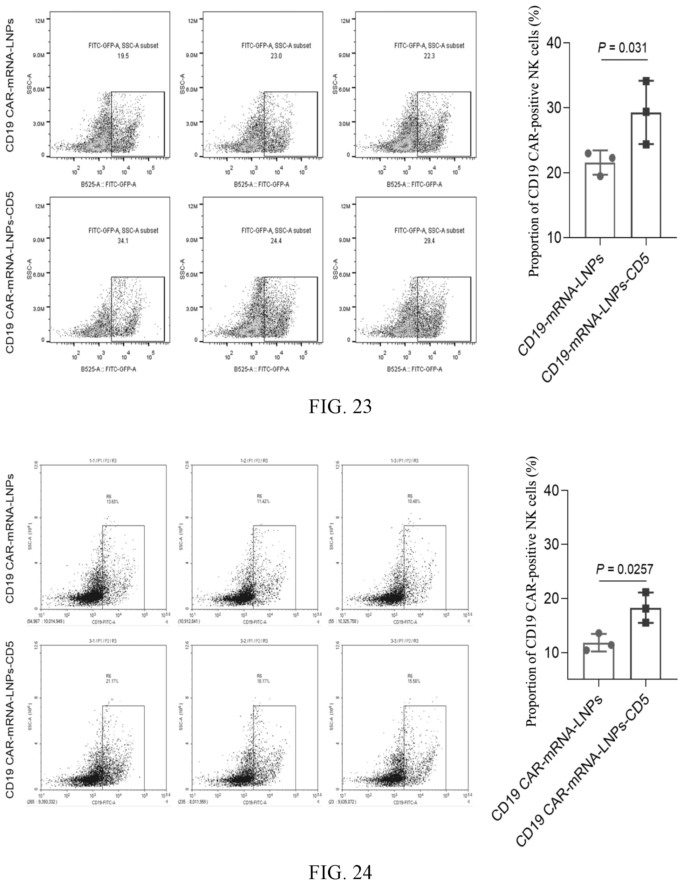

BALB/c mice were taken and injected intravenously via the tail vein with CD19 CAR-mRNA-LNPs or CD19 CAR-mRNA-LNPs-CD5 at a dose of 0.1 mg/kg to verify the *in vivo* transfection effect of the targeting lipid composition of the present invention. Mice were sacrificed at 24, 72, 120, 168 hours post-injection. Spleens were removed and homogenized, followed by lysis with red blood cell lysis buffer for 30 min, centrifugation at 1500 r/min for 5 min, and filtration through a 70 µm cell strainer. NK cells were then labeled by staining with BV421 Hamster Anti-Mouse CD3e (145-2C11) and APC Rat Anti-Mouse CD49b (DX5) for 45 min, followed by centrifugation and washing three times. Finally, changes in the proportion of GFP-positive NK cells were detected using an Agilent flow cytometer and statistically analyzed. The results are shown in Figures 23-26.

The results in Figure 23 show that 24 hours after intravenous injection of CD19 CAR-mRNA-LNPs or CD19 CAR-mRNA-LNPs-CD5, CD19 CAR-mRNA-LNPs could achieve NK cell transfection *in vivo,* with about 21.6% of NK cells in the mouse spleen being CD19 CAR-positive. CD19 CAR-mRNA-LNPs-CD5 could further enhance the transfection efficiency of NK cells *in vivo,* with about 29.3% of T cells in the mouse spleen being CD19 CAR-positive, demonstrating that the targeting lipid composition provided by the present invention can be used for *in vivo* NK cell transfection.

The results in Figure 24 show that 72 hours after intravenous injection of CD19 CAR-mRNA-LNPs or CD19 CAR-mRNA-LNPs-CD5, CD19 CAR-mRNA-LNPs could achieve NK cell transfection *in vivo,* with about 11.8% of NK cells in the mouse spleen being CD19 CAR-positive. CD19 CAR-mRNA-LNPs-CD5 could further enhance the transfection efficiency of NK cells *in vivo,* with about 18.3% of T cells in the mouse spleen being CD19 CAR-positive, demonstrating that the targeting lipid composition provided by the present invention can be used for *in vivo* NK cell transfection.

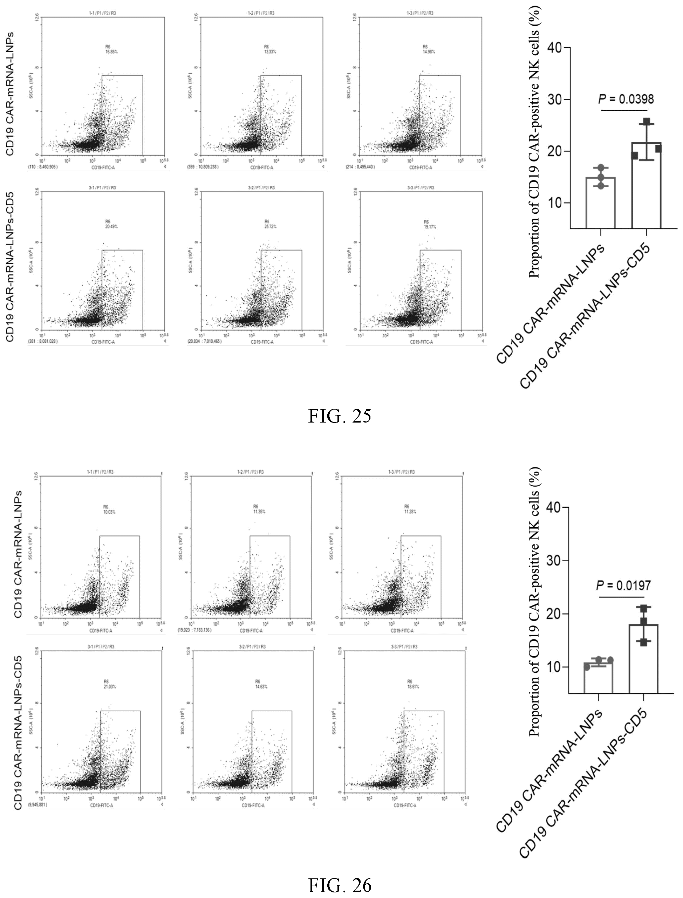

The results in Figure 25 show that 120 hours after intravenous injection of CD19 CAR-mRNA-LNPs or CD19 CAR-mRNA-LNPs-CD5, CD19 CAR-mRNA-LNPs could achieve NK cell transfection *in vivo,* with about 15.1% of NK cells in the mouse spleen being CD19 CAR-positive. CD19 CAR-mRNA-LNPs-CD5 could further enhance the transfection efficiency of NK cells *in vivo,* with about 21.8% of T cells in the mouse spleen being CD19 CAR-positive, demonstrating that the targeting lipid composition provided by the present invention can be used for *in vivo* NK cell transfection.

The results in Figure 26 show that 168 hours after intravenous injection of CD19 CAR-mRNA-LNPs or CD19 CAR-mRNA-LNPs-CD5, CD19 CAR-mRNA-LNPs could achieve NK cell transfection *in vivo,* with about 10.89% of NK cells in the mouse spleen being CD19 CAR-positive. CD19 CAR-mRNA-LNPs-CD5 could further enhance the transfection efficiency of NK cells *in vivo,* with about 18.09% of T cells in the mouse spleen being CD19 CAR-positive, demonstrating that the targeting lipid composition provided by the present invention can be used for long-term *in vivo* NK cell transfection.

### Examples 9-38. Lipid Nanoparticle Delivery Systems Encapsulating GFP mRNA Based on Quaternary Amine/Tetraamine/Quaternary Ammonium and Tertiary Amine/Triamine/Tertiary Ammonium Systems

In Examples 9-38, the mRNA encapsulated in the lipid nanoparticle delivery system is HiSignal^{®} Fluc-eGFP mRNA (5'CAP) provided by Jiangsu Shenji Biotechnology, with a mass ratio of mRNA to total lipid of 1:20. The preparation method for Examples 10-38 is as described in Example 9. The LNP suspensions prepared in Examples 10-38 are tested for particle size and PDI on a laser particle size analyzer as per Example 9, and the prepared LNP suspensions can be used for cell evaluation and *in vivo* animal evaluation.

Example 9: A quaternary amine-tertiary amine lipid nanoparticle system based on DOTAP (1,2-dioleoyl-3-trimethylammonium-propane (chloride salt)) and SM-102 (1-octylnonyl 8-[(2-hydroxyethyl)[6-oxo-6-(undecyloxy)hexyl]amino]-octanoate). The molar percentages of lipid components are shown in Table 2.

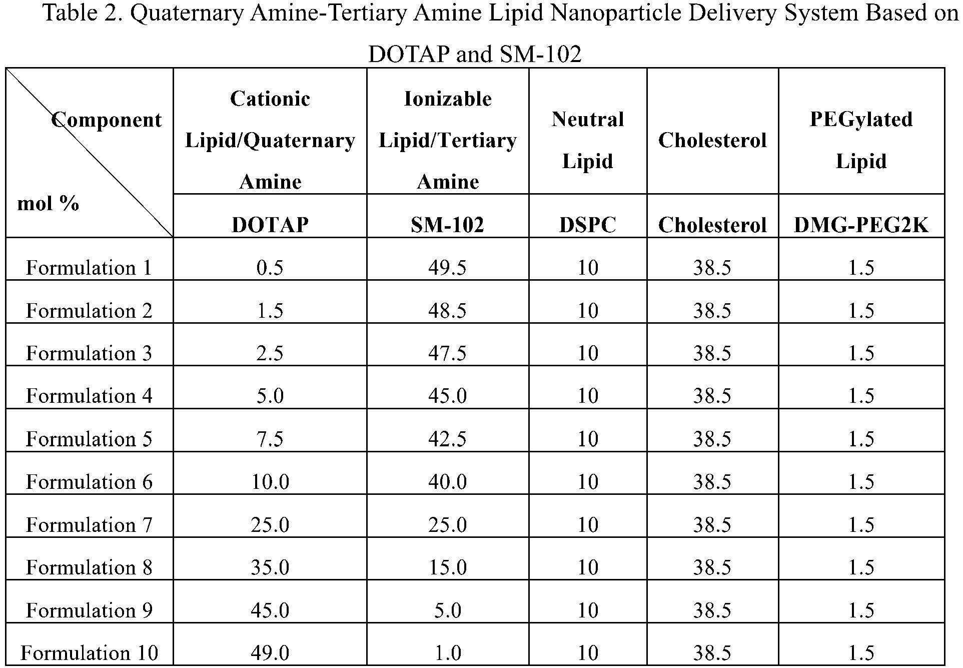

Preparation method is as follows:
1. An mRNA sodium acetate solution (pH=5.0, sodium acetate concentration 25 mM) with a concentration of 0.3334 mg/mL was prepared using sodium acetate buffer (pH=5.0, sodium acetate concentration 50 mM) and the mRNA solution;
2. A lipid ethanol solution with a total lipid concentration of 20 mg/mL was prepared by adding the lipids described in the table above to anhydrous ethanol;
3. 0.75 mL of the mRNA sodium acetate solution (nucleic acid concentration 0.3334 mg/mL, total amount 0.25 mg) was pre-warmed in a 25°C water bath;
4. 0.25 mL of the lipid solution (total lipid concentration 20 mg/mL, total amount 5 mg) was pre-warmed in a 25°C water bath;
5. Under magnetic stirring in a 25°C water bath, the 0.25 mL lipid solution was injected into the mRNA sodium acetate solution, incubated for 1 min, then diluted to four times the volume with sodium acetate buffer (pH=5.0, concentration 25 mM) to obtain the initial mRNA-loaded LNP solution;
6. If a larger quantity is needed, steps 1-5 can be repeated and the initial mRNA-loaded LNP solutions merged.
7. Ethanol was removed by centrifugal ultrafiltration using an ultrafiltration tube with a molecular weight cutoff of 100 KD at 4°C with a centrifugal acceleration of 2000 g. The nucleic acid concentration was adjusted to an appropriate level using 25 mM Tris buffer, and the pH was adjusted to 7.0 with 0.1 M sodium hydroxide solution to obtain the nucleic acid-loaded LNP suspension.

50 µL of the nucleic acid-loaded LNP suspension was added to 950 µL of pure water, then the particle size and polydispersity index (PDI or PI) were measured on a Malvern ZETASIZER PRO laser particle size analyzer. The prepared nucleic acid-loaded LNP suspension can be used for cell evaluation and *in vivo* animal evaluation.

Example 10: A quaternary amine-tertiary amine lipid nanoparticle system based on DOTAP and DODMA (1,2-dioleyloxy-3-dimethylaminopropane). The molar percentages of lipid components are shown in Table 3.

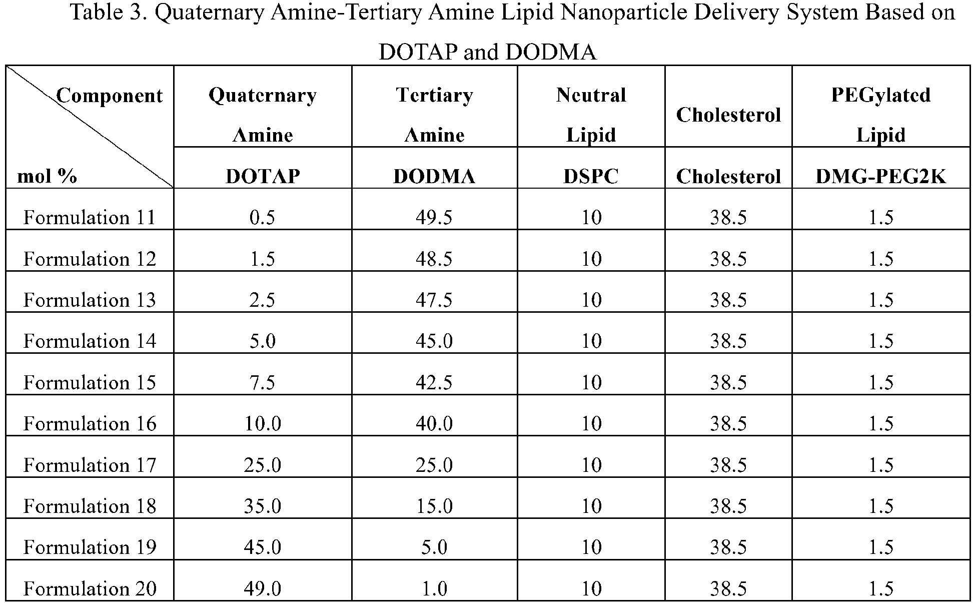

Example 11: A quaternary amine-tertiary amine lipid nanoparticle system based on DOTAP and DLin-MC3-DMA. The molar percentages of lipid components are shown in Table 4.

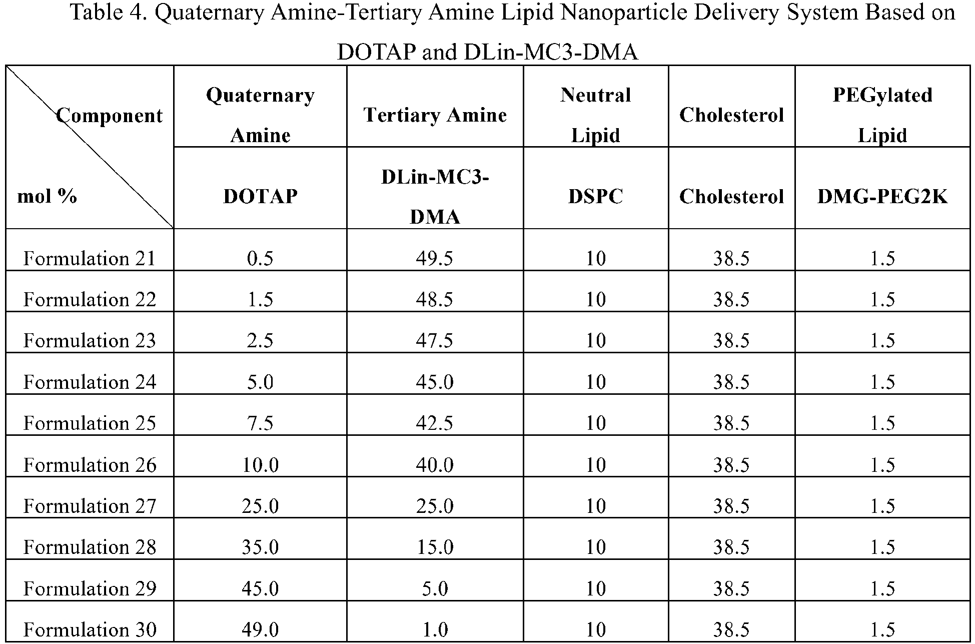

Example 12: A quaternary amine-tertiary amine lipid nanoparticle system based on DOTAP and ALC-0315. The molar percentages of lipid components are shown in Table 5.

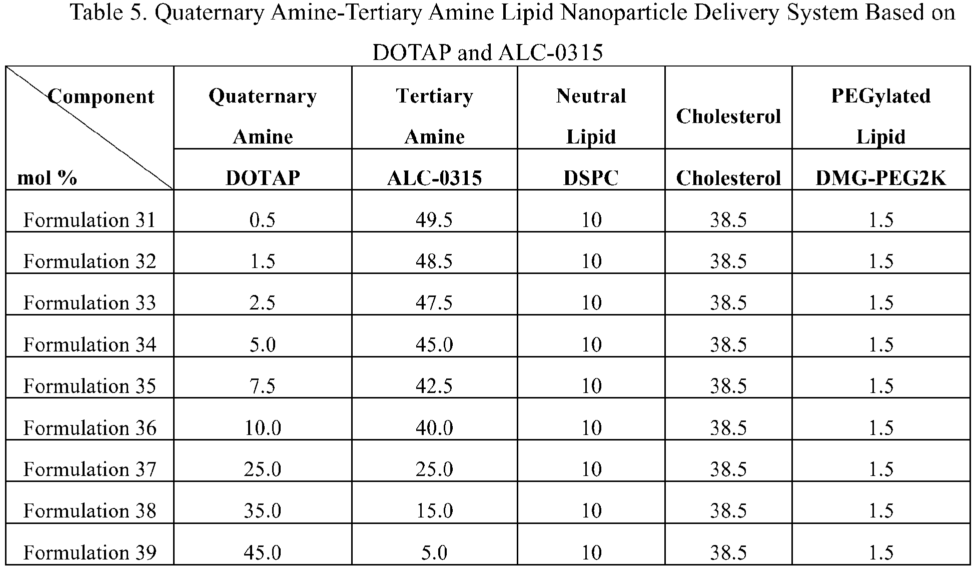

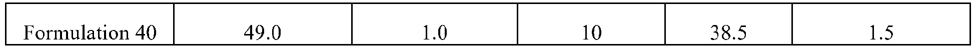

Example 13: A quaternary amine-tertiary amine lipid nanoparticle system based on DOTAP and A066 (1-(2,3-bis(((9Z,12Z)-octadeca-9,12-dien-1-yl)oxy)propyl)pyrrolidine). The molar percentages of lipid components are shown in Table 6.

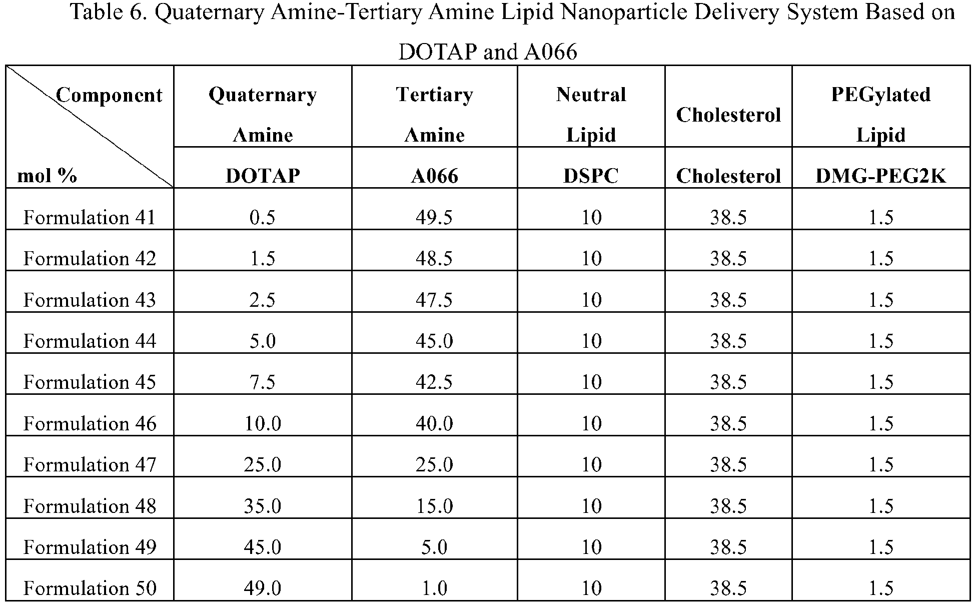

Example 14: A quaternary amine-tertiary amine lipid nanoparticle system based on DOTAP and DODAP (1,2-dioleoyl-3-dimethylammonium-propane). The molar percentages of lipid components are shown in Table 7.

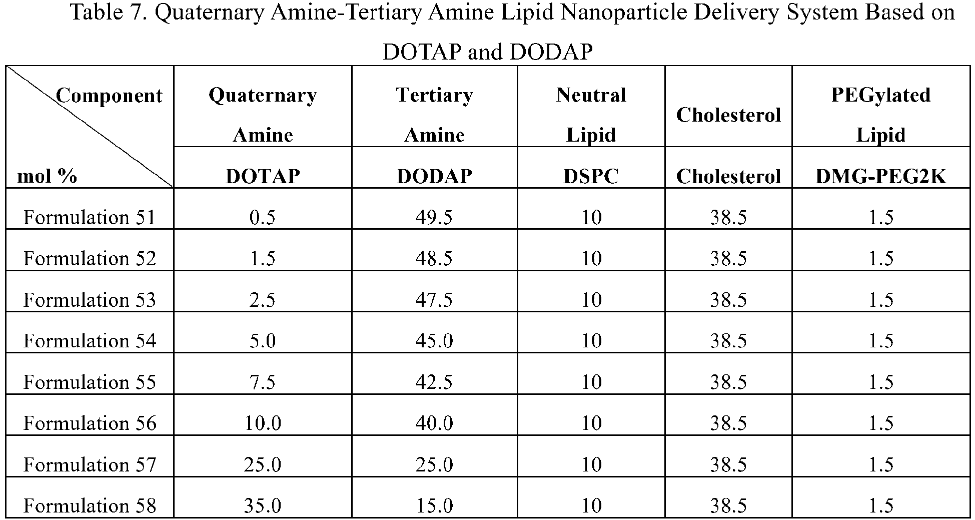

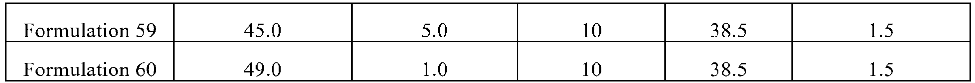

Example 15: A quaternary amine-tertiary amine lipid nanoparticle system based on DOTAP and Acuitas A9. The molar percentages of lipid components are shown in Table 8.

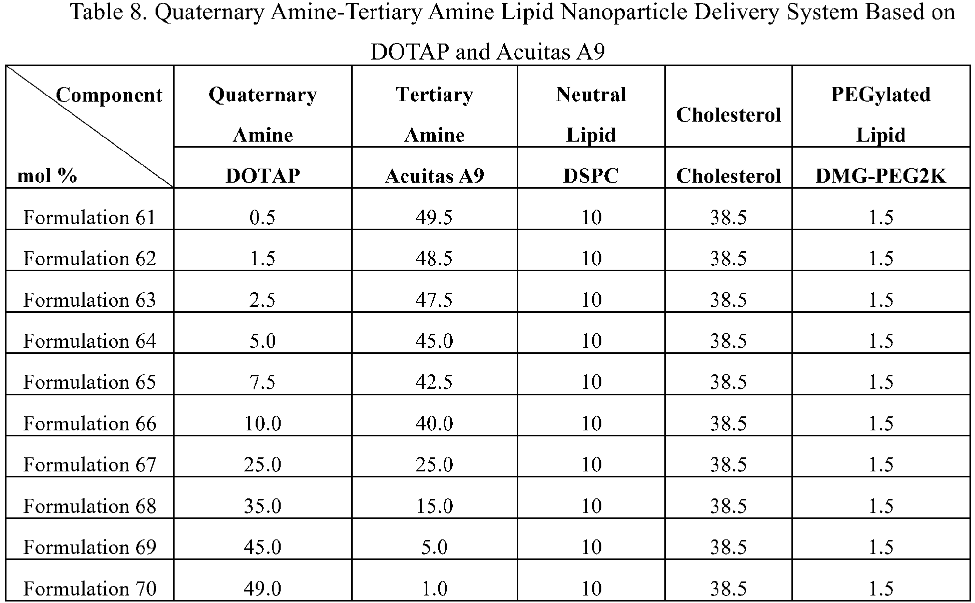

Example 16: A quaternary amine-tertiary amine lipid nanoparticle system based on DOTAP and Moderna Lipid 5. The molar percentages of lipid components are shown in Table 9.

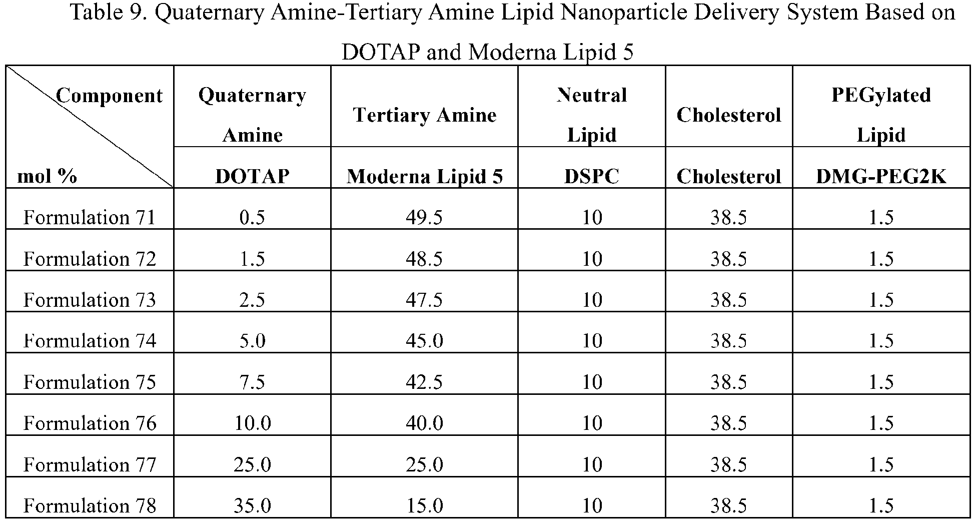

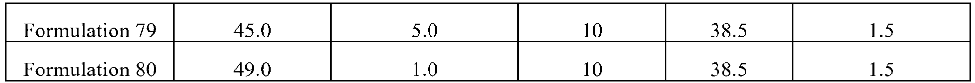

Example 17: A quaternary amine-tertiary amine lipid nanoparticle system based on DOTAP and C12-200. The molar percentages of lipid components are shown in Table 10.

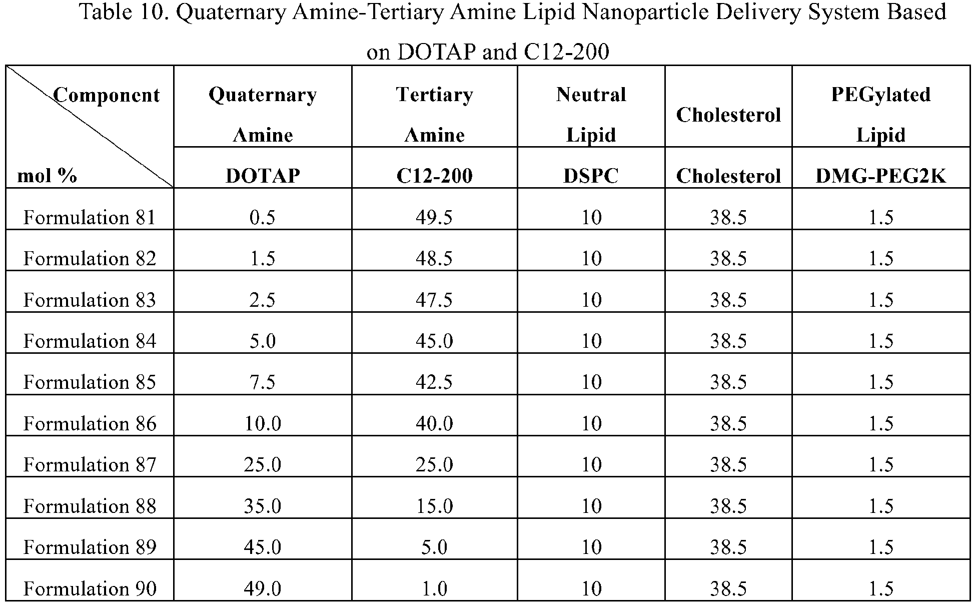

Example 18: A quaternary amine-tertiary amine lipid nanoparticle system based on DOTAP and Lipoid 5A2-SC8. The molar percentages of lipid components are shown in Table 11.

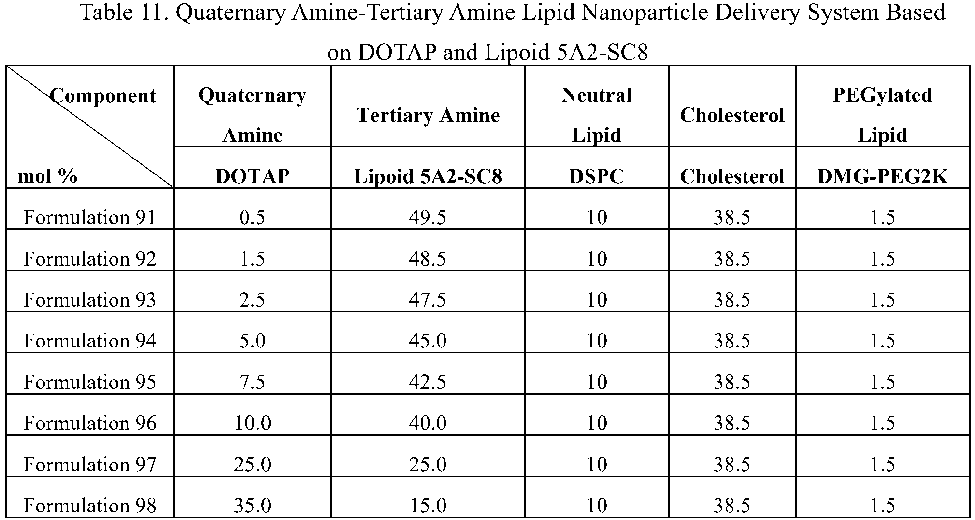

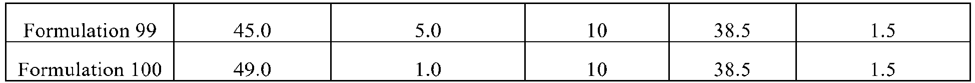

Example 19: A quaternary amine-tertiary amine lipid nanoparticle system based on DOTMA (1,2-di-O-octadecenyl-3-trimethylammonium propane (chloride salt)) and SM-102. The molar percentages of lipid components are shown in Table 12.

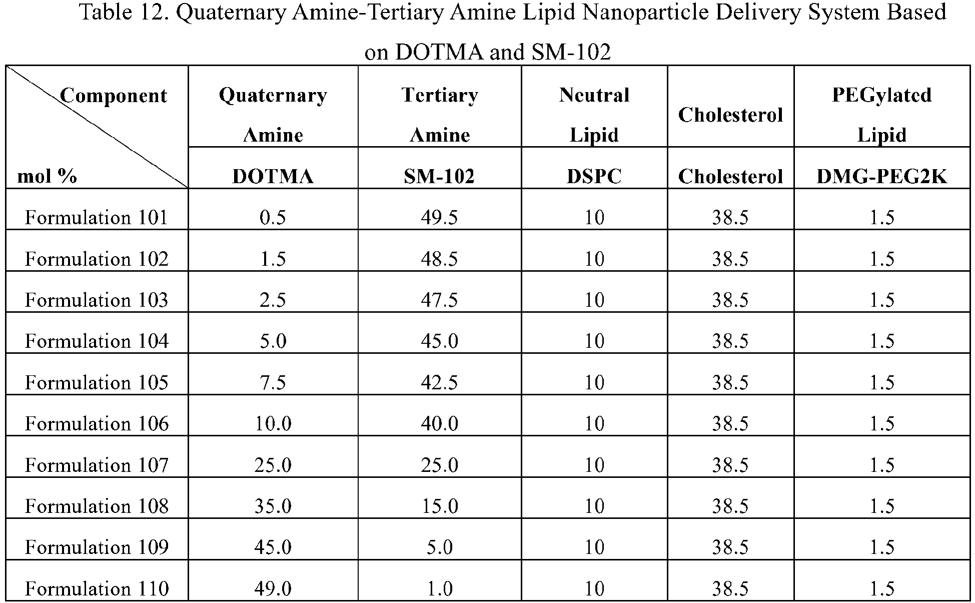

Example 20: A quaternary amine-tertiary amine lipid nanoparticle system based on DOTMA and DODMA. The molar percentages of lipid components are shown in Table 13.

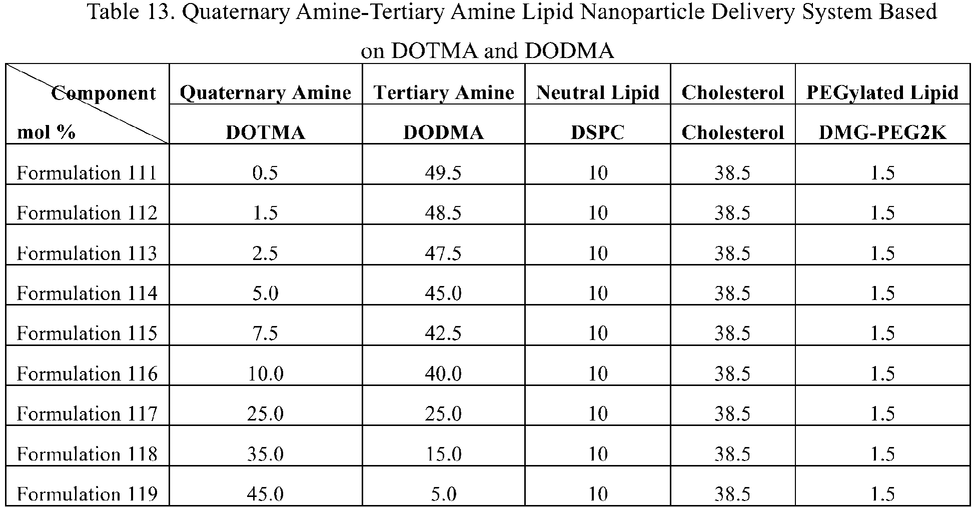

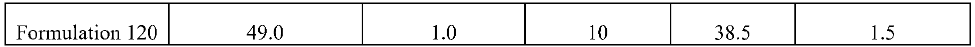

Example 21:A quaternary amine-tertiary amine lipid nanoparticle system based on DOTMA and DLin-MC3-DMA. The molar percentages of lipid components are shown in Table 14.

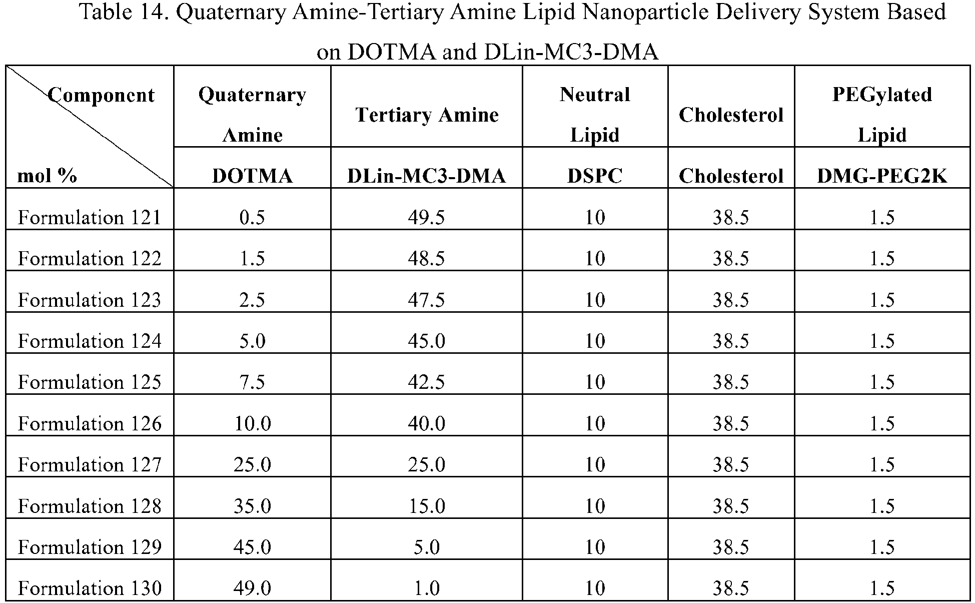

Example 22: A quaternary amine-tertiary amine lipid nanoparticle system based on DOTMA and ALC-0315. The molar percentages of lipid components are shown in Table 15.

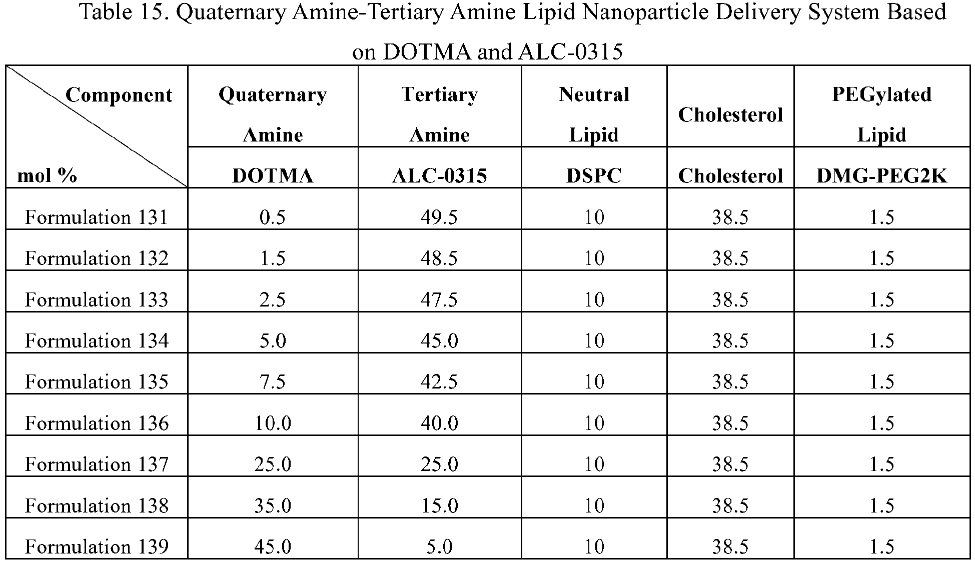

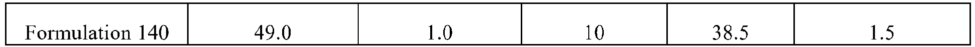

Example 23: A quaternary amine-tertiary amine lipid nanoparticle system based on DOTMA and A066. The molar percentages of lipid components are shown in Table 16.

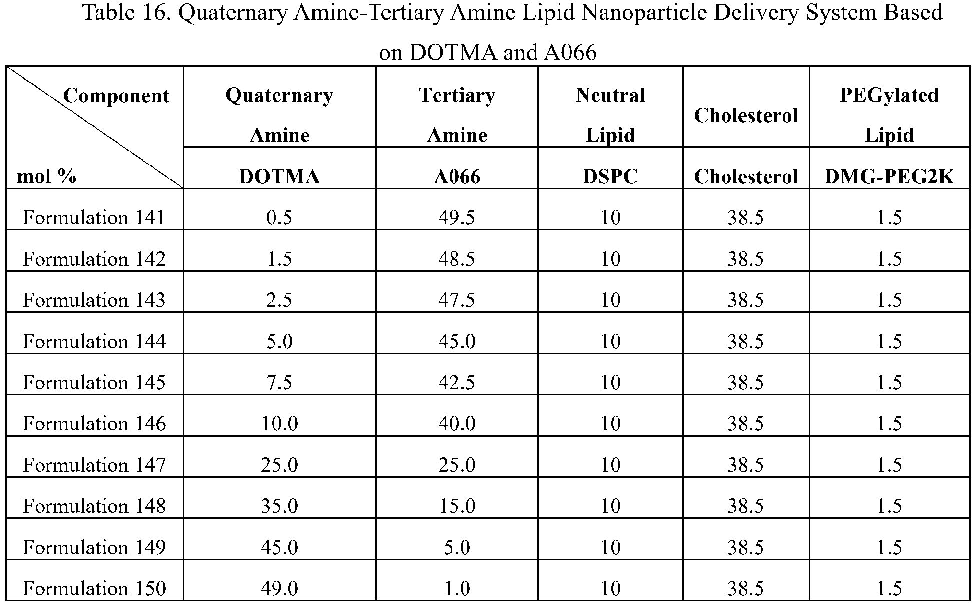

Example 24: A quaternary amine-tertiary amine lipid nanoparticle system based on DDBA (Didodecyldimethylammonium bromide) and SM-102. The molar percentages of lipid components are shown in Table 17.

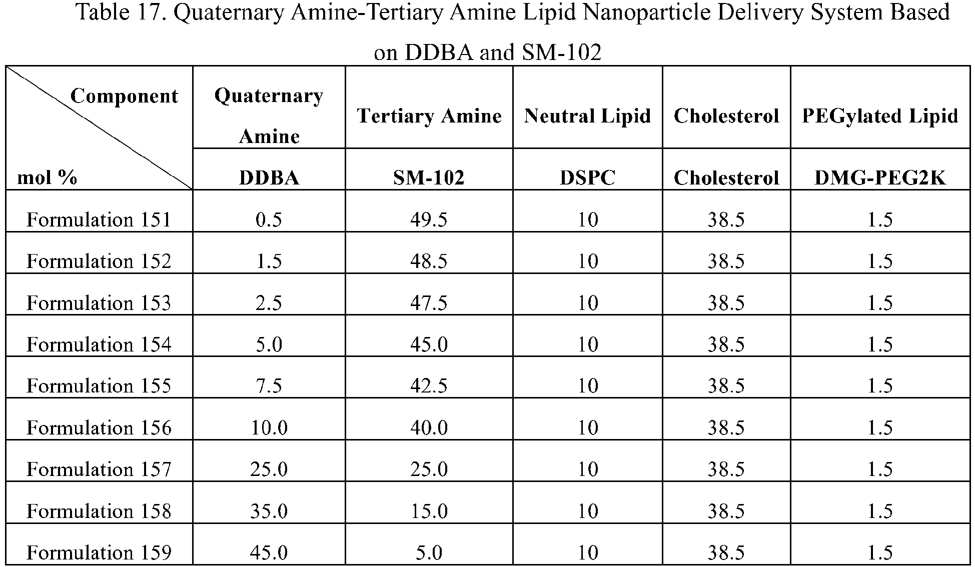

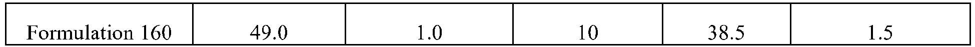

Example 25: A quaternary amine-tertiary amine lipid nanoparticle system based on DDBA and DODMA. The molar percentages of lipid components are shown in Table 18.

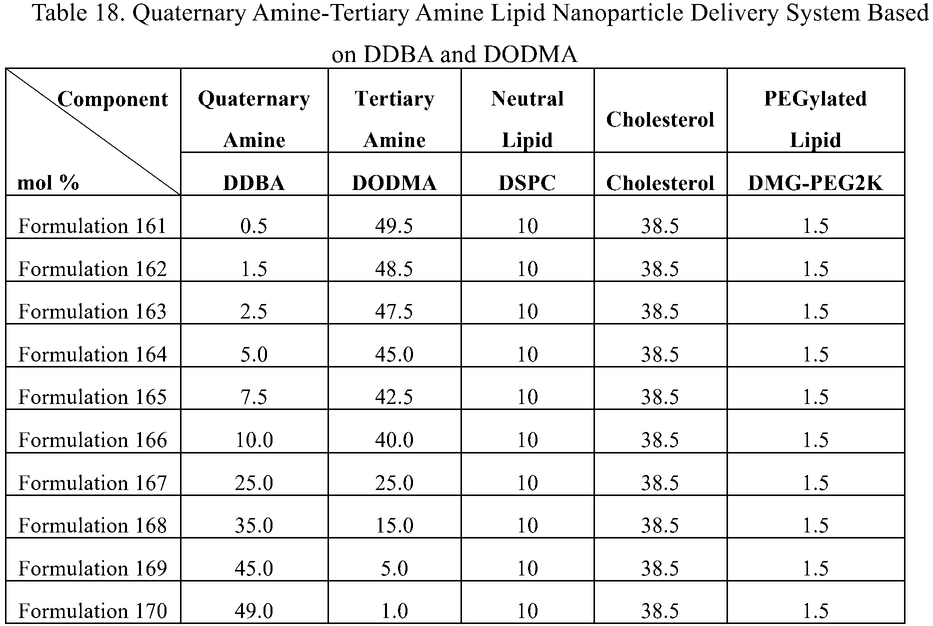

Example 26: A quaternary amine-tertiary amine lipid nanoparticle system based on DDBA and DLin-MC3-DMA. The molar percentages of lipid components are shown in Table 19.

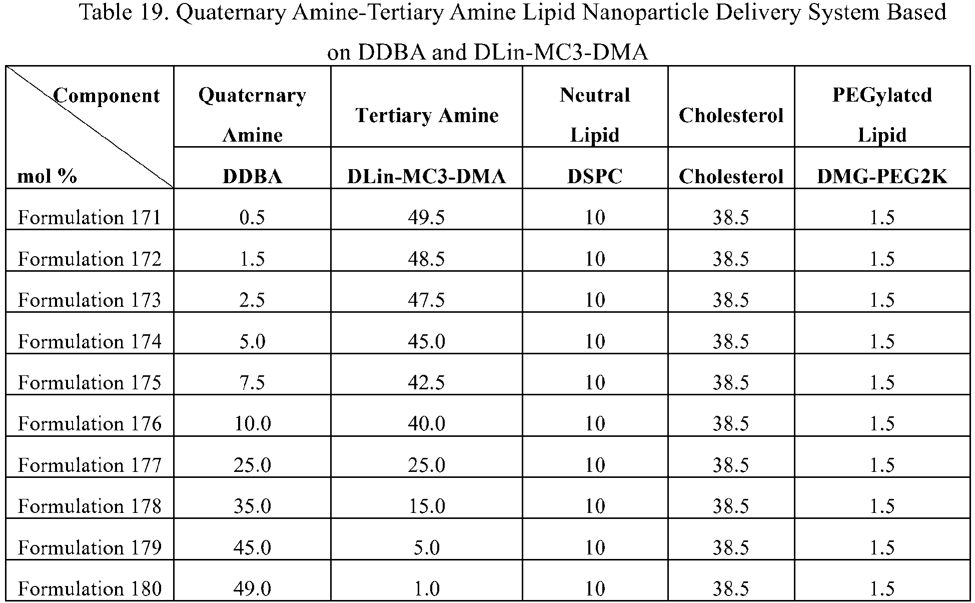

Example 27: A quaternary amine-tertiary amine lipid nanoparticle system based on DDBA and ALC-0315. The molar percentages of lipid components are shown in Table 20.

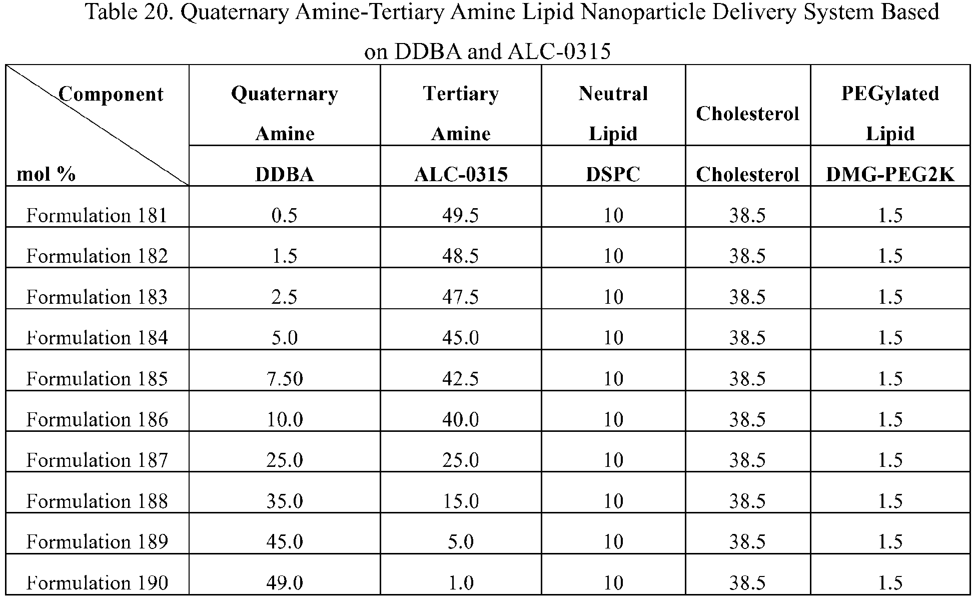

Example 28: A quaternary amine-tertiary amine lipid nanoparticle system based on DDBA and lipoid compound 5A2-SC8. The molar percentages of lipid components are shown in Table 21.

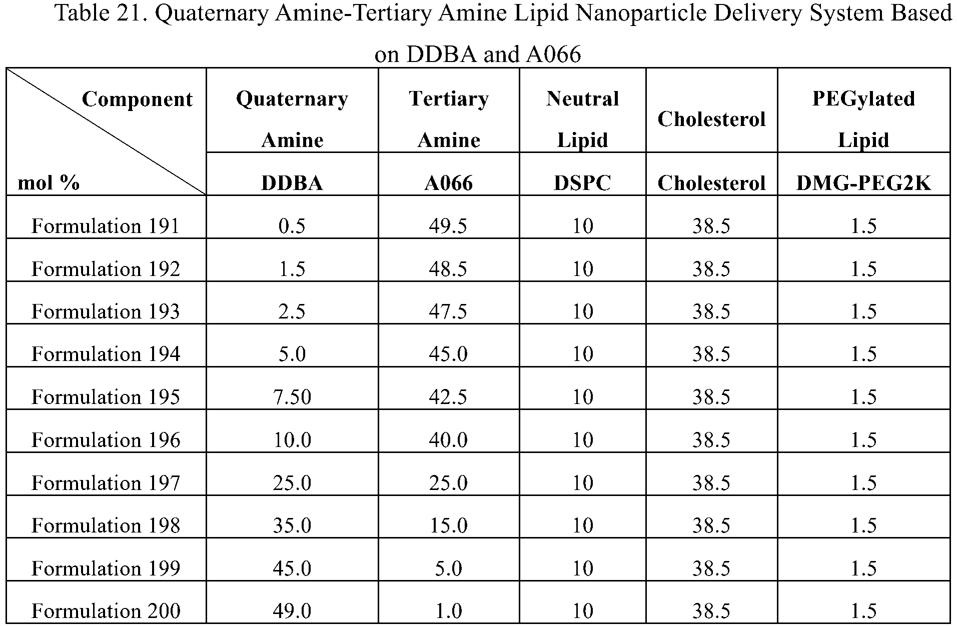

Example 29: A quaternary amine-tertiary amine lipid nanoparticle system based on DOTAP and SM-102. The molar percentages of lipid components are shown in Table 22.

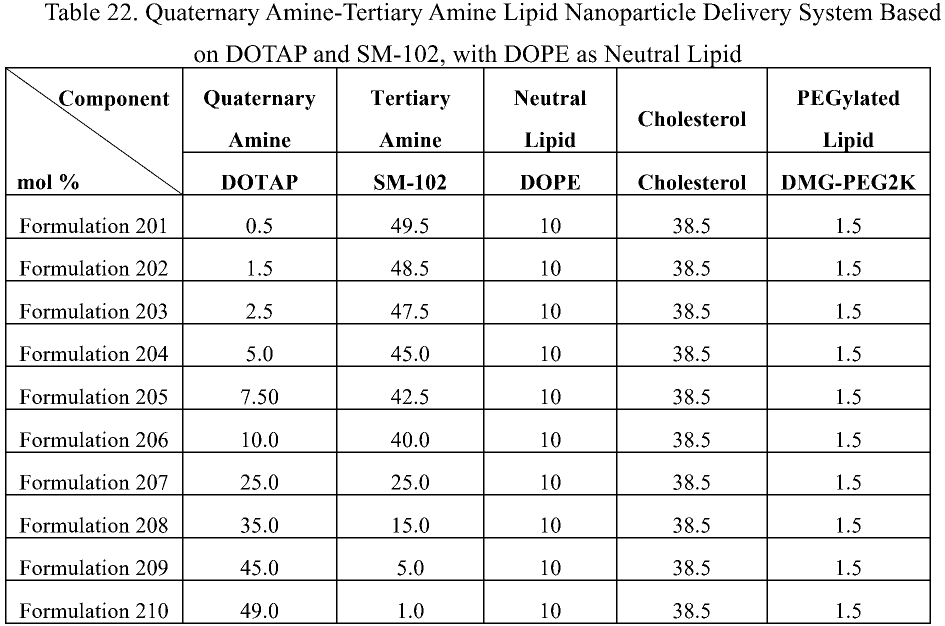

Example 30: A quaternary amine-tertiary amine lipid nanoparticle system based on DOTAP and DODMA. The molar percentages of lipid components are shown in Table 23.

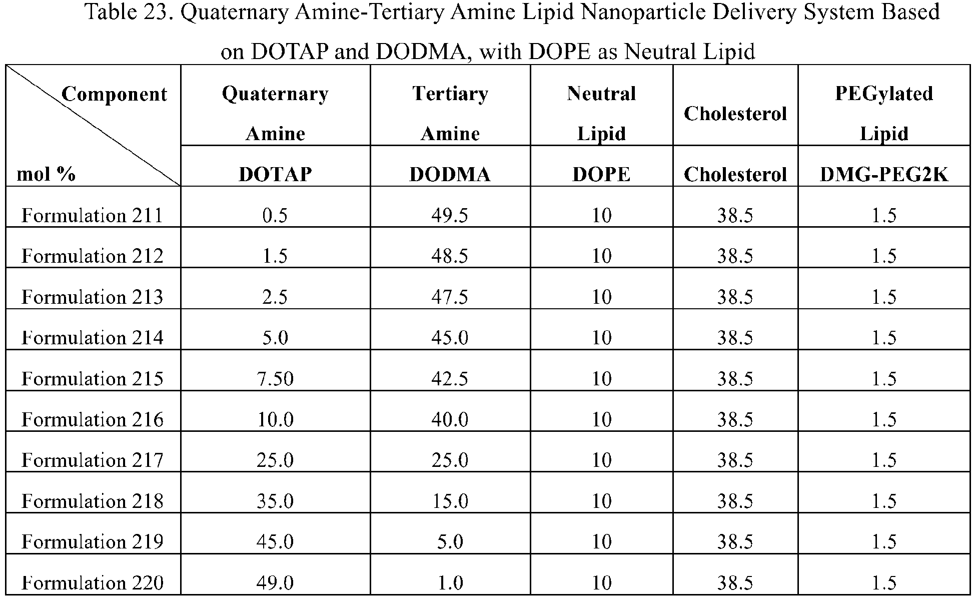

Example 31: A quaternary amine-tertiary amine lipid nanoparticle system based on DOTAP and DLin-MC3-DMA. The molar percentages of lipid components are shown in Table 24.

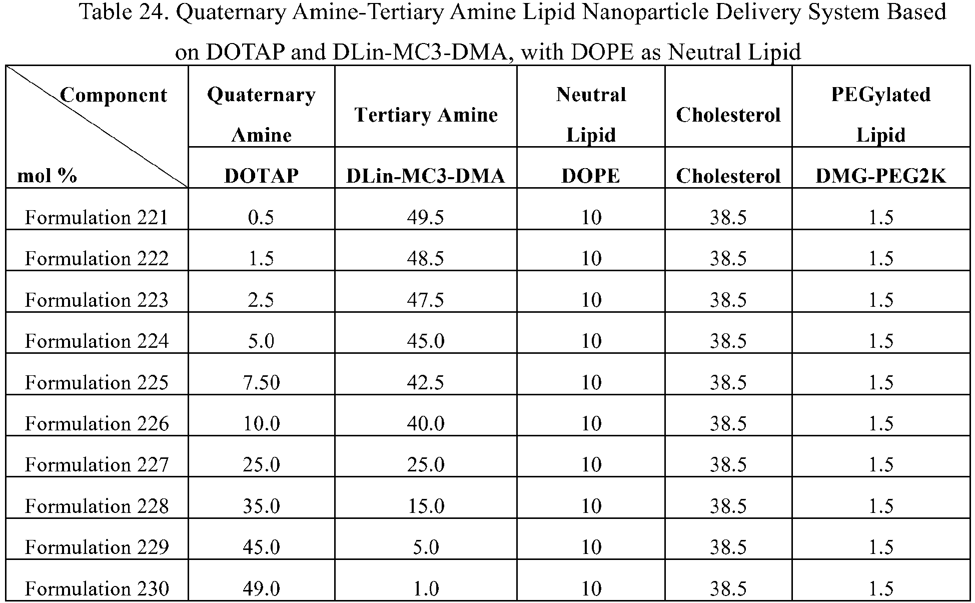

Example 32: A quaternary amine-tertiary amine lipid nanoparticle system based on DOTAP and ALC-0315. The molar percentages of lipid components are shown in Table 25.

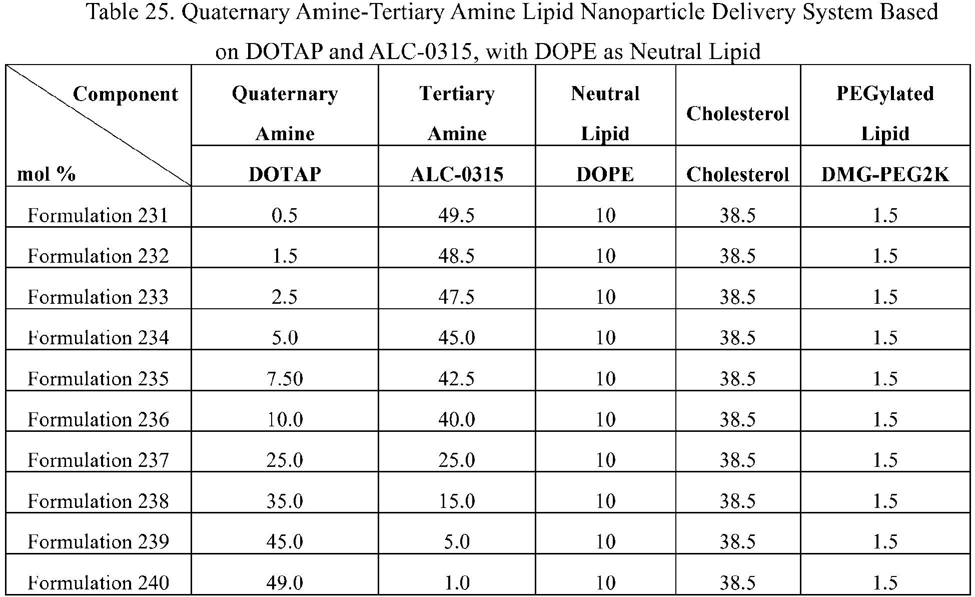

Example 33:A quaternary amine-tertiary amine lipid nanoparticle system based on DOTAP and A066. The molar percentages of lipid components are shown in Table 26.

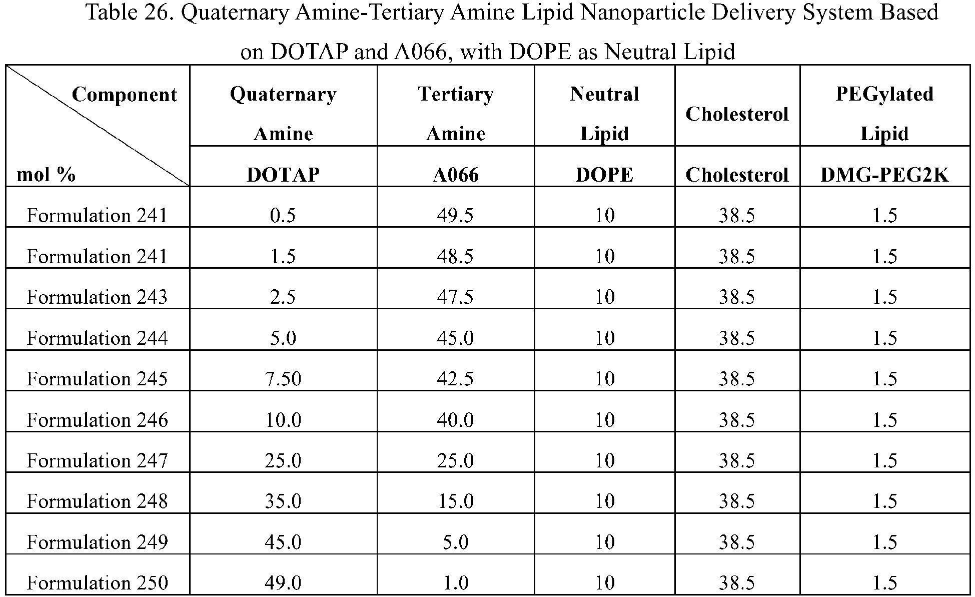

Example 34: A quaternary amine-tertiary amine lipid nanoparticle system based on DOTAP and SM-102. The molar percentages of lipid components are shown in Table 27.

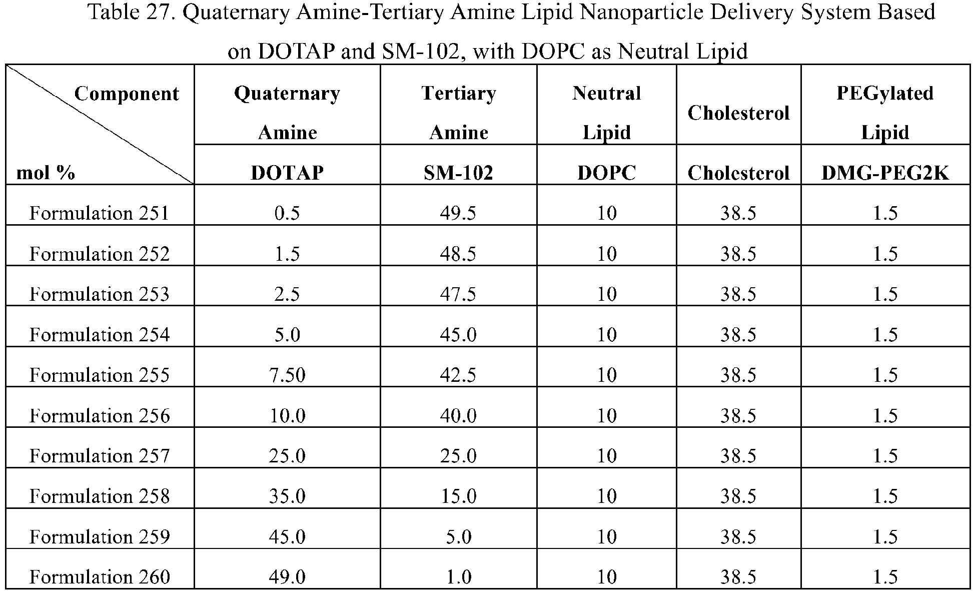

Example 35: A quaternary amine-tertiary amine lipid nanoparticle system based on DOTAP and DODMA. The molar percentages of lipid components are shown in Table 28.

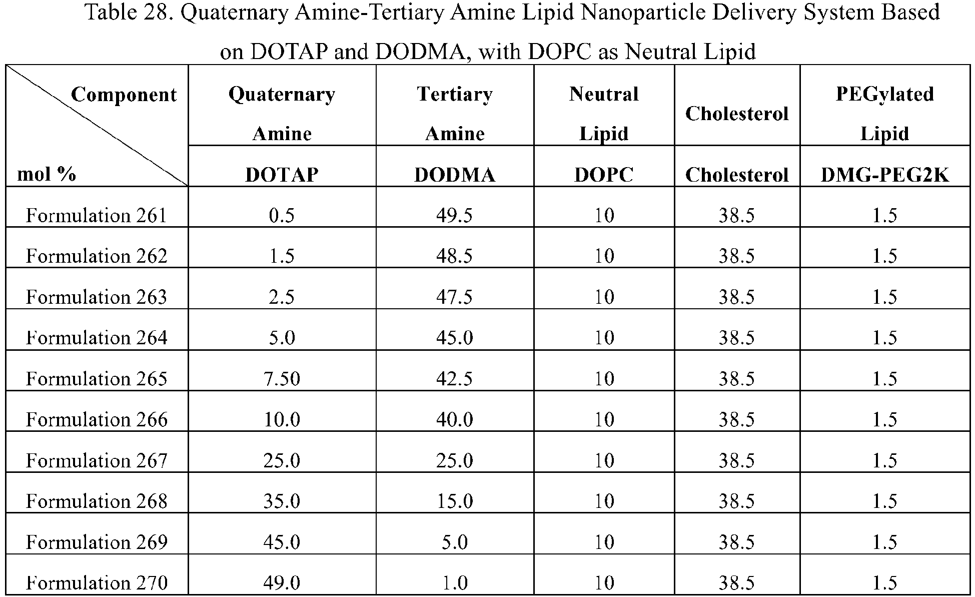

Example 36: A quaternary amine-tertiary amine lipid nanoparticle system based on DOTAP and DLin-MC3-DMA. The molar percentages of lipid components are shown in Table 29.

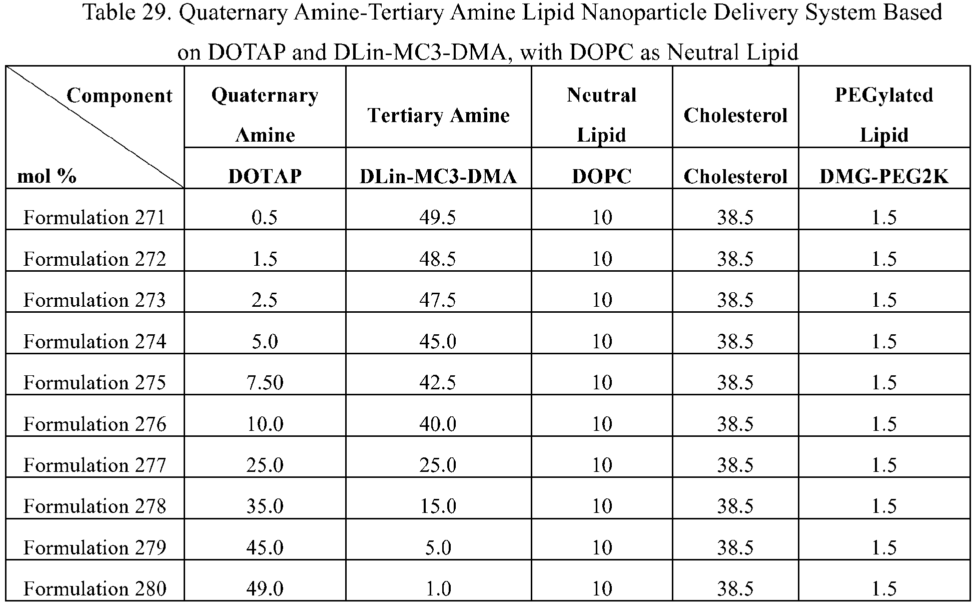

Example 37: A quaternary amine-tertiary amine lipid nanoparticle system based on DOTAP and ALC-0315. The molar percentages of lipid components are shown in Table 30.

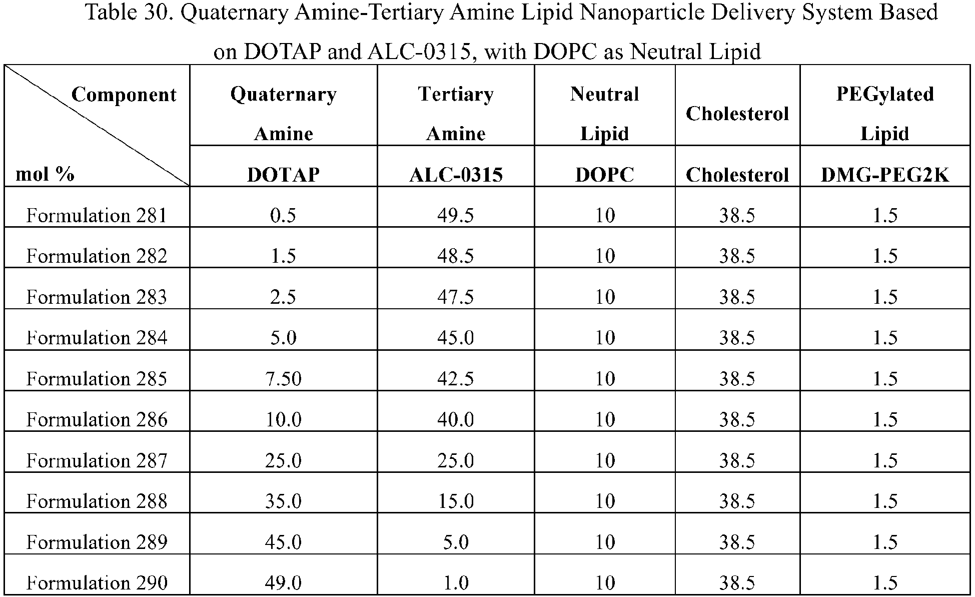

Example 38: A quaternary amine-tertiary amine lipid nanoparticle system based on DOTAP and A066. The molar percentages of lipid components are shown in Table 31.

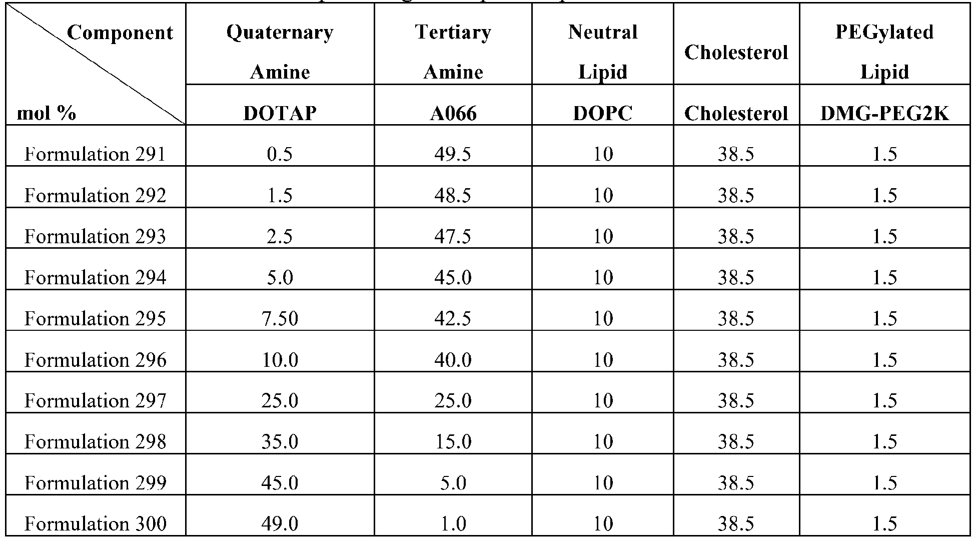

### Examples 39-42. Archexin-Encapsulating Lipid Nanoparticle Delivery Systems Based on Quaternary Amine/Tetraamine/Quaternary Ammonium and Tertiary Amine/Triamine/Tertiary Ammonium Systems

In Examples 39-42, the small nucleic acid drug Archexin encapsulated in the lipid nanoparticle delivery system is provided by Changzhou Hequan, with the sequence 5'-GCTGCATGATCTCCTTGGCG-3'. The mass ratio of RNA to total lipid is 1:10. The preparation method for Examples 40-42 is as described in Example 39. The LNP suspensions prepared in Examples 40-42 are tested for particle size and PDI on a laser particle size analyzer as per Example 39, and the prepared LNP suspensions can be used for cell evaluation and *in vivo* animal evaluation.

Example 39: A quaternary amine-tertiary amine lipid nanoparticle system based on DOTAP and SM-102. The molar percentages of lipid components are shown in Table 32.

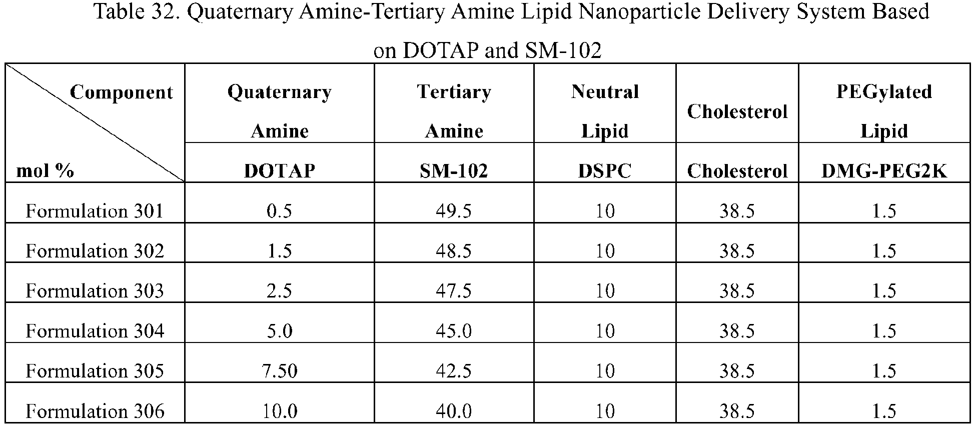

Preparation method is as follows:
1. An Archexin sodium acetate solution (pH=5.0, sodium acetate concentration 25 mM) with a concentration of 0.3334 mg/mL (calculated as Archexin free acid) was prepared using sodium acetate buffer (pH=5.0, sodium acetate concentration 50 mM) and the Archexin solution;
2. A lipid ethanol solution with a total lipid concentration of 10 mg/mL was prepared by adding the lipids described in the table above to anhydrous ethanol;
3. 0.75 mL of the Archexin sodium acetate solution (nucleic acid concentration 0.3334 mg/mL, total amount 0.25 mg) was pre-warmed in a 25°C water bath;
4. 0.25 mL of the lipid solution (total lipid concentration 10 mg/mL, total amount 2.5 mg) was pre-warmed in a 25°C water bath;
5. Under magnetic stirring in a 25°C water bath, the 0.25 mL lipid solution was injected into the Archexin sodium acetate solution, incubated for 1 min, then diluted to four times the volume with sodium acetate buffer (pH=5.0, concentration 25 mM) to obtain the initial Archexin-loaded LNP solution;
6. If a larger quantity is needed, steps 1-5 can be repeated and the initial Archexin-loaded LNP solutions merged;
7. Ethanol was removed by centrifugal ultrafiltration using an ultrafiltration tube with a molecular weight cutoff of 100 KD at 4°C with a centrifugal acceleration of 2000 g. The nucleic acid concentration was adjusted to an appropriate level using 25 mM Tris buffer, and the pH was adjusted to 7.0 with 0.1 M sodium hydroxide solution to obtain the Archexin-loaded LNP suspension;
50 µL of the Archexin-loaded LNP suspension was added to 950 µL of pure water, then the particle size and PDI were measured on a Malvern ZETASIZER PRO laser particle size analyzer. The prepared Archexin-loaded LNP suspension can be used for cell evaluation and *in vivo* animal evaluation.

Example 40: A quaternary amine-tertiary amine lipid nanoparticle system based on DOTAP and DODMA. The molar percentages of lipid components are shown in Table 33.

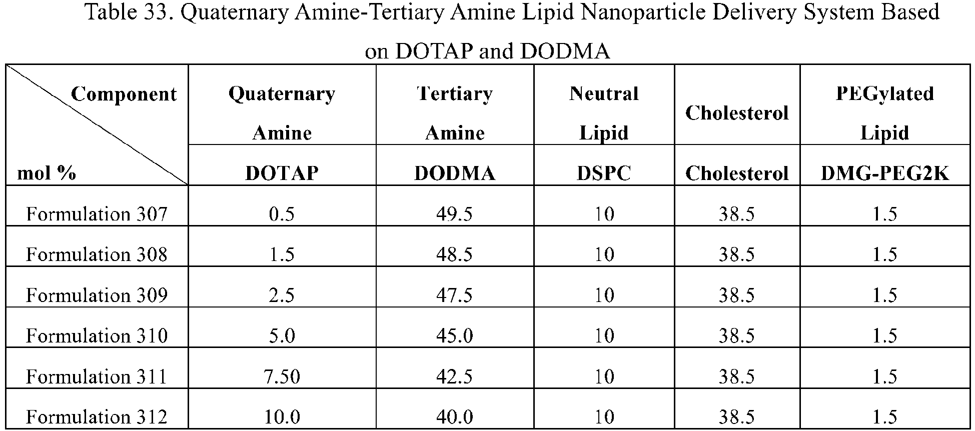

Example 41: A quaternary amine-tertiary amine lipid nanoparticle system based on DOTAP and DLin-MC3-DMA. The molar percentages of lipid components are shown in Table 34.

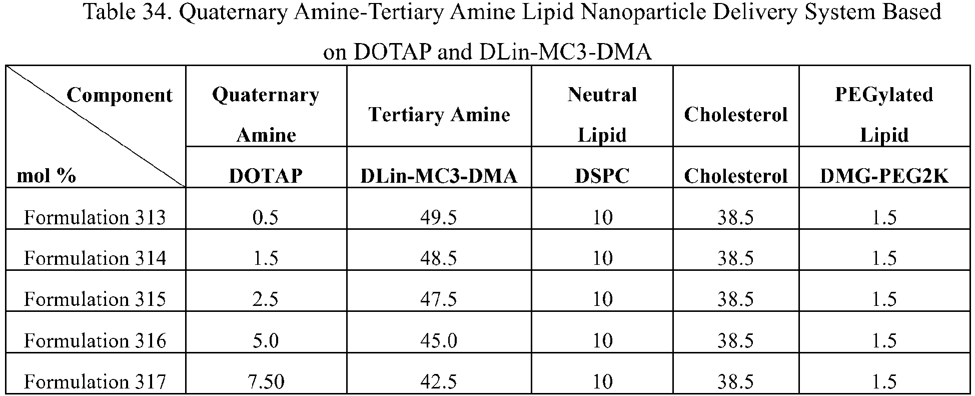

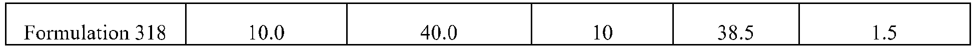

Example 42: A quaternary amine-tertiary amine lipid nanoparticle system based on DOTAP and A066. The molar percentages of lipid components are shown in Table 35.

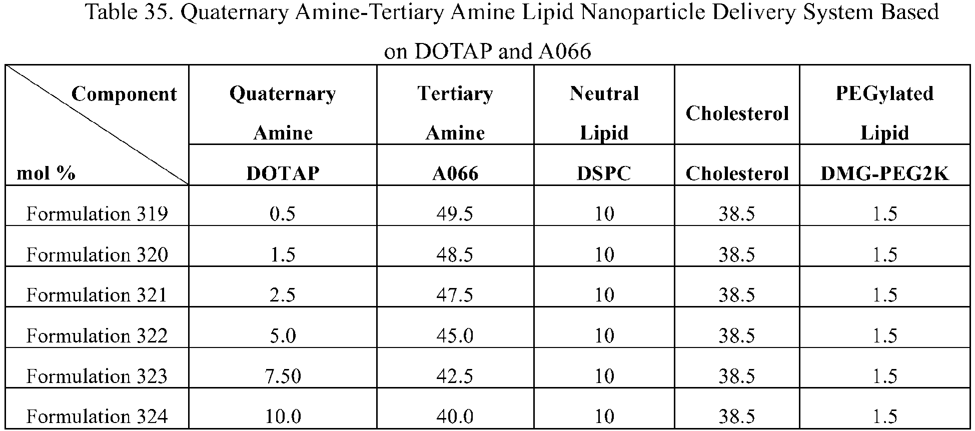

### Examples 43-46. Doxorubicin-Encapsulating Lipid Nanoparticle Delivery Systems Based on Quaternary Amine/Tetraamine/Quaternary Ammonium and Tertiary Amine/Triamine/Tertiary Ammonium Systems

In Examples 43-46, the small molecule drug Doxorubicin Hydrochloride encapsulated in the lipid nanoparticle delivery system is provided by Shanghai Yuanye Bio-Technology. The mass ratio of doxorubicin to total lipid is 1:10. The preparation method for Examples 44-46 is as described in Example 43. The LNP suspensions prepared in Examples 44-46 are tested for particle size and PDI on a laser particle size analyzer as per Example 43, and the prepared LNP suspensions can be used for cell evaluation and *in vivo* animal evaluation.

Example 43: A quaternary amine-tertiary amine lipid nanoparticle system based on DOTAP and SM-102. The molar percentages of lipid components are shown in Table 36.

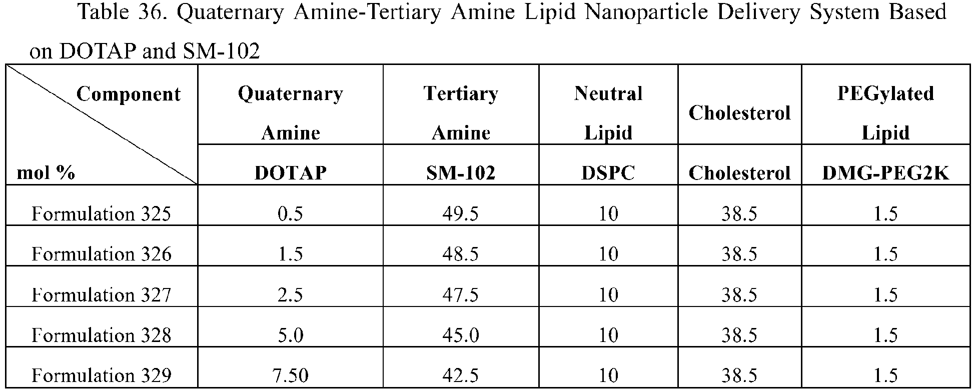

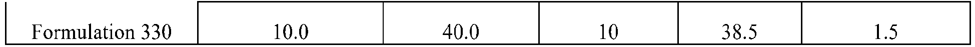

Preparation method is as follows:
1. A lipid ethanol solution with a total lipid concentration of 15 mg/mL was prepared by adding the lipids described in the table above to anhydrous ethanol;
2. 0.75 mL of citrate buffer at pH 4.0 (citrate concentration 25 mM) was pre-warmed in a 25°C water bath;
3. 0.25 mL of the lipid solution (total lipid concentration 10 mg/mL, total amount 2.5 mg) was pre-warmed in a 25°C water bath;
4. Under magnetic stirring in a 25°C water bath, the 0.25 mL lipid solution was injected into the citrate buffer at pH 4.0 (citrate concentration 25 mM), incubated for 1 min, then diluted to four times the volume with PBS buffer at pH 7.4 to obtain the initial blank LNP solution;
5. If a larger quantity is needed, steps 1-4 can be repeated and the initial blank LNP solutions merged;
6. Ethanol was removed by centrifugal ultrafiltration using an ultrafiltration tube with a molecular weight cutoff of 100 KD at 4°C with a centrifugal acceleration of 2000 g. Ultrafiltration and buffer exchange were performed five times with PBS buffer at pH 7.4 to obtain the blank LNP suspension;
7. A doxorubicin PBS solution with a concentration of 25 mg/mL was prepared using PBS buffer at pH 7.4 and doxorubicin hydrochloride, and setting aside;
8. The doxorubicin PBS solution prepared in step 7 and the blank LNP suspension prepared in step 6 were mixed at a mass ratio of total lipid to doxorubicin mass of 10:1, incubated at 37°C in a water bath for 2 h in the dark, then ultrafiltration and buffer exchange were performed five times with PBS buffer at pH 7.4 to obtain the doxorubicin-loaded LNP suspension;
50 µL of the doxorubicin-loaded LNP suspension was added to 950 µL of pure water, then the particle size and PDI were measured on a laser particle size analyzer. The prepared doxorubicin-loaded LNP suspension can be used for *in vivo* animal evaluation.

Example 44: A quaternary amine-tertiary amine lipid nanoparticle system based on DOTAP and DODMA. The molar percentages of lipid components are shown in Table 37.

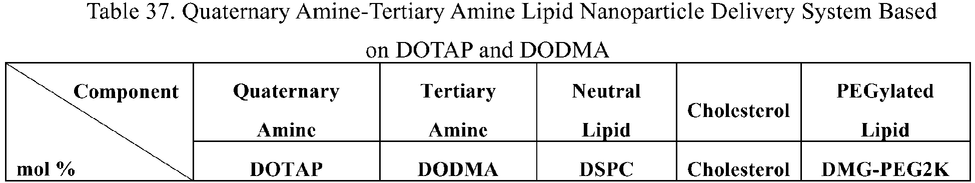

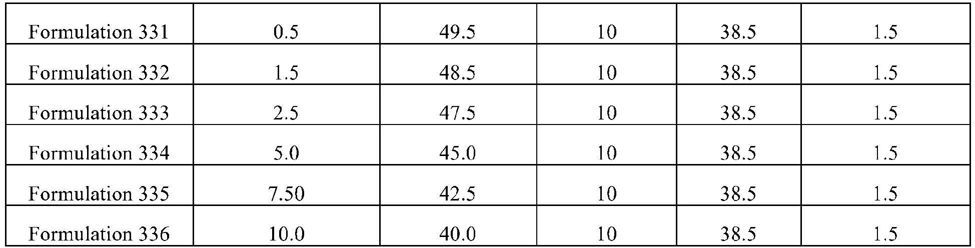

Example 45: A quaternary amine-tertiary amine lipid nanoparticle system based on DOTAP and DLin-MC3-DMA. The molar percentages of lipid components are shown in Table 38.

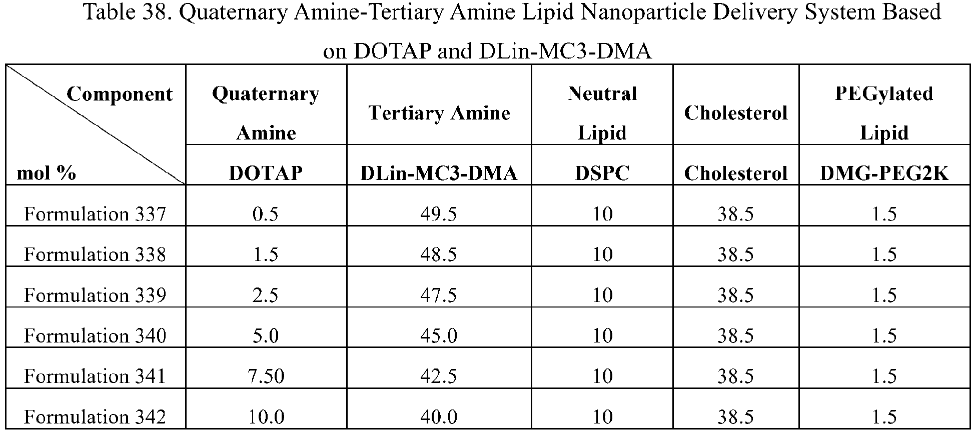

Example 46: A quaternary amine-tertiary amine lipid nanoparticle system based on DOTAP and ALC-0315. The molar percentages of lipid components are shown in Table 39.

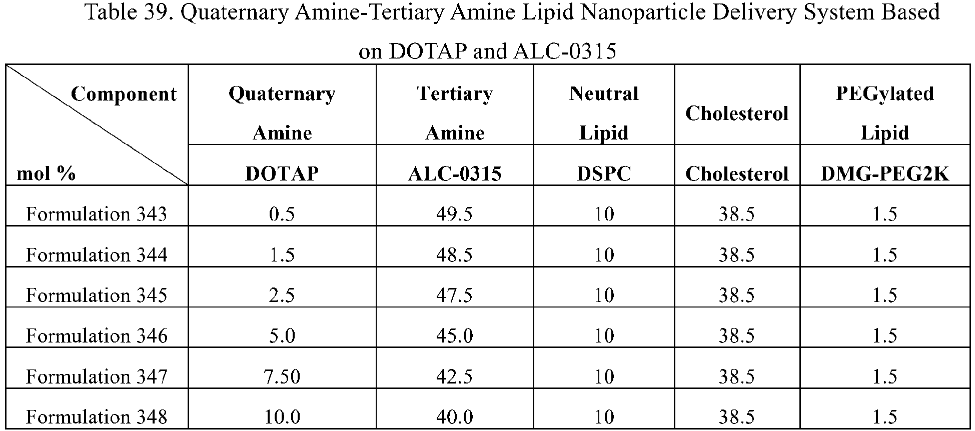

### Embodiment 47. Particle Size and PDI Characterization Results of Formulations

The characterization results for the 348 formulations in Examples 9-46 are shown in Table 40 below.

**Table 40. Particle Size and PDI Characterization of 348 Formulations**

| **Example 9** | **Particle Size** | **PDI** | **Example 10** | **Particle Size** | **PDI** | **Example 11** | **Particle Size** | **PDI** | **Example 12** | **Particle Size** | **PDI** |
|---|---|---|---|---|---|---|---|---|---|---|---|
| Formulation 1 | 95.91 | 0.1017 | Formulation 11 | 117.52 | 0.1602 | Formulation 21 | 108.00 | 0.1107 | Formulation 31 | 80.93 | 0.1943 |
| Formulation 2 | 81.16 | 0.1425 | Formulation 12 | 103.91 | 0.1183 | Formulation 22 | 107.45 | 0.1555 | Formulation 32 | 93.50 | 0.1948 |
| Formulation 3 | 94.85 | 0.1977 | Formulation 13 | 101.45 | 0.1751 | Formulation 23 | 85.41 | 0.1400 | Formulation 33 | 119.14 | 0.1668 |
| Formulation 4 | 112.15 | 0.1118 | Formulation 14 | 84.31 | 0.1479 | Formulation 24 | 109.81 | 0.1483 | Formulation 34 | 95.86 | 0.1933 |
| Formulation 5 | 92.39 | 0.1621 | Formulation 15 | 98.79 | 0.1059 | Formulation 25 | 109.21 | 0.1876 | Formulation 35 | 103.18 | 0.1185 |
| Formulation 6 | 93.10 | 0.1343 | Formulation 16 | 114.82 | 0.1141 | Formulation 26 | 86.52 | 0.1863 | Formulation 36 | 100.89 | 0.1583 |
| Formulation 7 | 82.58 | 0.0885 | Formulation 17 | 107.56 | 0.1174 | Formulation 27 | 89.81 | 0.1169 | Formulation 37 | 114.40 | 0.1988 |
| Formulation 8 | 102.53 | 0.1660 | Formulation 18 | 89.70 | 0.0940 | Formulation 28 | 99.77 | 0.1273 | Formulation 38 | 92.87 | 0.1186 |
| Formulation 9 | 98.84 | 0.0963 | Formulation 19 | 96.08 | 0.1389 | Formulation 29 | 87.89 | 0.1108 | Formulation 39 | 115.47 | 0.1061 |
| Formulation 10 | 106.24 | 0.1550 | Formulation 20 | 84.31 | 0.1252 | Formulation 30 | 112.73 | 0.1791 | Formulation 40 | 89.74 | 0.0800 |

| **Example 13** | **Particle Size** | **PDI** | **Example 14** | **Particle Size** | **PDI** | **Example 15** | **Particle Size** | **PDI** | **Example 16** | **Particle Size** | **PDI** |
|---|---|---|---|---|---|---|---|---|---|---|---|
| Formulation 41 | 90.06 | 0.1673 | Formulation 51 | 87.58 | 0.1593 | Formulation 61 | 123.17 | 0.1146 | Formulation 71 | 121.22 | 0.1859 |
| Formulation 42 | 84.18 | 0.1599 | Formulation 52 | 115.12 | 0.1244 | Formulation 62 | 110.11 | 0.1180 | Formulation 72 | 92.05 | 0.1116 |
| Formulation 43 | 93.92 | 0.1130 | Formulation 53 | 106.28 | 0.1429 | Formulation 63 | 129.26 | 0.1728 | Formulation 73 | 105.68 | 0.1182 |
| Formulation 44 | 103.97 | 0.1944 | Formulation 54 | 85.25 | 0.1132 | Formulation 64 | 120.79 | 0.1673 | Formulation 74 | 111.04 | 0.1375 |
| Formulation 45 | 88.60 | 0.1776 | Formulation 55 | 92.04 | 0.1375 | Formulation 65 | 115.98 | 0.0859 | Formulation 75 | 116.88 | 0.1024 |
| Formulation 46 | 96.40 | 0.1027 | Formulation 56 | 99.16 | 0.1193 | Formulation 66 | 108.25 | 0.1374 | Formulation 76 | 101.20 | 0.1698 |
| Formulation 47 | 106. 03 | 0.19 60 | Formulation 57 | 90.2 2 | 0.08 84 | Formulation 67 | 101.61 | 0.15 61 | Formulation 77 | 111.34 | 0.18 28 |
| Formulation 48 | 100. 47 | 0.15 13 | Formulation 58 | 88.9 6 | 0.13 95 | Formulation 68 | 99.82 | 0.15 04 | Formulation 78 | 114.82 | 0.16 10 |
| Formulation 49 | 117.2 3 | 0.12 34 | Formulation 59 | 107. 87 | 0.19 57 | Formulation 69 | 125.26 | 0.19 38 | Formulation 79 | 113.24 | 0.09 25 |
| Formulation 50 | 106. 55 | 0.13 83 | Formulation 60 | 107. 37 | 0.19 06 | Formulation 70 | 126.41 | 0.08 12 | Formulation 80 | 97.85 | 0.13 02 |

| **Example 17** | **Particle Size** | **PDI** | **Example 18** | **Particle Size** | **PDI** | **Example 19** | **Particle Size** | **PDI** | **Example 20** | **Particle Size** | **PDI** |
|---|---|---|---|---|---|---|---|---|---|---|---|
| Formulation 81 | 118.8 4 | 0.19 54 | Formulation 91 | 105. 70 | 0.14 83 | Formulation 101 | 92.43 | 0.09 80 | Formulation 111 | 103.71 | 0.18 44 |
| Formul ation 82 | 105. 29 | 0.17 56 | Formul ation 92 | 105. 10 | 0.12 12 | Formul ation 102 | 108.68 | 0.08 61 | Formul ation 112 | 128.59 | 0.16 21 |
| Formul ation 83 | 103. 06 | 0.15 60 | Formul ation 93 | 102. 85 | 0.16 74 | Formul ation 103 | 107.05 | 0.18 69 | Formul ation 113 | 110.31 | 0.12 16 |
| Formul ation 84 | 120. 20 | 0.13 32 | Formul ation 94 | 115.9 4 | 0.12 30 | Formul ation 104 | 98.22 | 0.18 73 | Formul ation 114 | 124.49 | 0.13 22 |
| Formul ation 85 | 112.1 4 | 0.11 22 | Formul ation 95 | 124. 32 | 0.18 43 | Formul ation 105 | 101.43 | 0.14 16 | Formul ation 115 | 94.43 | 0.18 15 |
| Formul ation 86 | 123. 79 | 0.14 53 | Formul ation 96 | 110.4 0 | 0.13 78 | Formul ation 106 | 113.97 | 0.19 07 | Formul ation 116 | 125.53 | 0.17 82 |
| Formulation 87 | 108.67 | 0.1542 | Formulation 97 | 106.27 | 0.1962 | Formulation 107 | 105.57 | 0.1462 | Formulation 117 | 125.39 | 0.1014 |
| Formulation 88 | 123. 04 | 0.15 80 | Formulation 98 | 92.5 2 | 0.11 37 | Formulation 108 | 114.21 | 0.10 24 | Formulation 118 | 114.14 | 0.17 14 |
| Formulation 89 | 129. 26 | 0.09 35 | Formulation 99 | 128. 74 | 0.13 06 | Formulation 109 | 109.25 | 0.09 71 | Formulation 119 | 91.74 | 0.17 99 |
| Formulation 90 | 91.5 4 | 0.12 83 | Formulation 100 | 99.7 4 | 0.17 69 | Formulation 110 | 119.45 | 0.12 05 | Formulation 120 | 123.73 | 0.18 96 |

| **Example 21** | **Particle Size** | **PDI** | **Example 22** | **Particle Size** | **PDI** | **Example 23** | **Particle Size** | **PDI** | **Example 24** | **Fluorescence** | **SD** |
|---|---|---|---|---|---|---|---|---|---|---|---|
| Formulation 121 | 101. 69 | 0.19 81 | Formulation 131 | 114.7 0 | 0.09 46 | Formulation 141 | 93.64 | 0.15 19 | Formulation 151 | 109.33 | 0.19 83 |
| Formulation 122 | 110.8 0 | 0.13 31 | Formulation 132 | 124. 74 | 0.18 56 | Formulation 142 | 109.67 | 0.12 44 | Formulation 152 | 117.21 | 0.24 41 |
| Formulation 123 | 94.9 0 | 0.14 15 | Formulation 133 | 106. 36 | 0.17 43 | Formulation 143 | 112.16 | 0.15 57 | Formulation 153 | 135.76 | 0.16 84 |
| Formulation 124 | 110.6 9 | 0.14 99 | Formulation 134 | 109. 55 | 0.08 11 | Formulation 144 | 113.35 | 0.14 76 | Formulation 154 | 134.95 | 0.23 65 |
| Formulation 125 | 93.8 1 | 0.13 47 | Formulation 135 | 90.3 9 | 0.17 97 | Formulation 145 | 91.60 | 0.19 04 | Formulation 155 | 115.63 | 0.20 70 |
| Formulation 126 | 109. 86 | 0.12 68 | Formulation 136 | 114.6 0 | 0.13 73 | Formulation 146 | 111.64 | 0.09 24 | Formulation 156 | 101.91 | 0.20 40 |
| Formulation 127 | 111.0 0 | 0.15 13 | Formulation 137 | 124. 84 | 0.10 93 | Formulation 147 | 123.38 | 0.08 89 | Formulation 157 | 100.38 | 0.15 96 |
| Formulation 128 | 96.1 8 | 0.15 45 | Formulation 138 | 97.6 0 | 0.09 30 | Formulation 148 | 117.02 | 0.09 59 | Formulation 158 | 116.56 | 0.13 25 |
| Formulation 129 | 103. 41 | 0.14 57 | Formulation 139 | 94.1 6 | 0.11 15 | Formulation 149 | 114.46 | 0.18 00 | Formulation 159 | 107.44 | 0.24 54 |
| Formulation 130 | 126. 61 | 0.19 00 | Formulation 140 | 126. 64 | 0.11 45 | Formulation 150 | 105.64 | 0.19 14 | Formulation 160 | 137.45 | **0.14** 78 |

| **Example 25** | **Particle Size** | **PDI** | **Example 26** | **Particle Size** | **PDI** | **Example 27** | **Fluorescence** | **SD** | **Example 28** | **Particle Size** | **PDI** |
|---|---|---|---|---|---|---|---|---|---|---|---|
| Formulation 161 | 139. 30 | 0.19 10 | Formulation 171 | 103. 79 | 0.19 08 | Formulation 181 | 128.81 | 0.24 32 | Formulation 191 | 129.925 | 0.21 16 |
| Formulation 162 | 134. 79 | 0.12 41 | Formulation 172 | 108. 02 | 0.16 15 | Formulation 182 | 129.33 | 0.18 08 | Formulation 192 | 120.269 | 0.20 11 |
| Formulation 163 | 116.1 2 | 0.09 74 | Formulation 173 | 104. 16 | 0.18 83 | Formul ation 183 | 104.32 | 0.22 84 | Formulation 193 | 113.249 | 0.10 51 |
| Formulation 164 | 116.2 1 | 0.14 12 | Formulation 174 | 107. 14 | 0.09 84 | Formulation 184 | 137.01 | 0.20 89 | Formulation 194 | 122.157 | 0.09 96 |
| Formulation 165 | 126. 58 | 0.22 73 | Formulation 175 | 127. 37 | 0.23 77 | Formulation 185 | 130.08 | 0.16 15 | Formulation 195 | 121.486 | 0.17 45 |
| Formulation 166 | 119.2 4 | 0.19 14 | Formulation 176 | 114.5 2 | 0.12 18 | Formulation 186 | 105.03 | 0.24 89 | Formulation 196 | 127.734 | 0.14 76 |
| Formulation 167 | 123. 86 | 0.09 18 | Formulation 177 | 138. 76 | 0.18 90 | Formulation 187 | 111.87 | 0.13 52 | Formulation 197 | 135.050 | 0.12 03 |
| Formulation 168 | 137. 41 | 0.23 85 | Formulation 178 | 121. 48 | 0.23 25 | Formulation 188 | 122.70 | 0.18 24 | Formulation 198 | 103.637 | 0.24 65 |
| Formulation 169 | 139. 17 | 0.15 47 | Formulation 179 | 106. 09 | 0.16 17 | Formulation 189 | 135.62 | 0.21 65 | Formulation 199 | 110.263 | 0.18 68 |
| Formulation 170 | 139. 09 | 0.24 95 | Formulation 180 | 113.4 5 | 0.11 74 | Formulation 190 | 113.46 | 0.21 99 | Formulation 200 | 134.635 | 0.22 51 |

| **Example 29** | **Particle Size** | **PDI** | **Example 30** | **Particle Size** | **PDI** | **Example 31** | **Particle Size** | **PDI** | **Example 32** | **Particle Size** | **PDI** |
|---|---|---|---|---|---|---|---|---|---|---|---|
| Formulation 201 | 105. 91 | 0.10 57 | Formulation 211 | 121. 63 | 0.15 84 | Formulation 221 | 111.98 | 0.16 44 | Formulation 231 | 122.60 | 0.15 14 |
| Formulation 202 | 101. 45 | 0.19 42 | Formulation 212 | 90.5 9 | 0.11 68 | Formulation 222 | 94.74 | 0.13 98 | Formulation 232 | 92.89 | 0.14 10 |
| Formulation 203 | 99.2 2 | 0.13 54 | Formulation 213 | 95.8 4 | 0.08 35 | Formulation 223 | 90.32 | 0.12 29 | Formulation 233 | 108.41 | 0.13 51 |
| Formulation 204 | 101. 79 | 0.18 18 | Formulation 214 | 113.8 0 | 0.19 92 | Formulation 224 | 88.38 | 0.16 86 | Formulation 234 | 91.52 | 0.14 57 |
| Formulation 205 | 118.2 0 | 0.09 83 | Formulation 215 | 116.7 7 | 0.19 44 | Formulation 225 | 107.59 | 0.16 93 | Formulation 235 | 112.86 | 0.12 03 |
| Formulation 206 | 82.8 9 | 0.13 78 | Formulation 216 | 100. 93 | 0.08 73 | Formulation 226 | 113.94 | 0.09 02 | Formulation 236 | 115.39 | 0.16 69 |
| Formulation 207 | 107. 50 | 0.08 68 | Formulation 217 | 99.7 8 | 0.12 50 | Formulation 227 | 118.20 | 0.17 60 | Formulation 237 | 101.10 | 0.18 85 |
| Formulation 208 | 88.3 2 | 0.16 20 | Formulation 218 | 118.8 8 | 0.16 72 | Formulation 228 | 90.64 | 0.13 20 | Formulation 238 | 99.02 | 0.09 39 |
| Formulation 209 | 107.40 | 0.1138 | Formulation 219 | 115.13 | 0.1999 | Formulation 229 | 96.21 | 0.0858 | Formulation 239 | 93.12 | 0.1181 |
| Formul ation 210 | 89.8 5 | 0.08 13 | Formul ation 220 | 124. 31 | 0.18 76 | Formul ation 230 | 112.87 | 0.11 67 | Formul ation 240 | 113.56 | 0.16 71 |

| **Examp le 33** | **Parti cle Size** | **PDI** | **Examp le 34** | **Parti cle Size** | **PDI** | **Examp le 35** | **Particle Size** | **PDI** | **Examp le 36** | **Particle Size** | **PDI** |
|---|---|---|---|---|---|---|---|---|---|---|---|
| Formul ation 241 | 108. 18 | 0.08 01 | Formul ation 251 | 88.0 9 | 0.08 73 | Formul ation 261 | 96.72 | 0.17 04 | Formul ation 271 | 107.79 | 0.10 83 |
| Formul ation 242 | 110.3 1 | 0.13 76 | Formul ation 252 | 98.6 9 | 0.13 39 | Formul ation 262 | 98.54 | 0.17 88 | Formul ation 272 | 82.79 | 0.17 23 |
| Formul ation 243 | 115.6 8 | 0.15 55 | Formul ation 253 | 83.6 0 | 0.09 01 | Formul ation 263 | 82.12 | 0.12 61 | Formul ation 273 | 87.70 | 0.19 99 |
| Formul ation 244 | 108. 31 | 0.08 85 | Formul ation 254 | 107. 28 | 0.16 56 | Formul ation 264 | 118.76 | 0.15 59 | Formul ation 274 | 85.53 | 0.16 46 |
| Formul ation 245 | 99.0 5 | 0.14 84 | Formul ation 255 | 85.7 6 | 0.13 70 | Formul ation 265 | 96.99 | 0.16 69 | Formul ation 275 | 100.84 | 0.17 18 |
| Formul ation 246 | 115.3 7 | 0.18 09 | Formul ation 256 | 106. 58 | 0.18 89 | Formul ation 266 | 106.36 | 0.16 36 | Formul ation 276 | 110.67 | 0.14 99 |
| Formul ation 247 | 116.6 9 | 0.09 45 | Formul ation 257 | 108. 56 | 0.18 94 | Formul ation 267 | 84.80 | 0.09 08 | Formul ation 277 | 82.20 | 0.08 94 |
| Formul ation 248 | 124. 67 | 0.11 53 | Formul ation 258 | 90.11 | 0.19 82 | Formul ation 268 | 96.39 | 0.09 34 | Formul ation 278 | 88.32 | 0.09 51 |
| Formul ation 249 | 91.0 5 | 0.19 80 | Formul ation 259 | 118.4 8 | 0.10 62 | Formul ation 269 | 81.42 | 0.16 08 | Formul ation 279 | 119.51 | 0.15 23 |
| Formul ation 250 | 99.7 2 | 0.17 66 | Formul ation 260 | 106. 48 | 0.16 11 | Formul ation 270 | 97.71 | 0.08 74 | Formul ation 280 | 116.94 | 0.14 07 |

| **Examp le 37** | **Parti cle Size** | **PDI** | **Examp le 38** | **Parti cle Size** | **PDI** | **Examp le 39** | **Particle Size** | **PDI** | **Examp le 40** | **Particle Size** | **PDI** |
|---|---|---|---|---|---|---|---|---|---|---|---|
| Formul ation 281 | 88.7 3 | 0.19 57 | Formul ation 291 | 95.8 31 | 0.14 65 | Formul ation 301 | 51.33 | 0.15 07 | Formul ation 307 | 35.58 | 0.09 98 |
| **Formul ation** 282 | 114.2 4 | 0.19 91 | Formul ation 292 | 111.5 69 | 0.11 45 | Formul ation 302 | 39.55 | 0.13 26 | Formul ation 308 | 59.62 | 0.09 07 |
| Formul ation 283 | 100. 14 | 0.18 85 | Formul ation 293 | 94.7 92 | 0.18 19 | Formul ation 303 | 56.83 | 0.11 82 | Formul ation 309 | 54.64 | 0.09 29 |
| Formul ation 284 | 98.1 2 | 0.17 21 | Formul ation 294 | 117.6 74 | 0.16 60 | Formul ation 304 | 41.91 | 0.12 14 | Formul ation 310 | 57.33 | 0.13 45 |
| Formul ation 285 | 90.0 6 | 0.17 27 | Formul ation 295 | 94.11 5 | 0.10 70 | Formul ation 305 | 47.26 | 0.15 95 | Formul ation 311 | 54.80 | 0.09 51 |
| Formul ation 286 | 105. 79 | 0.12 95 | Formul ation 296 | 94.2 72 | 0.16 42 | Formul ation 306 | 32.57 | 0.13 87 | Formul ation 312 | 43.16 | 0.11 08 |
| Formul ation 287 | 89.8 0 | 0.19 23 | Formul ation 297 | 110.3 84 | 0.08 95 | | | | | | |
| Formul ation 288 | 93.7 5 | 0.16 95 | Formul ation 298 | 101. 976 | 0.09 96 | | | | | | |
| Formul ation 289 | 89.2 2 | 0.14 64 | Formul ation 299 | 96.6 51 | 0.16 23 | | | | | | |
| Formul ation 290 | 81.9 5 | 0.13 48 | Formul ation 300 | 99.7 42 | 0.15 26 | | | | | | |

| **Examp le 41** | **Parti cle Size** | **PDI** | **Examp le 42** | **Parti cle Size** | **PDI** | **Examp le 43** | **Particle Size** | **PDI** | **Examp le 44** | **Particle Size** | **PDI** |
|---|---|---|---|---|---|---|---|---|---|---|---|
| Formul ation 313 | 47.1 6 | 0.08 95 | Formul ation 319 | 54.4 6 | 0.08 96 | Formul ation 325 | 133.67 | 0.13 36 | Formul ation 331 | 127.37 | 0.17 31 |
| Formul ation 314 | 40.3 9 | 0.15 90 | Formul ation 320 | 39.9 0 | 0.09 74 | Formul ation 326 | 136.21 | 0.13 63 | Formul ation 332 | 140.01 | 0.12 83 |
| Formul ation 315 | 45.2 9 | 0.12 69 | Formul ation 321 | 59.0 3 | 0.11 30 | Formul ation 327 | 134.84 | 0.13 96 | Formul ation 333 | 125.96 | 0.11 55 |
| Formul ation 316 | 58.7 8 | 0.10 55 | Formul ation 322 | 46.7 1 | 0.08 51 | Formul ation 328 | 128.93 | 0.14 22 | Formul ation 334 | 125.91 | 0.13 68 |
| Formul ation 317 | 53.6 0 | 0.09 96 | Formul ation 323 | 44.0 6 | 0.12 16 | Formul ation 329 | 125.42 | 0.13 24 | Formul ation 335 | 133.08 | 0.14 03 |
| Formul ation 318 | 31.9 1 | 0.08 89 | Formul ation 324 | 34.6 8 | 0.11 86 | Formul ation 330 | 136.18 | 0.14 43 | Formul ation 336 | 138.73 | 0.15 39 |

| **Examp le 45** | **Parti cle Size** | **PDI** | **Examp le 46** | **Parti cle Size** | **PDI** | **Examp le 45** | **Particle Size** | **PDI** | **Examp le 46** | **Particle Size** | **PDI** |
|---|---|---|---|---|---|---|---|---|---|---|---|
| Formul ation 337 | 125. 30 | 0.19 78 | Formul ation 343 | 130. 48 | 0.18 10 | Formul ation 340 | 125.16 | 0.09 30 | Formul ation 346 | 128.93 | 0.12 76 |
| Formul ation 338 | 135. 49 | 0.09 32 | Formul ation 344 | 132. 73 | 0.13 58 | Formul ation 341 | 135.89 | 0.12 94 | Formul ation 347 | 125.39 | 0.13 40 |
| Formul ation 339 | 141. 98 | 0.11 61 | Formul ation 345 | 132. 75 | 0.11 88 | Formul ation 342 | 132.90 | 0.09 33 | Formul ation 348 | 131.76 | 0.11 15 |

### Example 48. In Vivo and In Vitro Evaluation

### (a) In Vitro Experiments of Formulations Encapsulating Active Agents

*In vitro* cell experiments include *in vitro* cell expression assays and cell viability assays. The specific steps are as follows: Samples from Formulation 1 to Formulation 300 (containing 0.1 µg mRNA) were added to a 96-well plate pre-seeded with MC38 cells. After overnight incubation, Fluc-eGFP mRNA (5'CAP) transfection efficiency and cell viability were measured simultaneously using the ONE-Glo Luciferase Assay System (Promega) according to the manufacturer's instructions. Fluorescence and luminescence were quantified using a Tecan Infinite M200 Pro plate reader (Tecan). The post-transfection fluorescence intensity was normalized relative to Formulation 1 as 100%. Cell viability was normalized relative to the addition of blank PBS as 100%.

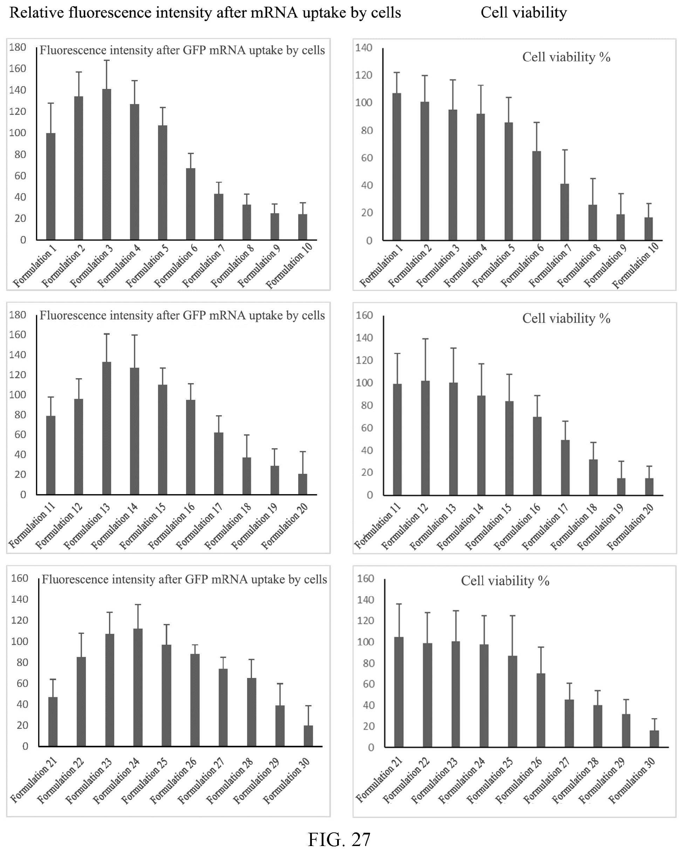

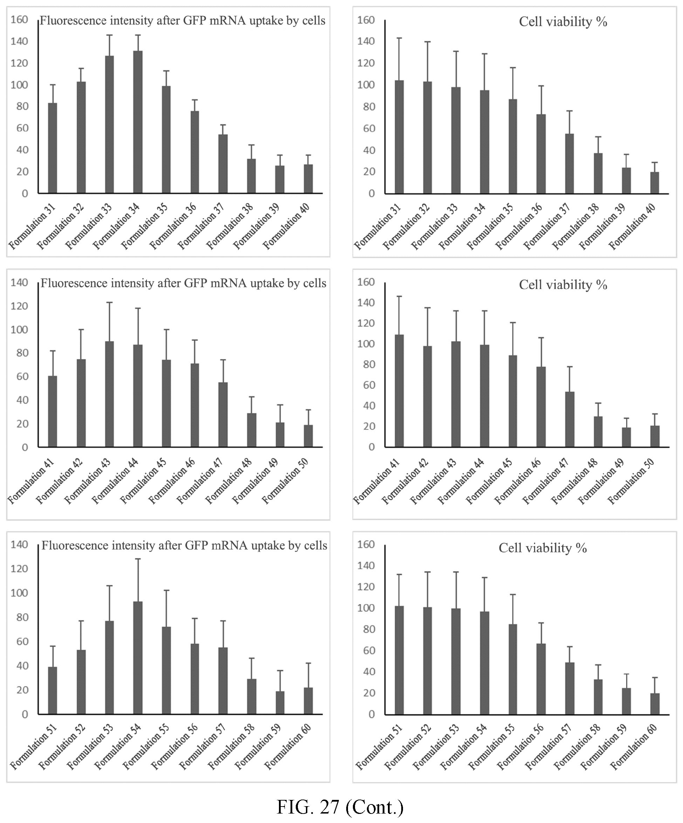

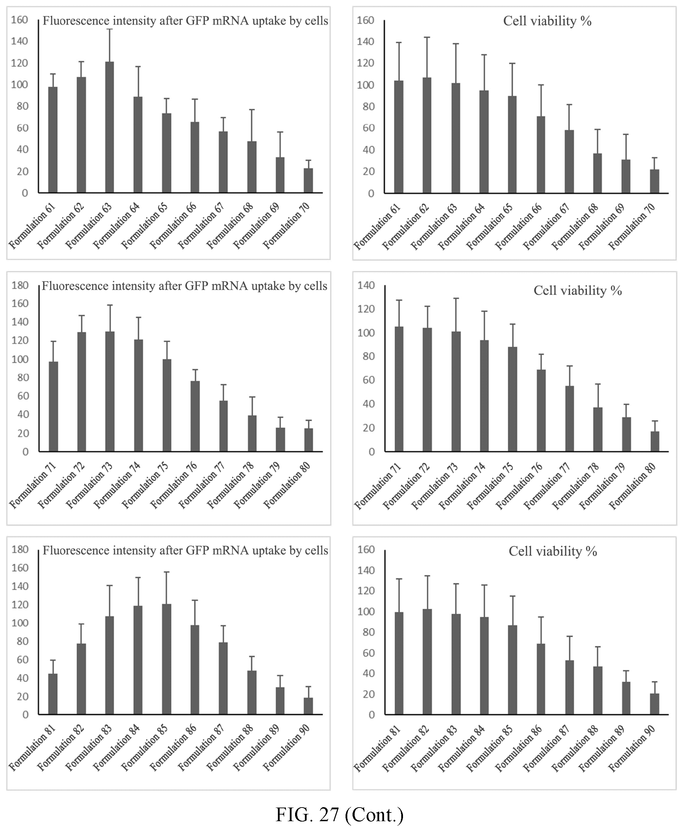

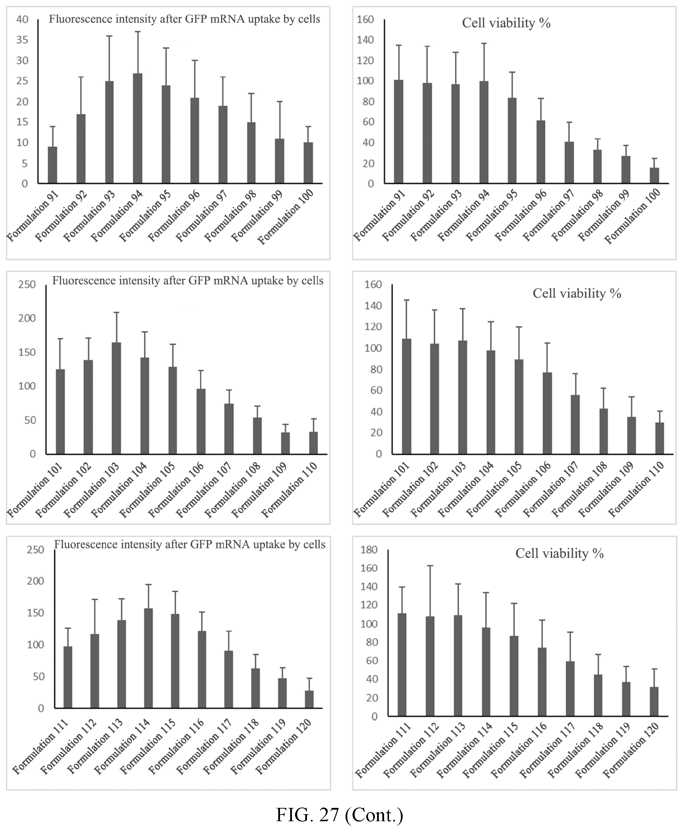

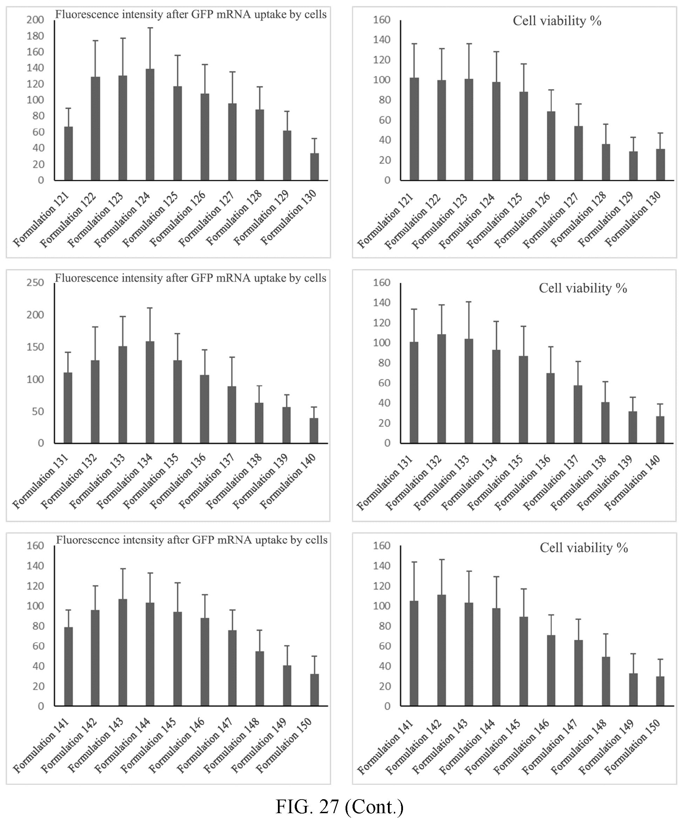

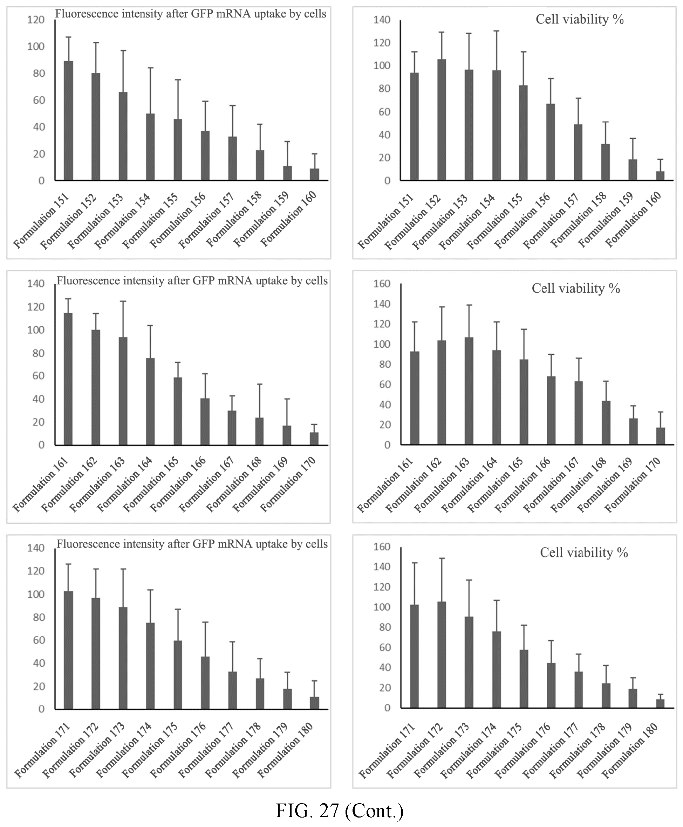

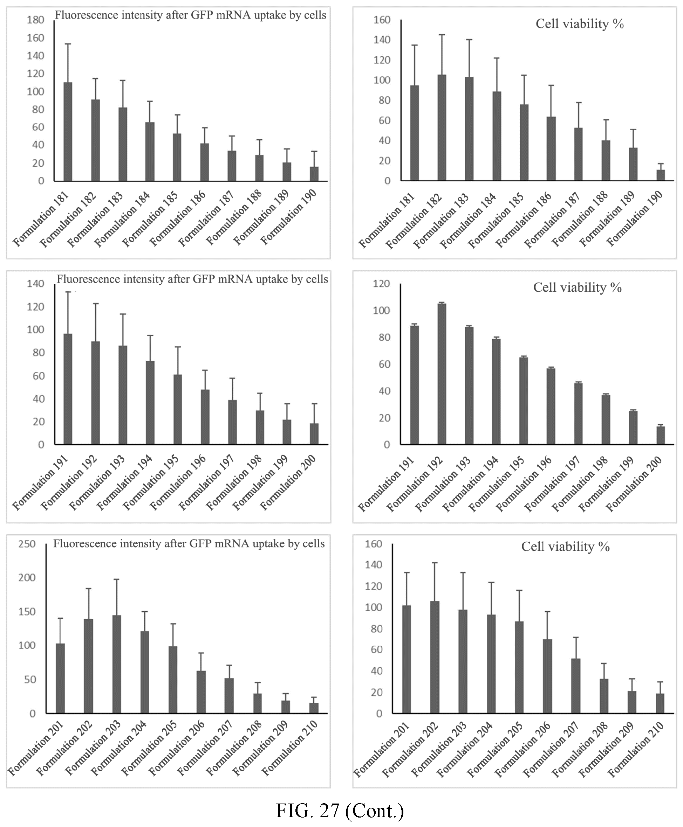

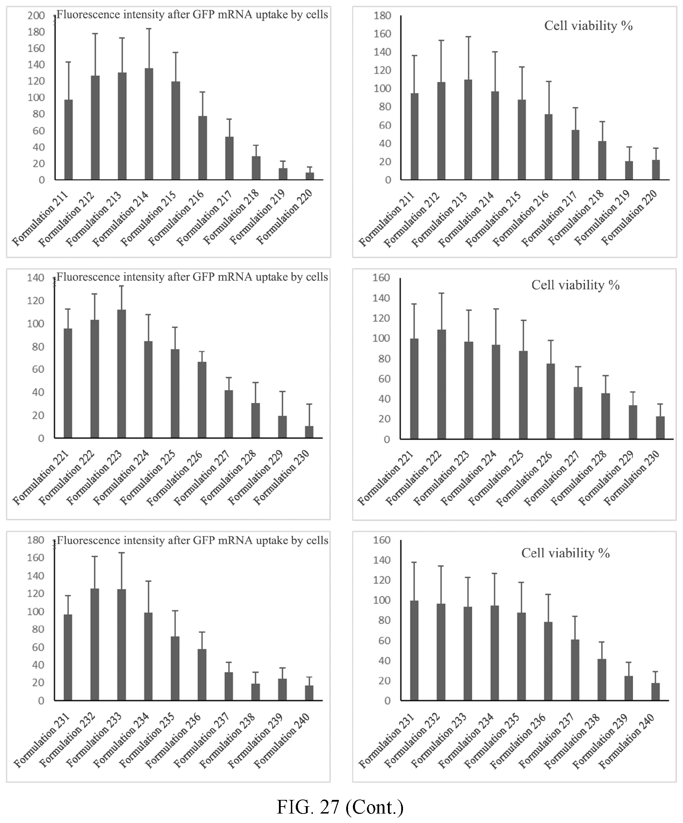

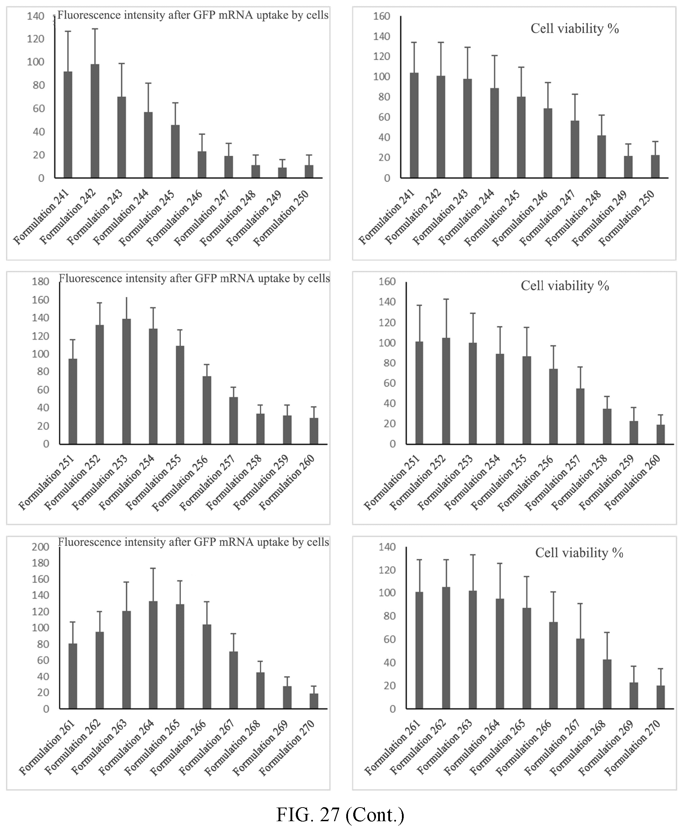

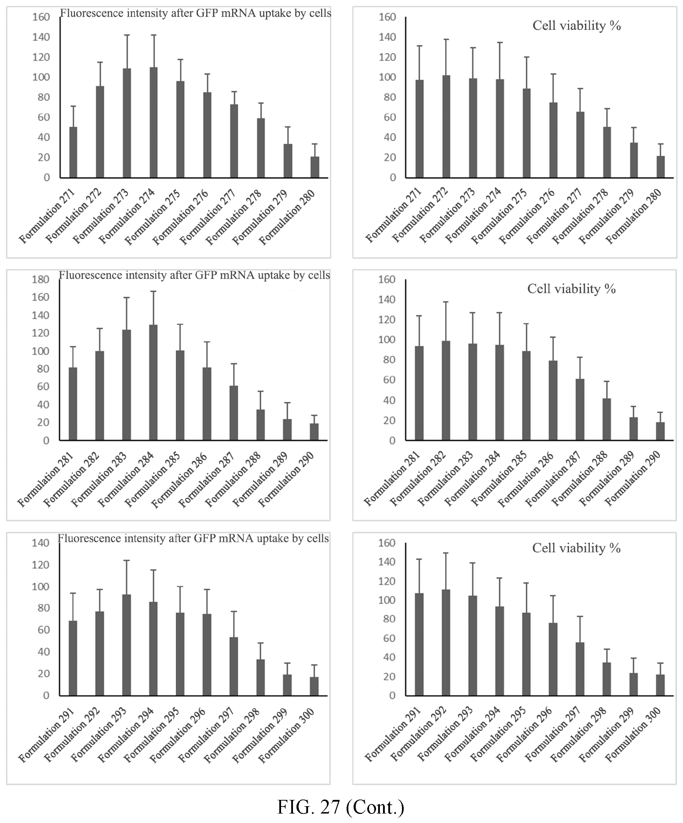

The results are shown in Figure 27. From the relative fluorescence intensity results, it can be seen that when the molar ratio of cationic lipid to ionizable lipid (mol:mol) is 1.5:48.5-7.5:42.5, a high level is maintained, while outside this ratio, fluorescence is weaker. Cell viability experiments show that when the molar ratio of cationic lipid to ionizable lipid is less than 7.5:42.5, cell viability can be maintained above 80%, indicating good safety.

### (b) Targeting Analysis of Formulations Encapsulating Active Agents in Non-Small Cell Lung Cancer A549 Cell Line Tumor Bearing Mice

In this experiment, quaternary amine-tertiary amine (QTsome) lipid nanoparticles were used to encapsulate HiSignal^{®} Fluc-eGFP mRNA (5'CAP) provided by Jiangsu Shenji Biotechnology, with a mass ratio of mRNA to total lipid of 1:20. The A549 cell line was used to construct tumor bearing mouse models and evaluate the tumor targeting of different formulations.

### Experimental Preparation Phase:

1) Different formulation preparations were aliquoted in advance and stored at -20°C, reconstituted on the day of use.
2) A549 cells were thawed, cultured *in vitro,* and passaged 2-3 times.
3) 360 seven-week-old BALB/c nude mice (equal male and female) were acclimatized for one week and weighed.

### Experimental Procedure:

1) Human non-small cell lung cancer A549 cells were thawed and passaged. When the cells were in good condition, A549 cells (inoculation amount 8×10⁶) were transplanted subcutaneously into mice, and changes in mouse body weight and tumor size were recorded (tumor bearing mouse model were constructed by subcutaneous implantation of the cell line on the back);
2) When tumors grew to approximately 500 mm³, mice were grouped based on tumor volume, with 6 mice per group;
3) Each mouse was intravenously administered 100 µl of different formulations encapsulating Fluc-eGFP mRNA at a dose of 50 µg (~2.5 mg kg⁻¹) Fluc-eGFP mRNA (5'CAP). Mice were sacrificed 12 hours post-injection, tumors were weighed, homogenized with an equal amount of 0.9% sodium chloride solution, and the homogenate was centrifuged at 1000 g. 200 µL of supernatant was taken, and eGFP fluorescence intensity was measured using a microplate reader. Fluorescence intensity per unit mass was calculated based on tumor weight, and the fluorescence intensity of each formulation was normalized relative to that of Formulation 1 as 100.

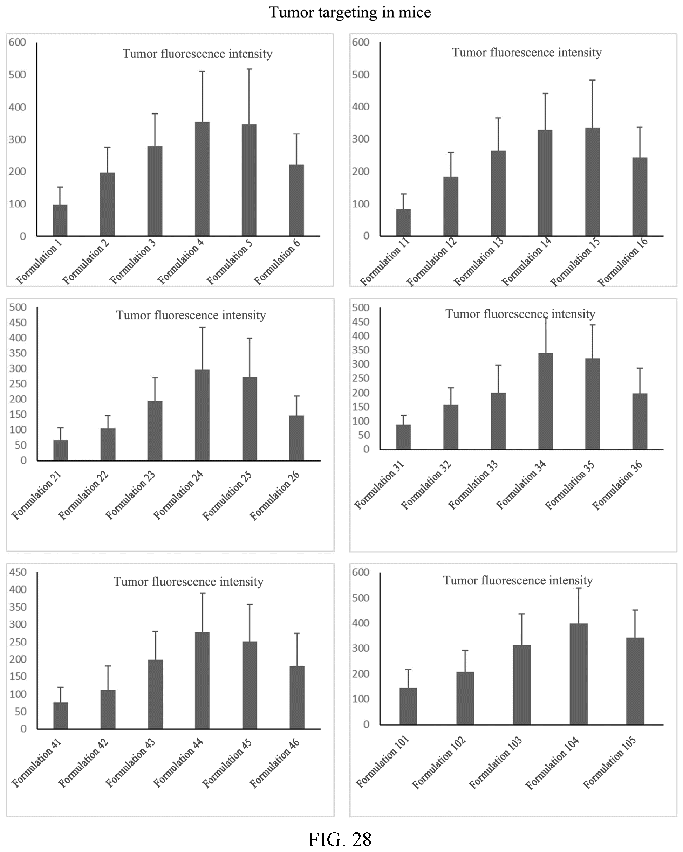

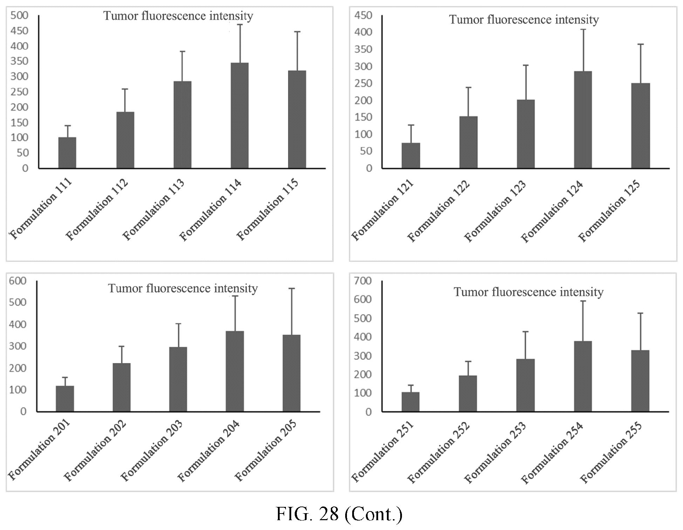

The results are shown in Figure 28. Contrary to common belief, tumor targeting did not change linearly with the change in the molar ratio of cationic lipid to ionizable lipid. When the molar ratio of cationic lipid to ionizable lipid was 1.5:48.5-7.5:42.5, good tumor targeting was achieved.

### (c) In Vivo Anti-tumor Efficacy Evaluation of Formulations Encapsulating Small Nucleic Acids

In this experiment, QTsome lipid nanoparticles were used to encapsulate the small nucleic acid Archexin, with a mass ratio of Archexin to total lipid of 1:10. The anti-tumor effect on the subcutaneous Hepa1-6 model in C57BL/6 mice was evaluated.

### Experimental Preparation Phase:

1) Different formulation preparations were aliquoted in advance and stored at -20°C, reconstituted on the day of use.
2) Hepa1-6 cells were thawed, cultured *in vitro,* and passaged 2-3 times.
3) Seven-week-old BALB/c nude mice (equal male and female) were acclimatized for one week and weighed.

### Experimental Procedure:

1) Hepa1-6 cells were thawed and passaged. When the cells were in good condition, Hepa1-6 cells (inoculation amount 8×10⁶) were transplanted subcutaneously into mice, and changes in mouse body weight and tumor size were recorded (tumor bearing mouse model were constructed by subcutaneous implantation of the cell line on the back);
2) When tumors grew to approximately 100 mm³, mice were grouped based on tumor volume, with 5 mice per group;
3) Each mouse was intravenously administered 100 µl of different formulations encapsulating Archexin at a dose of 7.5 mg/kg for QTsome lipid nanoparticles encapsulating Archexin. The blank control group received an intravenous injection of 100 µL of physiological saline. Injections were given once every 4 days, for a total of 5 administrations, with the first injection on day 0;
4) The growth and proliferation of subcutaneous tumors on the back of tumor-bearing mice in the experimental and blank control groups were observed and recorded daily, including measuring mouse body weight and tumor volume;
5) Mice were sacrificed on day 28 after the first administration, and tumor growth curves were plotted.

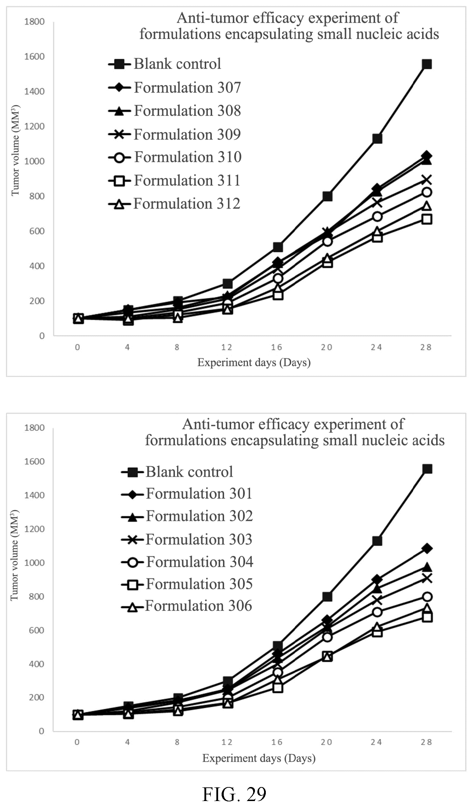

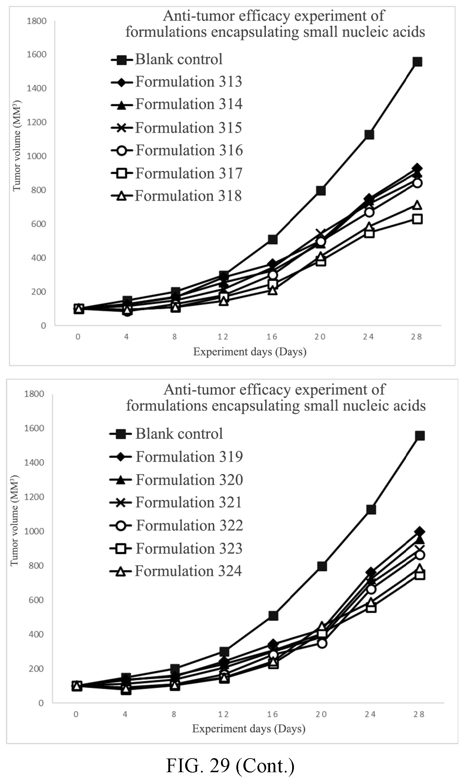

The results are shown in Figure 29. Archexin encapsulated in different formulations showed better tumor inhibition effects compared to the blank control. As the molar ratio of cationic lipid to ionizable lipid gradually increased, the anti-tumor effect also gradually increased. The most significant increase in tumor inhibition effect was observed when the molar ratio of cationic lipid to ionizable lipid was 1.5:48.5-7.5:42.5.

### (d) In Vivo Anti-tumor Efficacy Evaluation of Formulations Encapsulating the Small Molecule Chemotherapy Drug Doxorubicin

In this experiment, QTsome lipid nanoparticles were used to encapsulate the small molecule chemotherapy drug doxorubicin, with a mass ratio of doxorubicin to total lipid of 1:10. The anti-tumor effect on the subcutaneous A549 model in BALB/c nude mice was evaluated.

### Experimental Preparation Phase:

1) Different formulation preparations were aliquoted in advance and stored at -20°C, reconstituted on the day of use.
2) A549 cells were thawed, cultured *in vitro,* and passaged 2-3 times.
3) Seven-week-old BALB/c nude mice (equal male and female) were acclimatized for one week and weighed.

### Experimental Procedure:

1) A549 cells were thawed and passaged. When the cells were in good condition, A549 cells (inoculation amount 8×10⁶) were transplanted subcutaneously into mice, and changes in mouse body weight and tumor size were recorded (tumor bearing mouse model were constructed by subcutaneous implantation of the cell line on the back);
2) When tumors grew to approximately 100 mm³, mice were grouped based on tumor volume, with 5 mice per group;
3) Each mouse was intravenously administered 100 µl of different formulations encapsulating doxorubicin at a dose of 3 mg/kg for QTsome lipid nanoparticles encapsulating doxorubicin. The blank control group received an intravenous injection of 100 µL of physiological saline, and the positive control group received an intravenous injection of doxorubicin injection at a dose of 3 mg/kg. All injections were given once every 4 days, for a total of 5 administrations, with the first injection on day 0;
4) The growth and proliferation of subcutaneous tumors on the back of tumor-bearing mice in the experimental and control groups were observed and recorded daily, and tumor volume was measured;
5) Mice were sacrificed on day 28 after the first administration, and tumor growth curves were plotted.

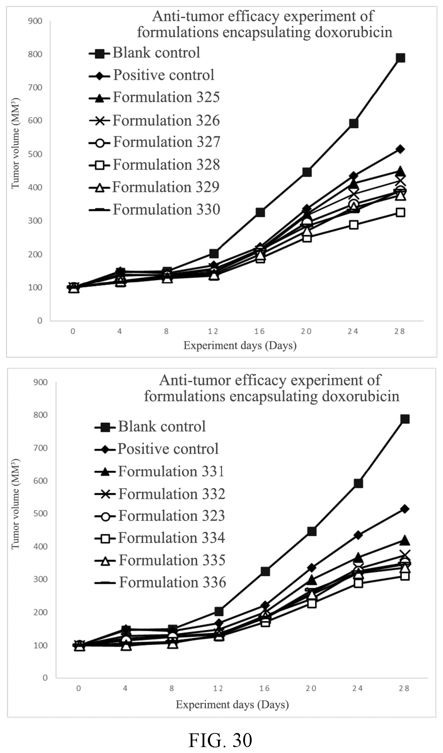

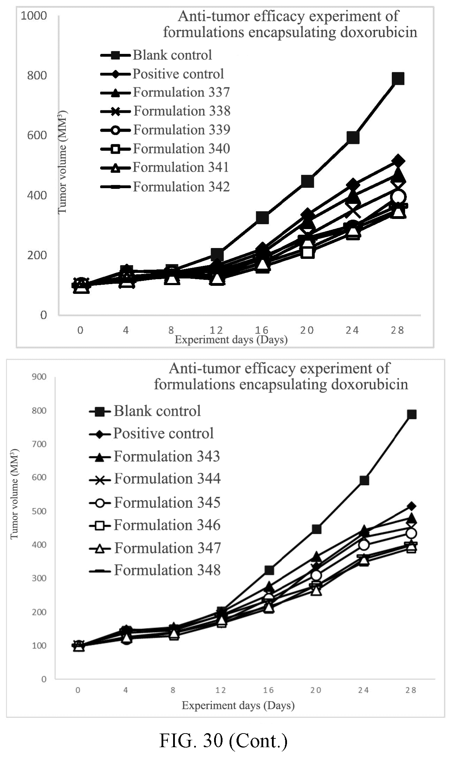

The results are shown in Figure 30. As the molar ratio of cationic lipid to ionizable lipid gradually increased, the anti-tumor effect also gradually increased. The most significant increase in tumor inhibition effect was observed when the molar ratio of cationic lipid to ionizable lipid was 1.5:48.5-7.5:42.5.

### Example 49. Preparation of TP53 mRNA-Loaded LNP Suspension and Control

### (a) TP53 mRNA-Loaded LNP Suspension

A quaternary amine-tertiary amine lipid nanoparticle system based on DOTAP and SM-102, with lipid component molar percentages as shown for Formulation 303 in Table 32. The encapsulated nucleic acid is TP53 mRNA, provided by Zhejiang Haichang Biological Nucleic Acid Innovation Research Institute. The mass ratio of mRNA to total lipid is 1:20.

Preparation method is as follows:
1. A TP53 mRNA sodium acetate solution (pH=5.0, sodium acetate concentration 25 mM) with a concentration of 0.3334 mg/mL (calculated as mRNA) was prepared using sodium acetate buffer (pH=5.0, sodium acetate concentration 50 mM) and the TP53 mRNA solution;
2. A lipid ethanol solution with a total lipid concentration of 20 mg/mL was prepared by adding lipids to anhydrous ethanol according to the lipid ratio of Formulation 303 in Table 32;
3. 0.75 mL of the TP53 mRNA sodium acetate solution (mRNA concentration 0.3334 mg/mL, total amount 0.25 mg) was pre-warmed in a 25°C water bath;
4. 0.25 mL of the lipid solution (total lipid concentration 20 mg/mL, total amount 5 mg) was pre-warmed in a 25°C water bath;
5. Under magnetic stirring in a 25°C water bath, the 0.25 mL lipid solution was injected into the TP53 mRNA sodium acetate solution, incubated for 1 min, then diluted to four times the volume with sodium acetate buffer (pH=5.0, concentration 25 mM) to obtain the initial TP53 mRNA-loaded LNP solution;
6. If a larger quantity is needed, steps 1-5 can be repeated and the initial TP53 mRNA-loaded LNP solutions merged;
7. Ethanol was removed by centrifugal ultrafiltration using an ultrafiltration tube with a molecular weight cutoff of 100 KD at 4°C with a centrifugal acceleration of 2000 g. The nucleic acid concentration was adjusted to an appropriate level using 25 mM Tris buffer, and the pH was adjusted to 7.0 with 0.1 M sodium hydroxide solution to obtain the TP53 mRNA-loaded LNP suspension.

50 µL of the TP53 mRNA-loaded LNP suspension was added to 950 µL of pure water, then the particle size and PDI were measured on a Malvern ZETASIZER PRO laser particle size analyzer. The particle size is 111 nm, and the PDI is 0.1236. The prepared TP53 mRNA-loaded LNP suspension can be used for subsequent *in vivo* anti-tumor evaluation in Example 50.

### (b) Moderna Formulation Control Group

Using SM102 as the ionizable lipid, a Moderna formulation control group encapsulating TP53 mRNA was prepared according to the Moderna lipid formulation, where TP53 mRNA was provided by Zhejiang Haichang Biological Nucleic Acid Innovation Research Institute. The mass ratio of mRNA to total lipid is 1:20. The molar percentages of lipid components in the Moderna formulation are SM-102:DSPC:cholesterol:DMG-PEG2K = 50:10:38.5:1.5.

Preparation method is as follows:
1. A TP53 mRNA sodium acetate solution (pH=5.0, sodium acetate concentration 25 mM) with a concentration of 0.3334 mg/mL (calculated as mRNA) was prepared using sodium acetate buffer (pH=5.0, sodium acetate concentration 50 mM) and the TP53 mRNA solution;
2. A lipid ethanol solution with a total lipid concentration of 20 mg/mL was prepared by adding lipids to anhydrous ethanol according to the lipid component molar percentages SM-102:DSPC:cholesterol:DMG-PEG2K = 50:10:38.5:1.5;
3. 0.75 mL of the TP53 mRNA sodium acetate solution (mRNA concentration 0.3334 mg/mL, total amount 0.25 mg) was pre-warmed in a 25°C water bath;
4. 0.25 mL of the lipid solution (total lipid concentration 20 mg/mL, total amount 5 mg) was pre-warmed in a 25°C water bath;
5. Under magnetic stirring in a 25°C water bath, the 0.25 mL lipid solution was injected into the TP53 mRNA sodium acetate solution, incubated for 1 min, then diluted to four times the volume with sodium acetate buffer (pH=5.0, concentration 25 mM) to obtain the initial TP53 mRNA-loaded LNP solution;
6. If a larger quantity is needed, steps 1-5 can be repeated and the initial TP53 mRNA-loaded LNP solutions merged;
7. Ethanol was removed by centrifugal ultrafiltration using an ultrafiltration tube with a molecular weight cutoff of 100 KD at 4°C with a centrifugal acceleration of 2000 g. The nucleic acid concentration was adjusted to an appropriate level using 25 mM Tris buffer, and the pH was adjusted to 7.0 with 0.1 M sodium hydroxide solution to obtain the TP53 mRNA-loaded LNP suspension, i.e., the Moderna formulation control.

50 µL of the TP53 mRNA-loaded LNP suspension was added to 950 µL of pure water, then the particle size and PDI were measured on a Malvern ZETASIZER PRO laser particle size analyzer. The particle size is 123 nm, and the PDI is 0.1302. The prepared Moderna formulation control can be used for subsequent *in vivo* anti-tumor evaluation in Example 50.

### Example 50. In Vivo Anti-tumor Evaluation of TP53 mRNA-Loaded LNP Suspension

In this experiment, the TP53 mRNA-loaded LNP suspension prepared in Example 49 (Formulation 303 experimental group), the Moderna formulation control (Moderna formulation group), and the PBS negative control (PBS negative control group) were used to evaluate the anti-tumor effect on the subcutaneous TC1 cell model expressing tp53 mutation in C57/B6 mice.

### Experimental Preparation Phase:

1) Preparations were aliquoted in advance and stored at -20°C, reconstituted on the day of use.
2) TC1 cells expressing the tp53 mutation were thawed, cultured *in vitro,* and passaged 2-3 times.
3) Seven-week-old C57/B6 mice (equal male and female) were acclimatized for one week and weighed.

### Experimental Procedure:

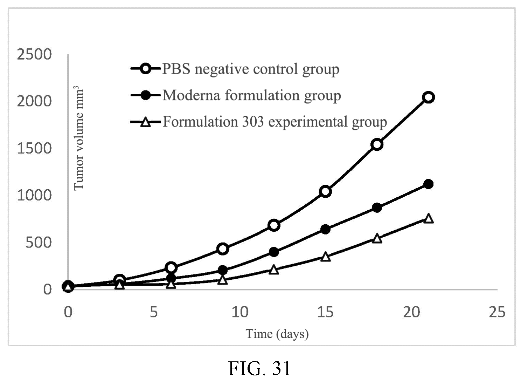

1) TC1 cells expressing the tp53 mutation were thawed and passaged. When the cells were in good condition, TC1 cells expressing the tp53 mutation (inoculation amount 8×10⁶) were transplanted subcutaneously into mice, and changes in mouse body weight and tumor size were recorded (tumor bearing mouse model were constructed by subcutaneous implantation of the cell line on the back);
2) When tumors grew to approximately 30 mm³, mice were grouped based on tumor volume into the Moderna formulation group, the Formulation 303 experimental group, and the PBS negative control group, with 4 mice per group;
3) Each mouse was intravenously administered 100 µl, once a week, for a total of three administrations;
4) The growth and proliferation of subcutaneous tumors on the back of tumor-bearing mice in each group were observed and recorded every 3 days, including measuring mouse body weight and tumor volume;
5) Mice were sacrificed on day 21 after the first administration, and tumor growth curves were plotted, as shown in Figure 31.

The experimental results show that the tumor growth inhibition rate (TGI) for the Formulation 303 experimental group was 62.8%, while the TGI for the Moderna formulation group was 45.1%, with a significant difference between the two (p < 0.05).

### Example 51. Determination of pKa of LNP Formulations with Different Cationic Lipid Ratios Using the TNS Method

The following different pH buffers were prepared: 1) citrate-sodium citrate buffer was prepared (25 mM * 500 mL, containing 100 mM sodium chloride), pH = 4.0, 4.5, 5.0, 5.5, 6.0, 6.5; 2) Hepes buffer was prepared (25 mM * 500 mL, containing 100 mM sodium chloride), pH = 7.0, 7.5; 3) Tris-hydrochloric acid buffer was prepared (25 mM * 500 mL, containing 100 mM sodium chloride), pH = 8.0, 8.5, 9.0; 4) Sodium carbonate-sodium hydroxide buffer was prepared (25 mM * 500 mL, containing 100 mM sodium chloride), pH = 10.0, 11.0; 5) Potassium chloride-sodium hydroxide buffer was prepared (25 mM * 500 mL), pH = 12.0, 13.0.

A dimethyl sulfoxide (DMSO) solution containing 2-(p-toluidino)-6-naphthalenesulfonic acid (TNS) was prepared: 3.134 mg of TNS (Mw:313.37) was dissolved by adding 2 mL DMSO, to obtain a 5 mM TNS DMSO solution. Lipid nanoparticle suspensions (containing the small nucleic acid Archexin) were prepared according to the lipid ratios of different formulations, with a total lipid concentration of 8 mg/mL. The samples for measurement were prepared according to Table 41.

**Table 41. Components and Volumes for Measurement Samples**

| **Buffer Volume** | **Lipid Nanoparticle Suspension Volume** | **TNS DMSO Solution Volume** |
|---|---|---|
| 5mL | 37.5 µL | 6 µL |

37.5 µL of the above lipid nanoparticle suspension (if the total lipid concentration was not 8 mg/mL, adjust the added volume proportionally) was added to 5 mL of the prepared buffer, followed by mixing gently; then 6 µL of the above TNS DMSO solution was added, followed by mixing gently; incubation was performed at room temperature in the dark for 1 hour; the mixture was added to a 96-well plate, 0.2 mL per well, and fluorescence intensity (λex = 322 nm, λem = 431 nm) was measured using a SPARK tecan microplate reader. Fluorescence intensity was plotted against pH value and the pKa value of the lipid nanoparticle suspension was calculated. The obtained pKa values are displayed in Table 42 below.

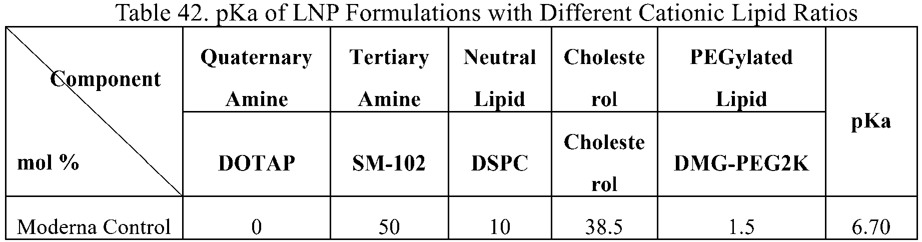

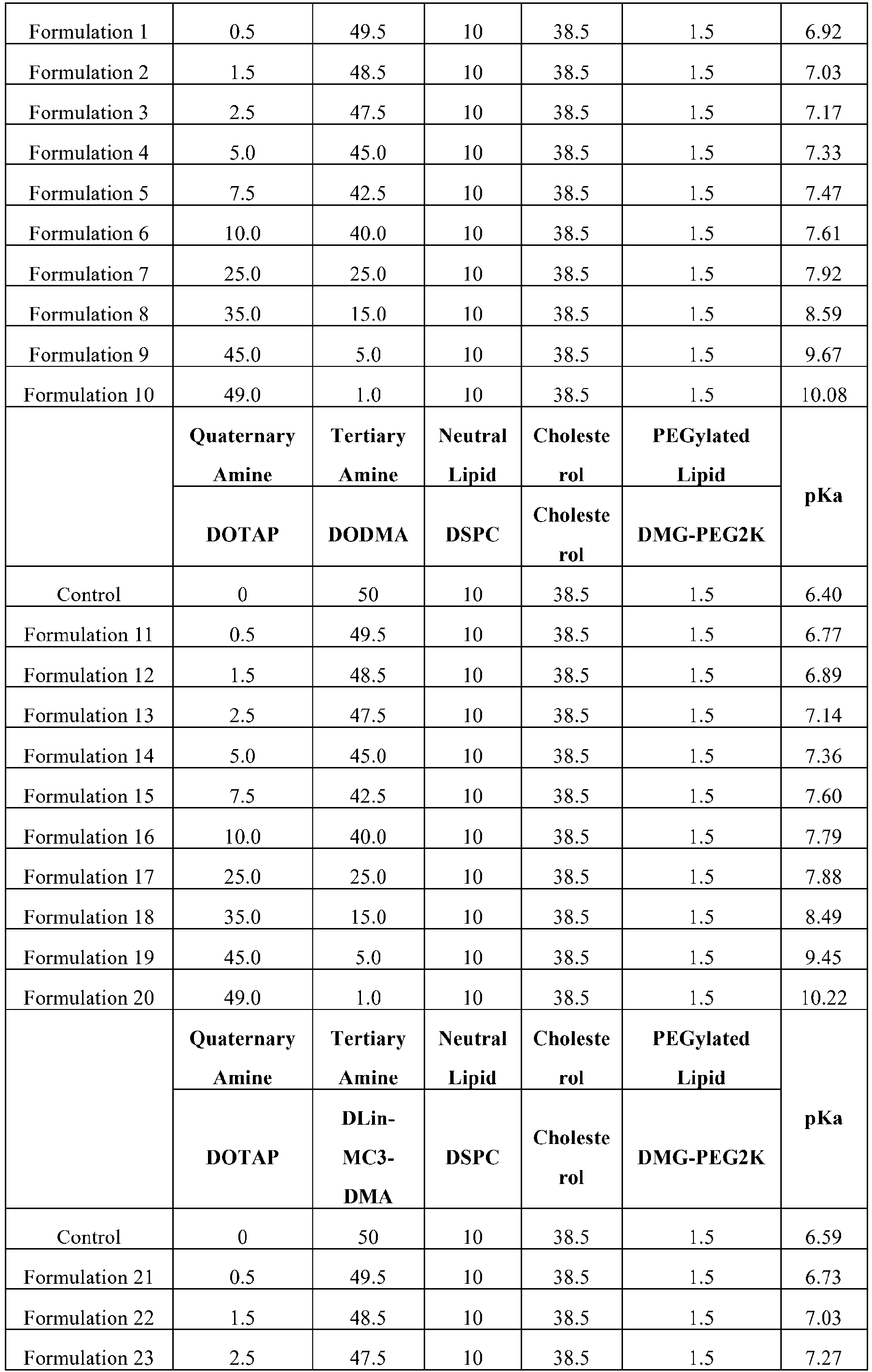

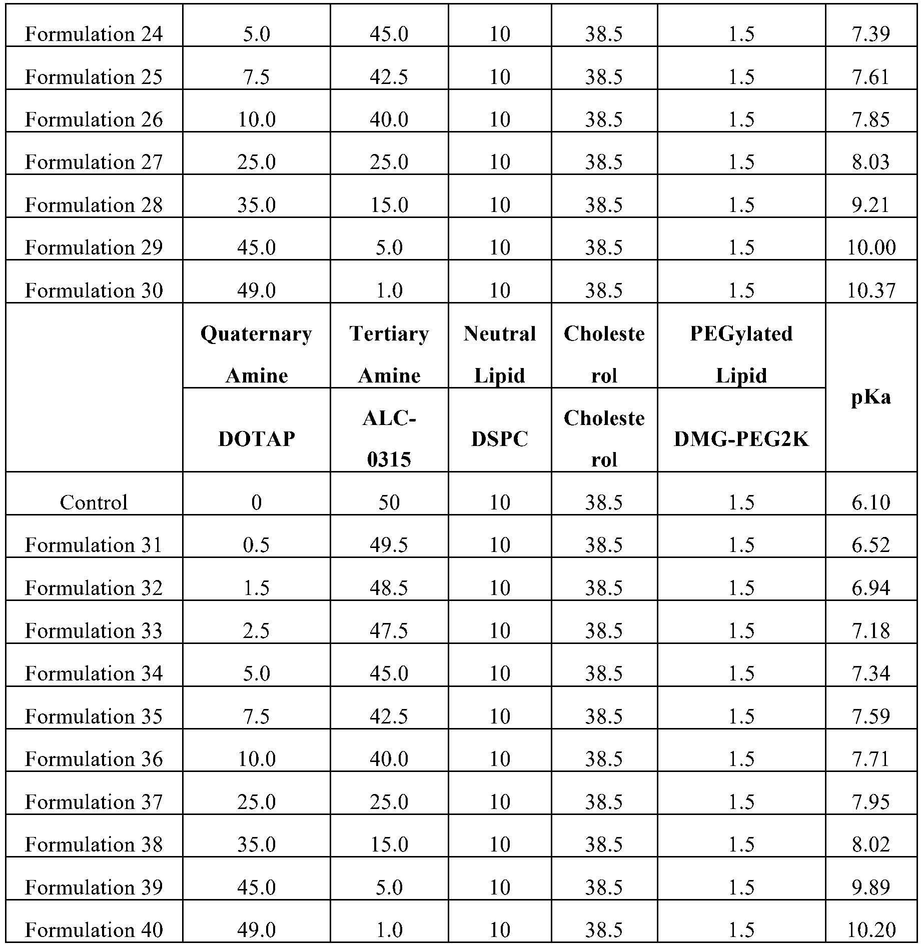

The above describes only some embodiments of the present disclosure. For those of ordinary skill in the art, various modifications and improvements can be made without departing from the creative concept of the present disclosure, and these all fall within the scope of protection of the present disclosure.

## Claims

1. A lipid composition, comprising an active component, a lipid carrier, and a targeting component, wherein the lipid carrier comprises a positively charged component and a phospholipid, and the molar percentage of the positively charged component in the lipid carrier is 0.5%-80%.

2. The lipid composition according to claim 1, wherein the targeting component is one or more selected from the group consisting of transferrin (Trf), lactoferrin, albumin, fibronectin, vascular endothelial growth factor, epidermal growth factor, insulin-like growth factor, antibodies, polypeptides, polysaccharides, and glycolipids; preferably, the targeting component is one or more selected from the group consisting of transferrin, serum albumin, and anti-CD5 antibodies.

3. The lipid composition according to claim 1, wherein the active component is one or more selected from the group consisting of nucleic acid drugs, small molecule drugs, polypeptides, proteins, antibodies, chemical drugs, chemotherapeutic drugs, fluorescent labels, and radioactive diagnostic agents; preferably, the active component is at least one selected from of the group consisting of GFP-mRNA, Archexin, doxorubicin, CD19 CAR-mRNA, DIR, and TP53 mRNA.

4. The lipid composition according to claim 1, wherein the lipid carrier has a pKa value measured by TNS method greater than 6.8, preferably 6.8-11, more preferably 6.8-10; preferably, the positively charged component is one or more selected from the group consisting of quaternary ammonium and derivatives thereof, tertiary amine and derivatives thereof, guanidine-containing compounds, polyethyleneimine and derivatives thereof, alkanolamine and derivatives thereof, meglumine and derivatives thereof, lysine and derivatives thereof, histidine and derivatives thereof, arginine and derivatives thereof, protamine and derivatives thereof; more preferably, the positively charged component is DOTAP.

5. The lipid composition according to claim 1 or 4, wherein the phospholipid is one or more selected from the group consisting of ionizable lipids, neutral phospholipids, and PEGylated lipids; preferably, the phospholipid is one or more selected from the group consisting of SM-102, distearoylphosphatidylcholine, egg phosphatidylcholine, soybean lecithin, hydrogenated soybean lecithin, dioleoylphosphatidylethanolamine, dilauroylphosphatidylcholine, dimyristoylphosphatidylcholine, dipalmitoylphosphatidylcholine, 1,2-dioleoylphosphatidylcholine, diarachidoylphosphatidylcholine, dimyristoylphosphatidylethanolamine, dilauroylphosphatidylethanolamine, distearoylphosphatidylethanolamine, dipalmitoylphosphatidylethanolamine, 1-palmitoyl-2-oleoylphosphatidylethanolamine, and DMG-PEG-2000.

6. The lipid composition according to any one of claims 1, 4 and 5, wherein the molar percentage of the phospholipid in the lipid carrier is 8.5%-85%.

7. The lipid composition according to any one of claims 1, 4 and 5, wherein the lipid carrier further comprises a non-phospholipid neutral lipid, preferably cholesterol.

8. The lipid composition according to claim 7, wherein the molar percentage of the non-phospholipid neutral lipid in the lipid carrier is 20%-75%.

9. The lipid composition according to claim 7, wherein the lipid carrier comprises a positively charged component, an ionizable lipid, a neutral phospholipid, a PEGylated lipid, and a non-phospholipid neutral lipid; preferably, the molar ratio of the positively charged component to the ionizable lipid is 1:0.1 to 1:30; more preferably, the molar percentage of the positively charged component in the lipid carrier is 0.5%-80%, the molar percentage of the ionizable lipid in the lipid carrier is 2.5%-70%, the molar percentage of the neutral phospholipid in the lipid carrier is 5%-15%, the molar percentage of the PEGylated lipid in the lipid carrier is 0.5%-5%, and the molar percentage of the non-phospholipid neutral lipid in the lipid carrier is 20%-75%.

10. The lipid composition according to claim 9, wherein the positively charged component comprises DOTAP, the ionizable lipid comprises SM-102, the neutral phospholipid comprises DSPC, the non-phospholipid neutral lipid comprises cholesterol, and the PEGylated lipid comprises DMG-PEG2000.

11. The lipid composition according to claim 10, wherein the lipid carrier comprises any one of the following (a)-(f):
(a) DOTAP at a molar percentage of 2.5%, SM-102 at 47.5%, DSPC at 10%, cholesterol at 38.5%, and DMG-PEG2000 at 1.5% in the lipid carrier;
(b) DOTAP at a molar percentage of 5%, SM-102 at 45%, DSPC at 10%, cholesterol at 38.5%, and DMG-PEG2000 at 1.5% in the lipid carrier;
(c) DOTAP at a molar percentage of 7.5%, SM-102 at 42.5%, DSPC at 10%, cholesterol at 38.5%, and DMG-PEG2000 at 1.5% in the lipid carrier;
(d) DOTAP at a molar percentage of 10%, SM-102 at 40%, DSPC at 10%, cholesterol at 38.5%, and DMG-PEG2000 at 1.5% in the lipid carrier;
(e) DOTAP at a molar percentage of 25%, SM-102 at 25%, DSPC at 10%, cholesterol at 38.5%, and DMG-PEG2000 at 1.5% in the lipid carrier;
(f) DOTAP at a molar percentage of 45%, SM-102 at 5%, DSPC at 10%, cholesterol at 38.5%, and DMG-PEG2000 at 1.5% in the lipid carrier.

12. The lipid composition according to claim 1 or 11, comprising any one or more of the following lipid compositions: Archexin-LNPs-Trf, eGFP-mRNA-LNPs-Trf, LNPs-DIR-CD5, eGFP-mRNA-LNPs-CD5, CD19 CAR-mRNA-LNPs-Trf, CD19 CAR-mRNA-LNPs-CD5, Archexin-LNPs-albumin, eGFP-mRNA-LNPs-albumin, and CD19 CAR-mRNA-LNPs-albumin.

13. The lipid composition according to any one of claims 1 or 11, wherein the mass ratio of the lipid carrier to the active component is 1:0.01 to 1:0.5, or the mass ratio of the lipid carrier to the targeting component is 1:0.01 to 1:0.5.

14. A preparation method for the lipid composition according to any one of claims 1-13, or a method for modifying LNPs with targeting moieties on their surface, comprising steps (a)-(e):
(a) preparing a solution I comprising an active component using a buffer and the active component;
(b) preparing a lipid solution using an organic solvent and a lipid carrier;
(c) mixing the lipid solution and the solution I, diluting to obtain an initial lipid nanoparticle (LNP) solution; optionally repeating steps (a)-(c);
(d) removing the organic solvent from the initial lipid nanoparticle solution, diluting and adjusting pH to 6.0-8.0 to obtain an active component-loaded lipid nanoparticle suspension;
(e) preparing a targeting component solution, then mixing the active component-loaded lipid nanoparticle suspension and the targeting component solution; preferably, mixing the active component-loaded lipid nanoparticle suspension and the targeting component solution is performed prior to use.

15. The preparation method according to claim 14, wherein the organic solvent is removed from the initial lipid nanoparticle solution by ultrafiltration and/or dialysis in step (d).

16. A lipid nanoparticle delivery system, comprising an active component, a lipid carrier, and optionally a targeting component, wherein the lipid carrier comprises a cationic lipid, an ionizable lipid, and a neutral phospholipid, and the molar ratio of the cationic lipid to the ionizable lipid is 0.5:49.5 to 49:1, preferably 0.5:49.5 to 10:40, more preferably 1.5:48.5 to 7.5:42.5.

17. The lipid nanoparticle delivery system according to claim 16, wherein the lipid carrier has a pKa value measured by TNS method greater than 6.8, preferably 6.8-11, more preferably 6.8-10, most preferably 6.8-8.

18. The lipid nanoparticle delivery system according to claim 16, wherein the active component is one or more selected from the group consisting of nucleic acid drugs, small molecule drugs, polypeptides, proteins, antibodies, chemical drugs, chemotherapeutic drugs, fluorescent labels, and radioactive diagnostic agents; preferably, the active component is at least one selected from of the group consisting of GFP-mRNA, Archexin, doxorubicin, CD19 CAR-mRNA, DIR, and TP53 mRNA.

19. The lipid nanoparticle delivery system according to claim 16, wherein the cationic lipid is one or more selected from the group consisting of quaternary ammonium and derivatives thereof, guanidine-containing compounds, polyethyleneimine and derivatives thereof, alkanolamine and derivatives thereof, meglumine and derivatives thereof, lysine and derivatives thereof, histidine and derivatives thereof, arginine and derivatives thereof, protamine and derivatives thereof; preferably, the cationic lipid is at least one selected from the group consisting of DOTAP, DOTMA, and DDBA.

20. The lipid nanoparticle delivery system according to claim 16, wherein the ionizable lipid is one or more selected from the group consisting of tertiary amine and derivatives thereof, pyrrolidine and derivatives thereof, piperazine and derivatives thereof, and piperidine and derivatives thereof; preferably, the ionizable lipid is at least one selected from the group consisting of SM-102, DODMA, DLin-MC3-DMA, ALC-0315, A066, DODAP, Acuitas A9, Moderna Lipid 5, C12-200, and Lipoid 5A2-SC8.

21. The lipid nanoparticle delivery system according to claim 16, wherein the neutral phospholipid is one or more selected from the group consisting of phosphatidylcholines and/or phosphatidylethanolamines; preferably, the neutral phospholipid is at least one selected from the **group** consisting of DSPC, DOPC, EPC, HSPC, DOPE, POPE, DPPE, and DSPE.

22. The lipid nanoparticle delivery system according to claim 16, wherein the molar percentage of the neutral phospholipid in the lipid carrier is 5%-15%, preferably 10%.

23. The lipid nanoparticle delivery system according to claim 16 or 22, wherein the lipid carrier further comprises cholesterol and/or a PEGylated lipid; preferably, the molar percentage of cholesterol in the lipid carrier is 20%-75%, more preferably 38.5%; preferably, the molar percentage of the PEGylated lipid in the lipid carrier is 0.5%-5%, more preferably 1.5%.

24. The lipid nanoparticle delivery system according to any one of claims 16, 22, and 23, wherein the lipid carrier comprises any one of the following formulations:

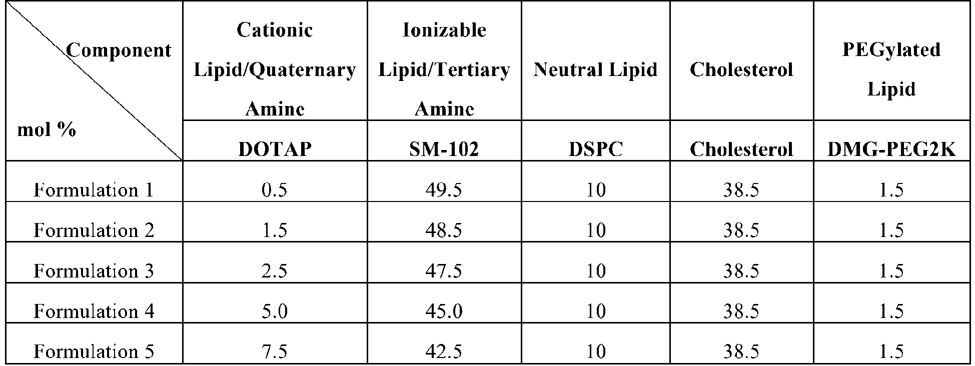

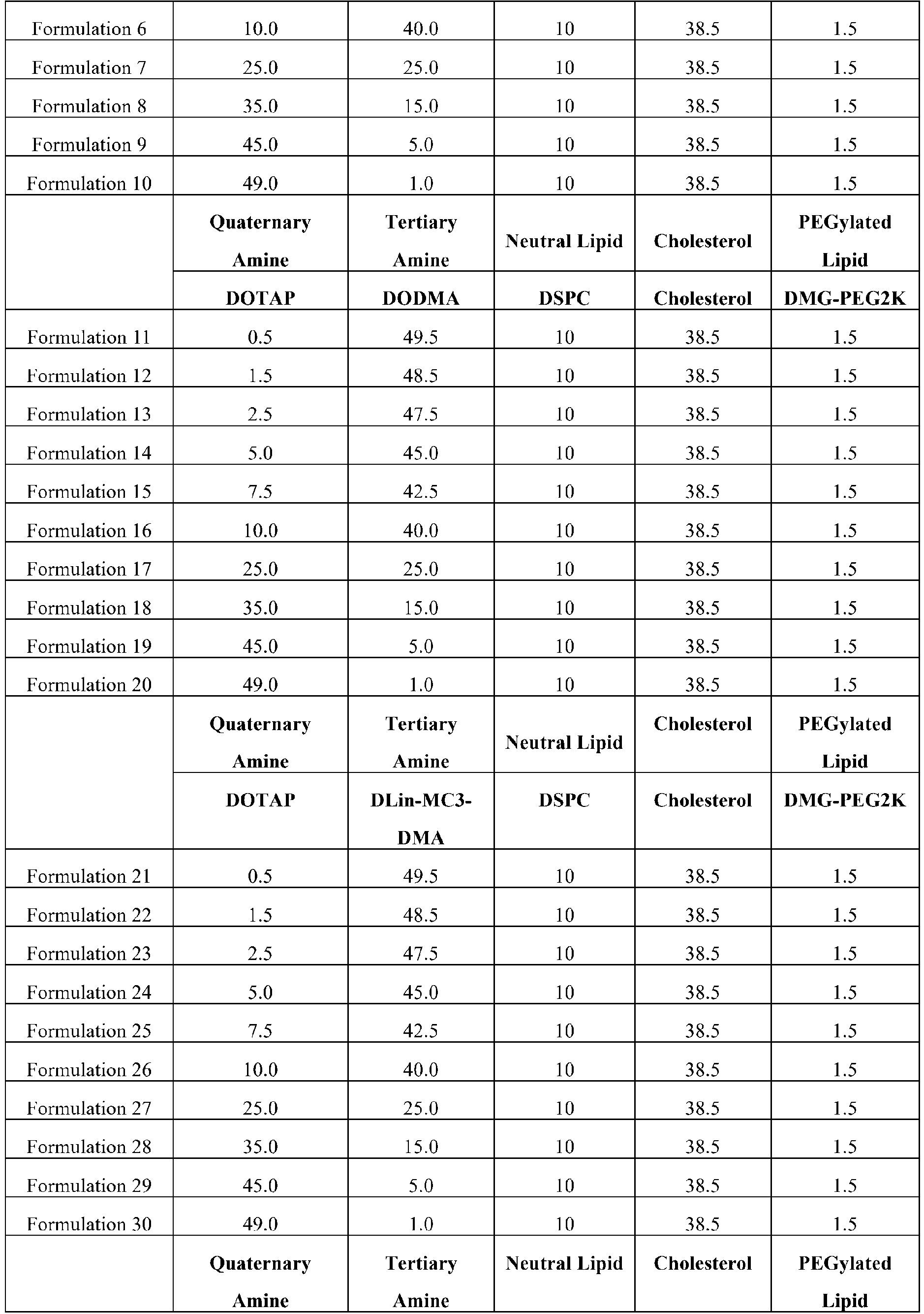

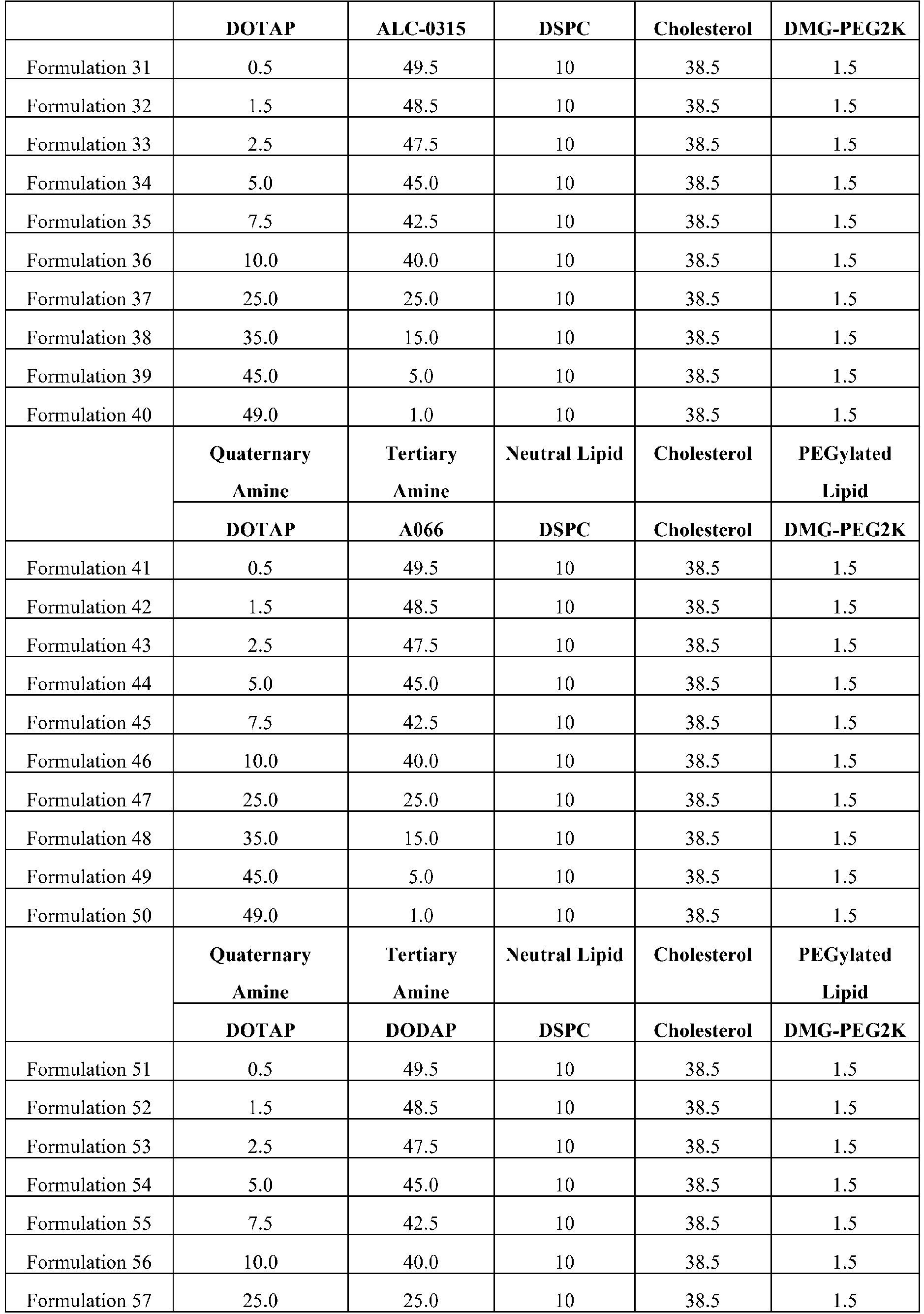

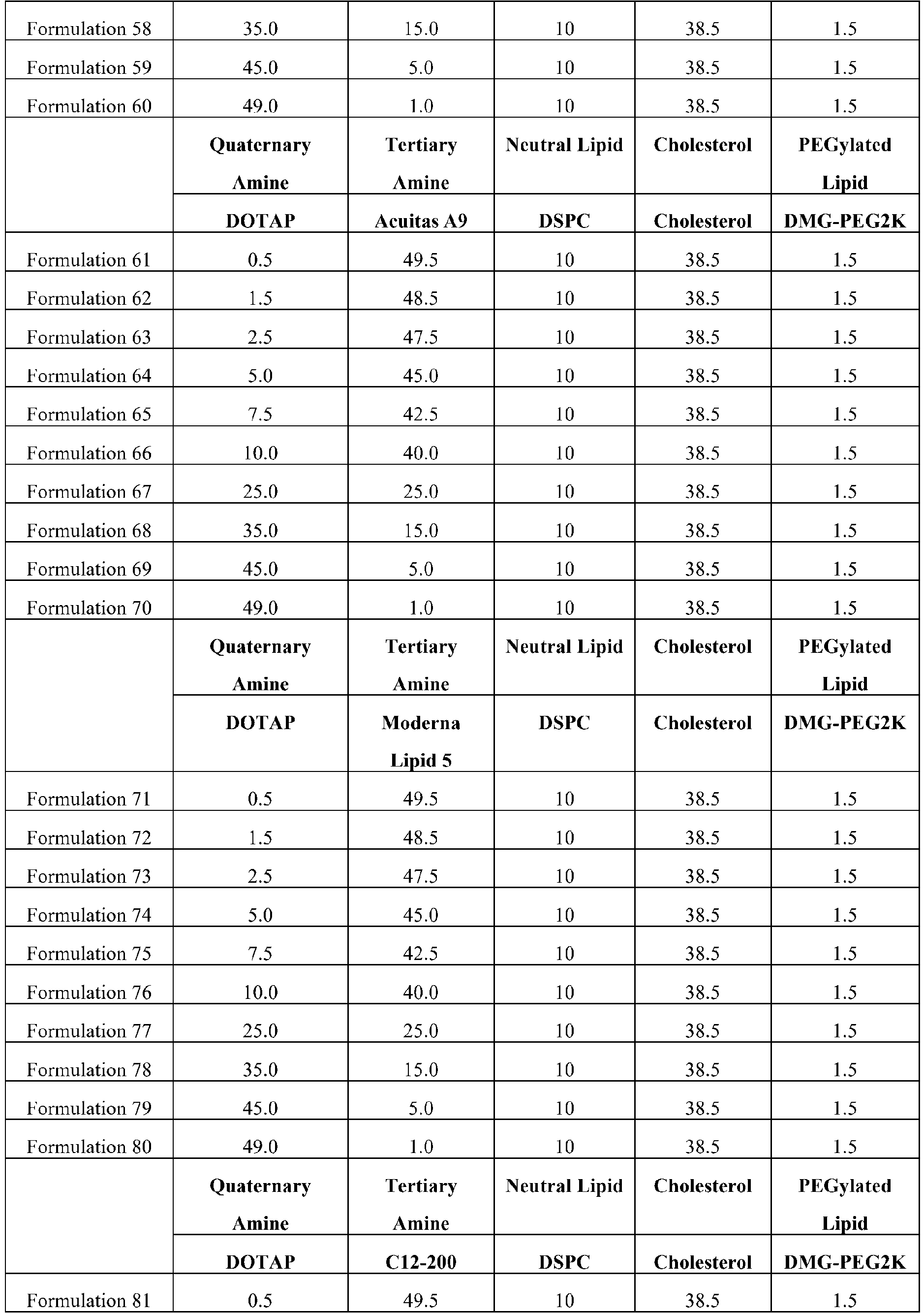

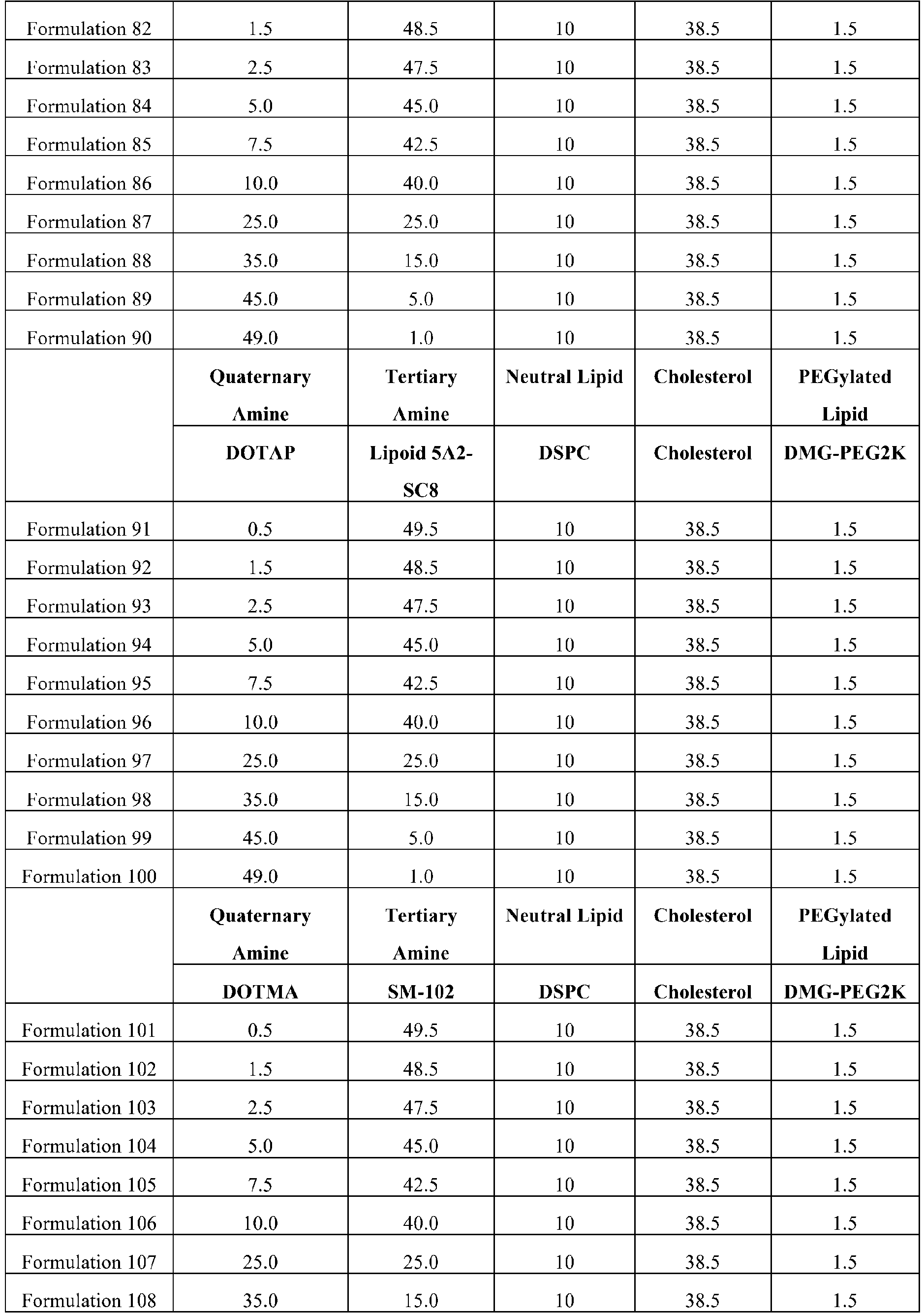

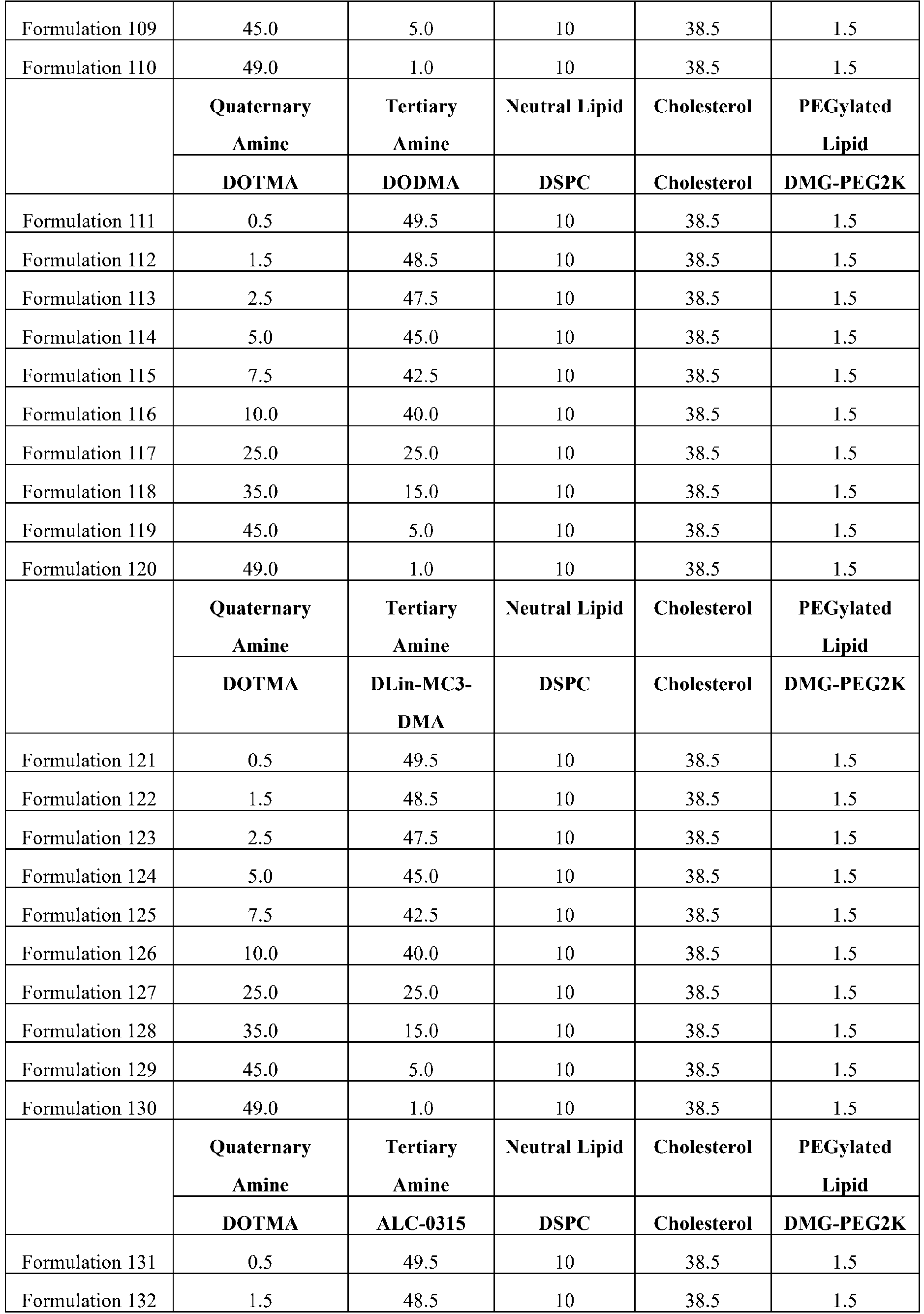

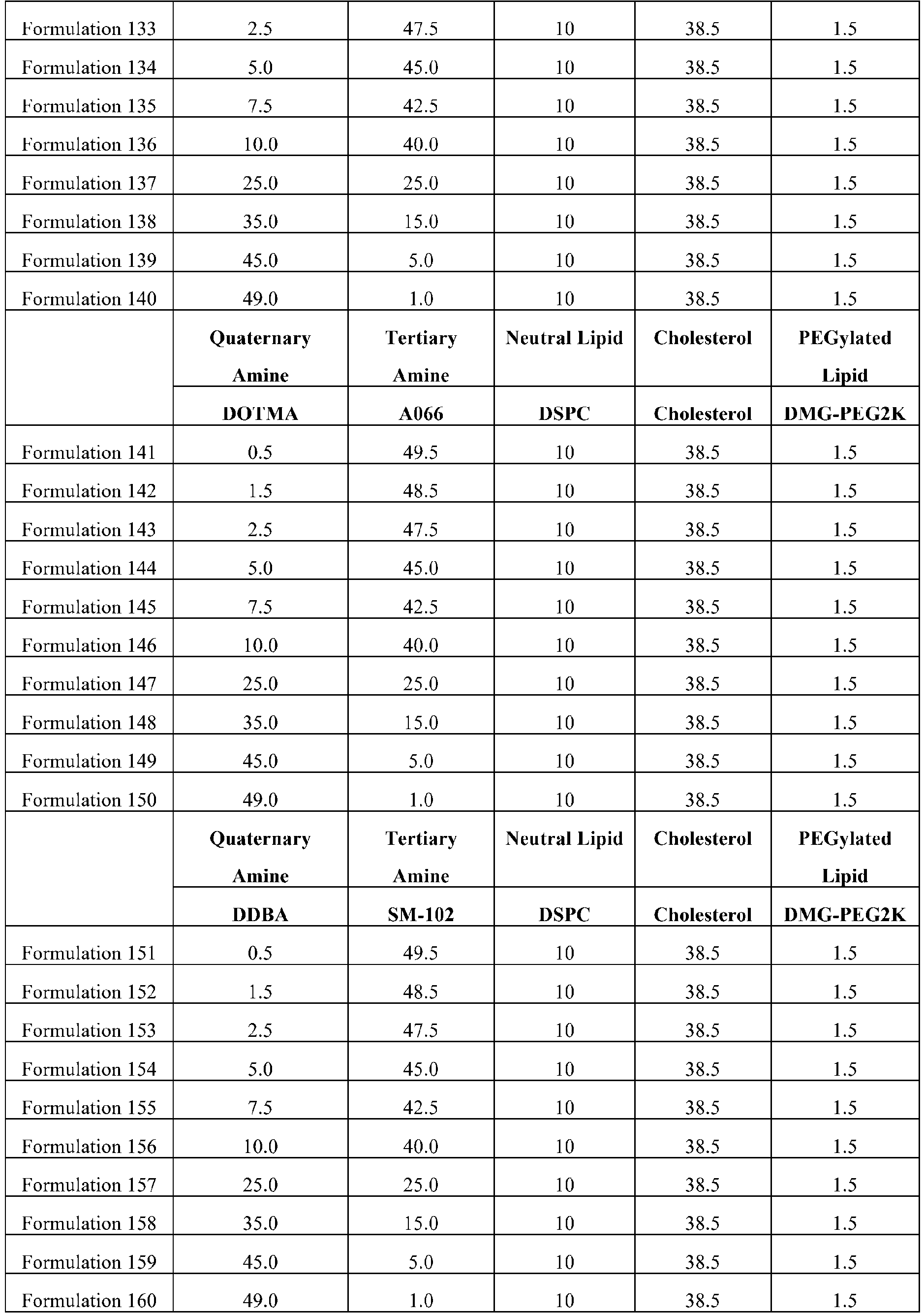

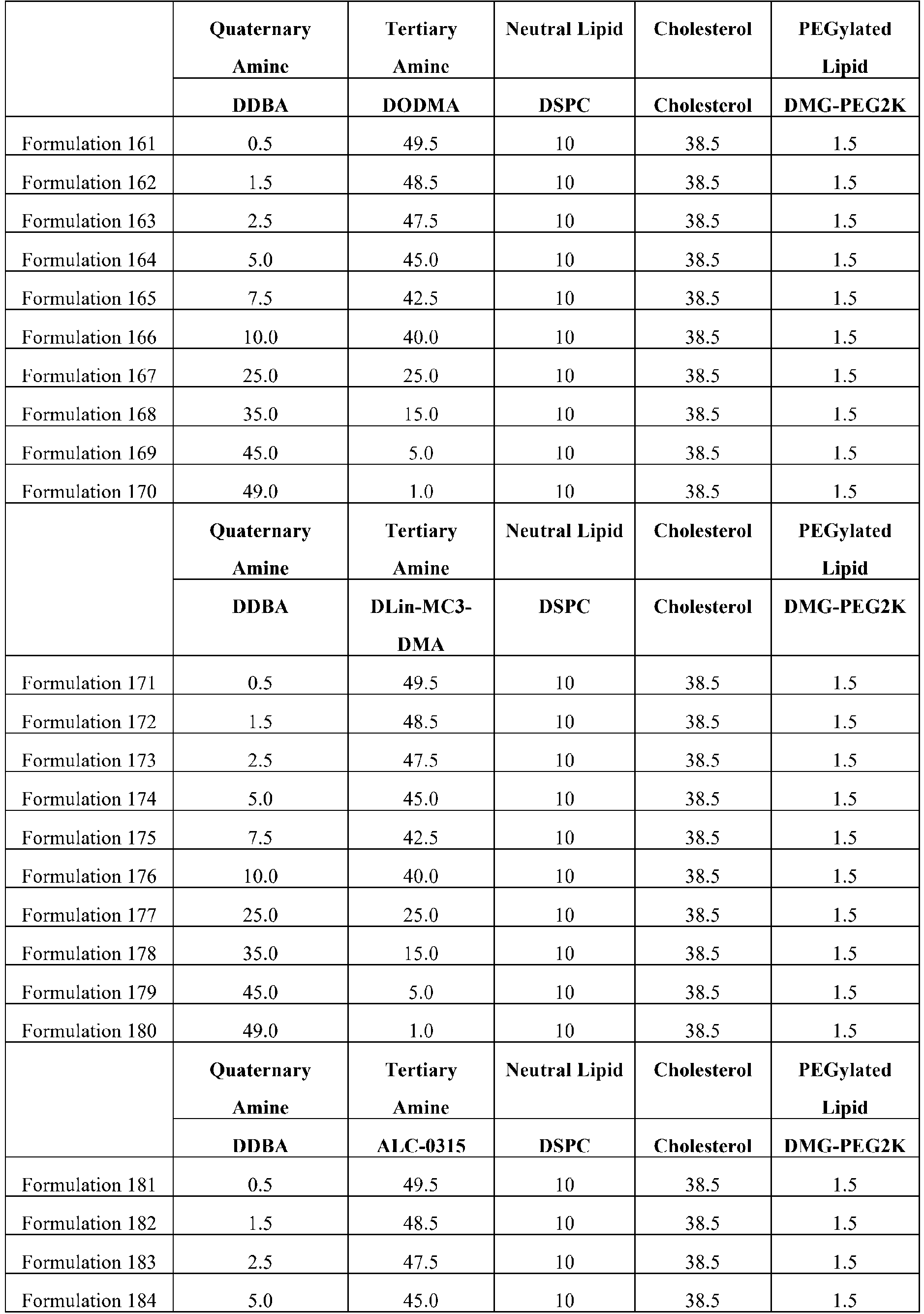

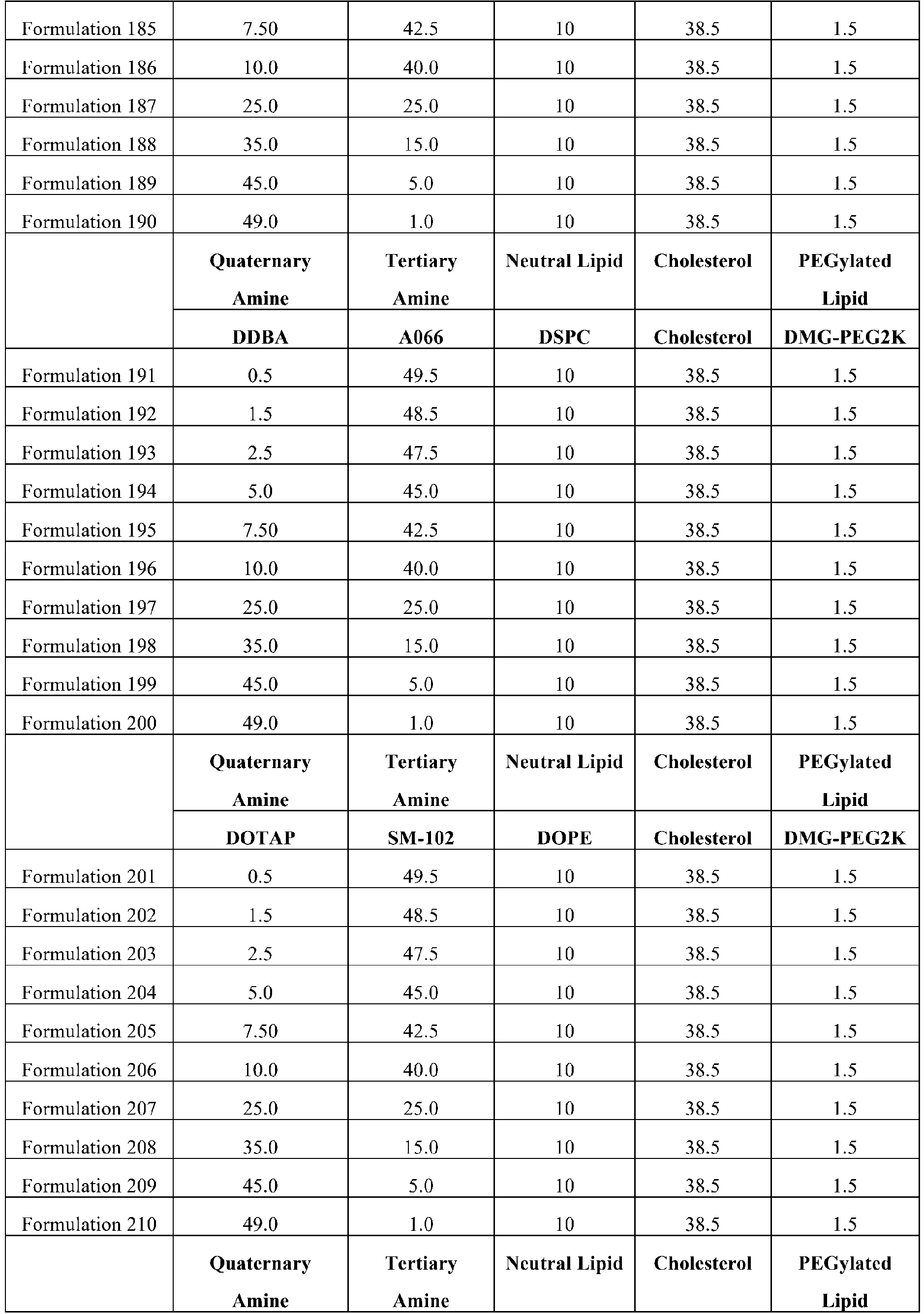

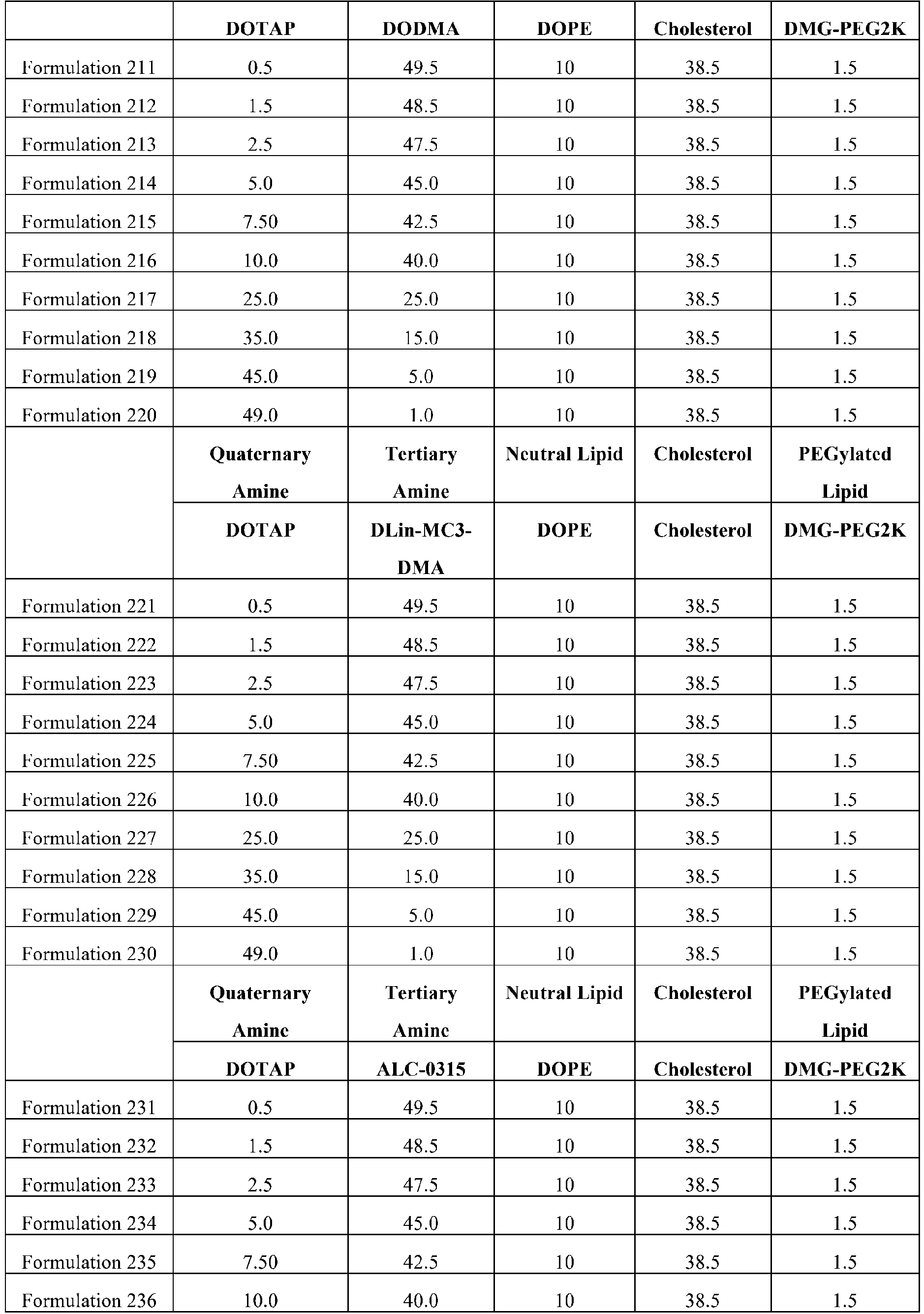

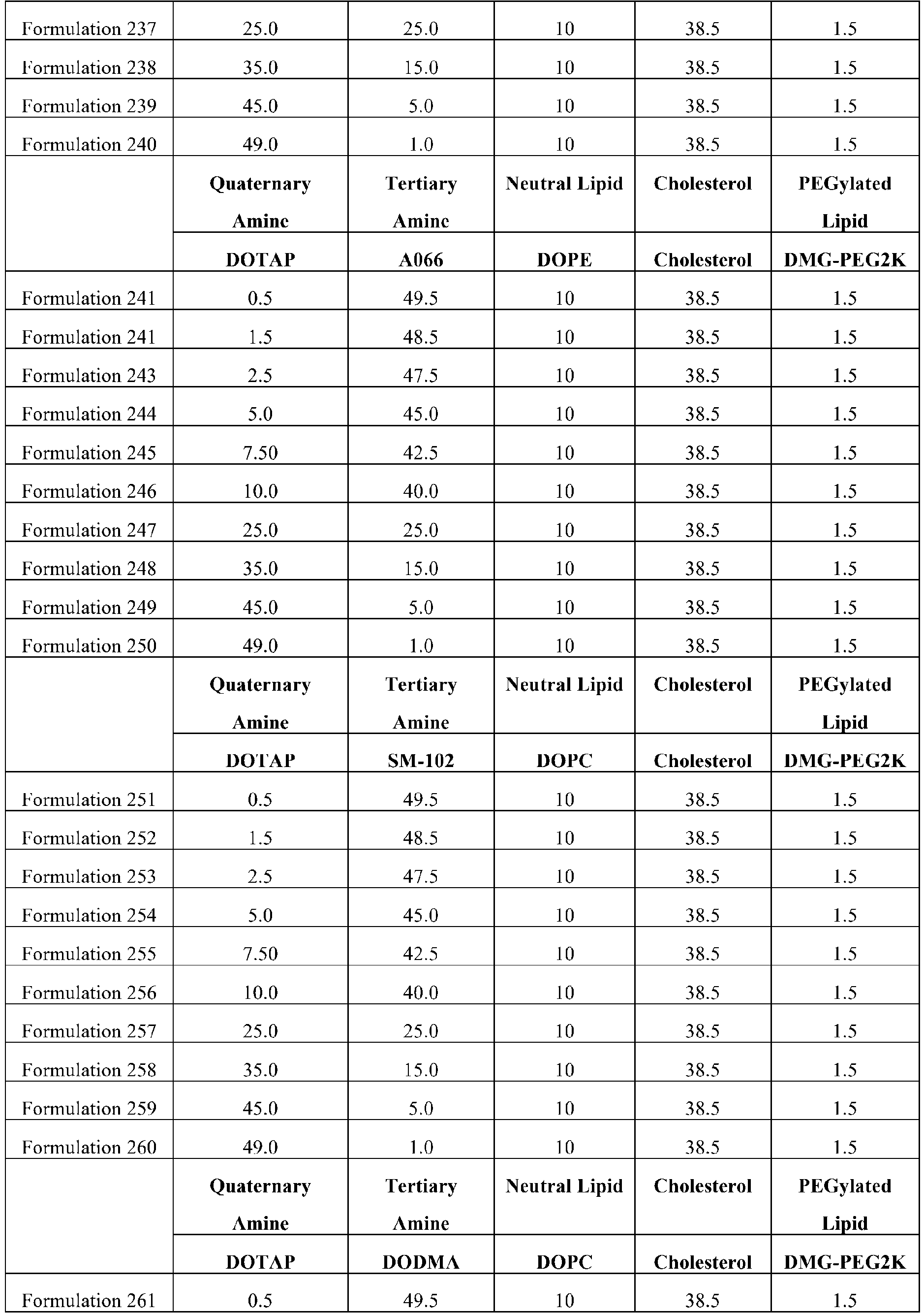

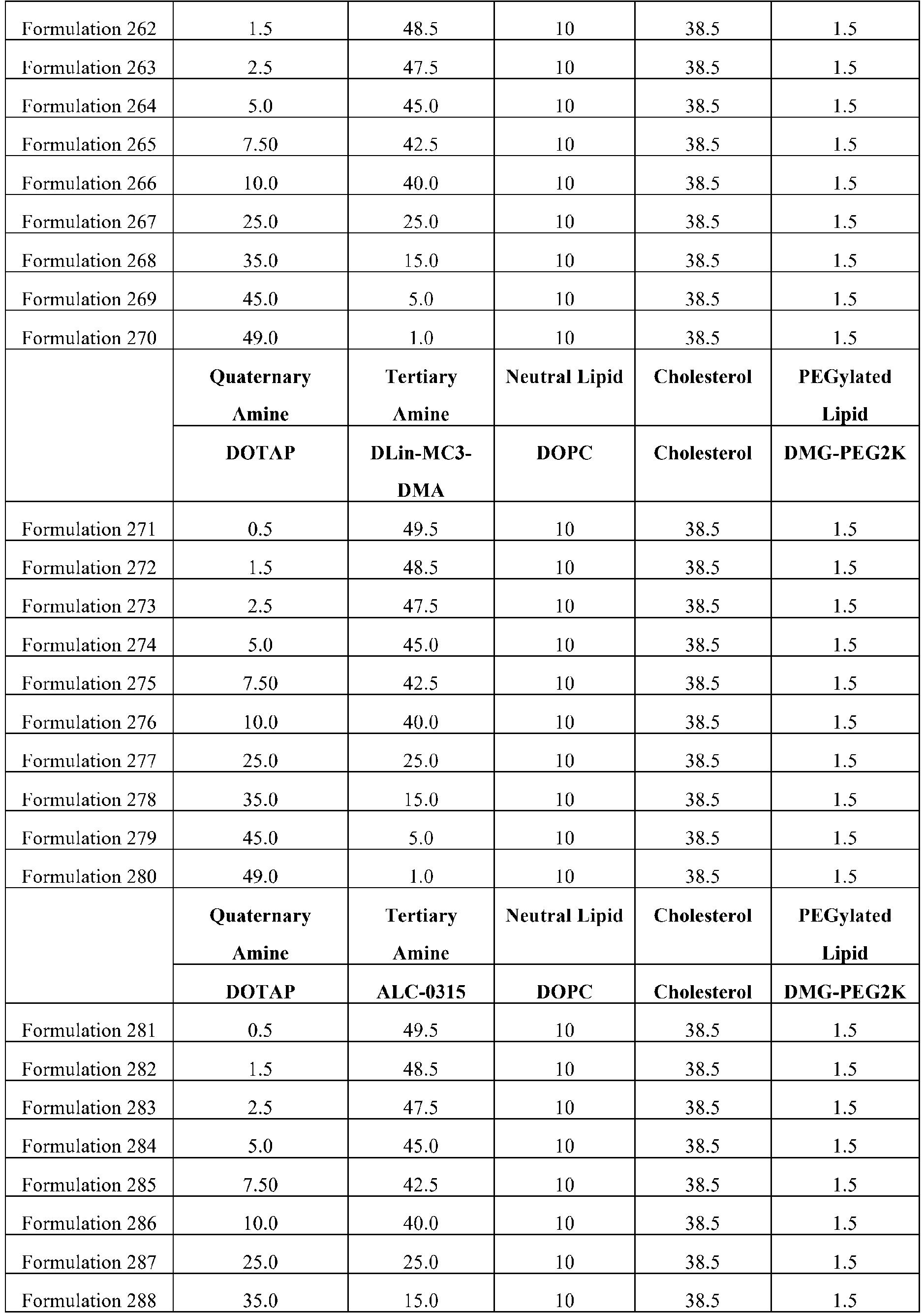

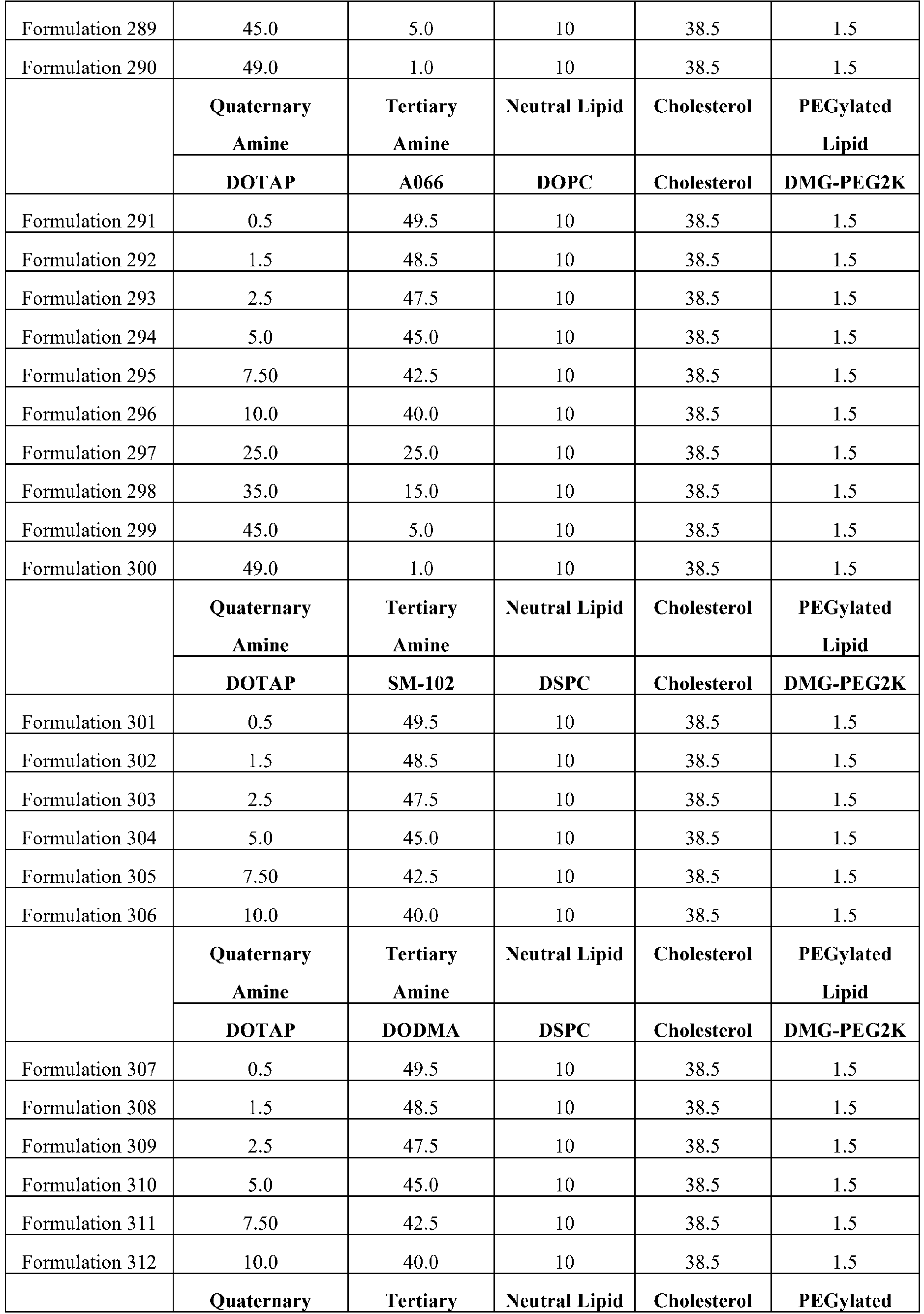

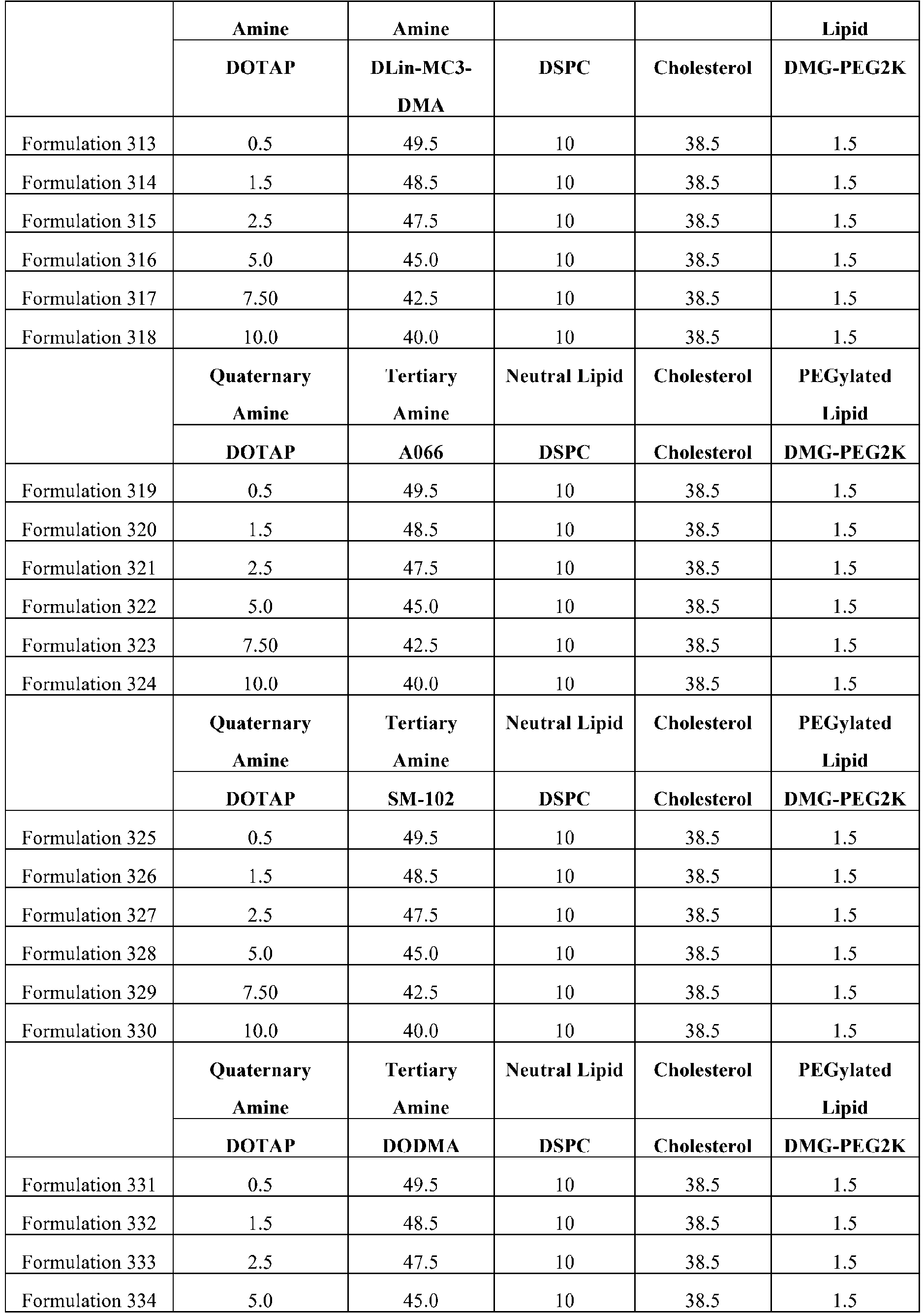

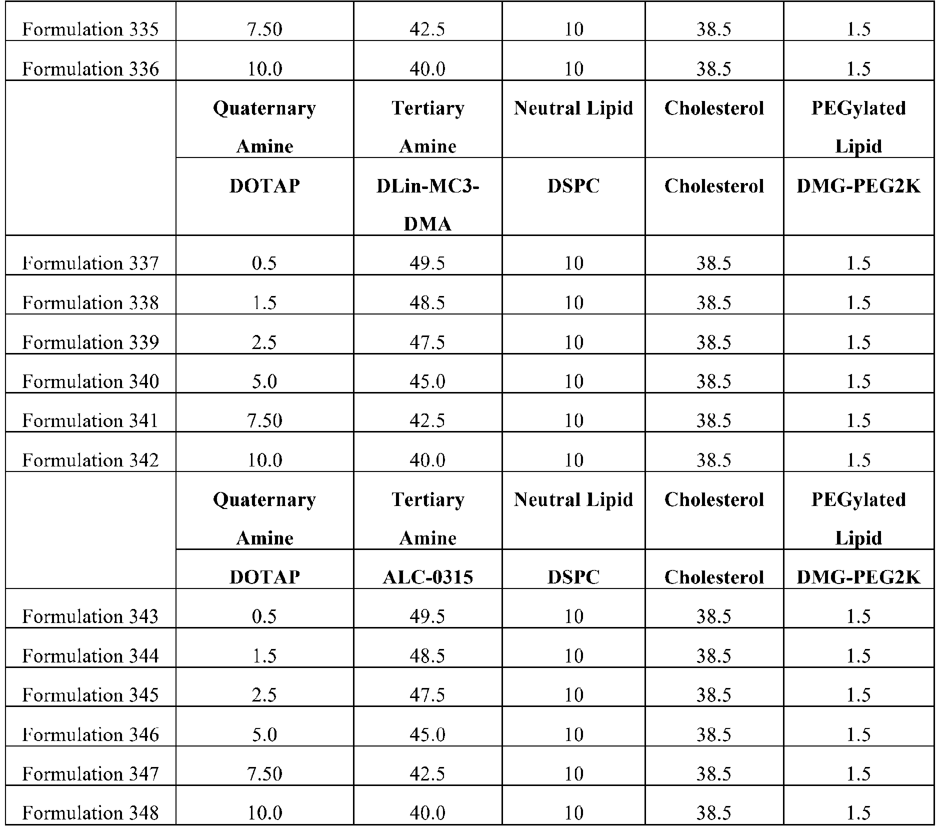

25. The lipid nanoparticle delivery system according to claim 16 or 24, wherein the active component is at least one selected from the group consisting of GFP-mRNA, TP53 mRNA, Archexin, and doxorubicin; preferably, the mass ratio of the active component to the lipid carrier is 1:5 to 1:30, preferably 1:10 or 1:20.

26. A preparation method for the lipid nanoparticle delivery system according to any one of claims 16-25, comprising steps (a)-(d) of process I, or steps (e)-(i) of process II:
Process I: (a) preparing a solution I comprising an active component using a buffer and the active component, (b) preparing a lipid solution using an organic solvent and a lipid carrier, (c) mixing the lipid solution and the solution I, diluting to obtain an initial lipid nanoparticle (LNP) solution, optionally repeating steps (a)-(c), (d) removing the organic solvent from the initial LNP solution, diluting and adjusting pH to 6.0-8.0, to obtain an active component-loaded LNP suspension;
Process II: (e) preparing a lipid solution using an organic solvent and a lipid carrier, (f) preparing an initial blank LNP solution using a buffer and the lipid solution, optionally repeating steps (e)-(f), (g) removing the organic solvent from the initial LNP solution, diluting and adjusting pH to 6.0-8.0, to obtain a blank LNP suspension, (h) preparing a solution I comprising an active component using a buffer and the active component, (i) mixing the solution I and the blank LNP suspension, then adjusting pH to 6.0-8.0, to obtain an active component-loaded LNP suspension;
preferably, the organic solvent is removed from the initial LNP solution by ultrafiltration and/or dialysis.

27. Use of the lipid composition according to any one of claims 1-13 or the lipid nanoparticle delivery system according to any one of claims 16-25 in the manufacture of a medicament for treating or preventing a disease, or a disease diagnostic agent.

28. The use according to claim 27, wherein the disease is one or more selected from the group consisting of tumors, immune diseases, inflammatory diseases, and infectious diseases.

29. A pharmaceutical composition, comprising the lipid composition according to any one of claims 1-13 or the lipid nanoparticle delivery system according to any one of claims 16-25, and one or more pharmaceutically acceptable carriers or excipients.

30. The pharmaceutical composition according to claim 29, further comprising a second therapeutic agent.

31. A method for treating or preventing a disease, comprising administering a therapeutically or prophylactically effective amount of the lipid composition according to any one of claims 1-13, the lipid nanoparticle delivery system according to any one of claims 16-25, or the pharmaceutical composition according to claim 29 or 30 to a subject in need thereof; preferably, prior to administering the therapeutically or prophylactically effective amount of the lipid composition to the subject in need thereof, the active component-loaded lipid nanoparticle suspension and the targeting component solution are mixed according to the preparation process of claim 14.

32. The method according to claim 31, wherein the lipid composition is administered together with an additional medicament; preferably, the lipid composition and the additional medicament are administered sequentially or simultaneously.

33. The method according to claim 31, wherein the lipid composition or pharmaceutical composition is administered via one or more of the following routes: intratumoral injection, intravenous injection, subcutaneous injection, intramuscular injection, intra-arterial injection, intraperitoneal injection, intra-central-nervous-system injection, intracavitary instillation, intravesical instillation, interventional therapy, oral, transdermal, pulmonary, ocular, and topical administration.

34. A method for transfecting a host cell, comprising administering the lipid composition according to any one of claims 1-13 or the lipid nanoparticle delivery system according to any one of claims 16-25 to the host cell.

35. A method for targeting delivery of an active component, comprising administering the lipid composition according to any one of claims 1-13 or the lipid nanoparticle delivery system according to any one of claims 16-25 to a subject or a biological sample.
